# EUROPEAN PATENT APPLICATION

(11) **EP 4 481 493 A1**
(43) Date of publication of application: **25.12.2024**
(21) Application number: 23756163.4
(22) Date of filing: 01.02.2023
(51) Int. Cl.: G03F 7/004, C07C 311/51, C07C 381/12, G03F 7/038, G03F 7/039, G03F 7/20

(54) **ACTINIC-RAY-SENSITIVE OR RADIATION-SENSITIVE RESIN COMPOSITION, ACTINIC-RAY-SENSITIVE OR RADIATION-SENSITIVE FILM, PATTERN FORMING METHOD, METHOD FOR PRODUCING ELECTRONIC DEVICE, AND COMPOUND**

(30) Priority: 16.02.2022 JP 2022022485
(71) Applicant: FUJIFILM Corporation, Tokyo 106-8620 (JP)
(72) Inventor: UEMURA Minoru, Haibara-gun, Shizuoka 421-0396 (JP); KOJIMA Masafumi, Haibara-gun, Shizuoka 421-0396 (JP); GOTO Akiyoshi, Haibara-gun, Shizuoka 421-0396 (JP); FUJIMAKI Nishiki, Haibara-gun, Shizuoka 421-0396 (JP); HIURA Nobuhiro, Haibara-gun, Shizuoka 421-0396 (JP); MARUMO Kazuhiro, Haibara-gun, Shizuoka 421-0396 (JP); MORI Takahiro, Haibara-gun, Shizuoka 421-0396 (JP); SHIRAKAWA Michihiro, Haibara-gun, Shizuoka 421-0396 (JP)
(74) Representative: HGF
(86) International application number: PCT/JP2023/003132
(87) International publication number: WO 2023/157635

(57) **Abstract**

An actinic ray-sensitive or radiation-sensitive resin composition and the like that have both of high roughness performance and high temporal roughness performance is provided. The actinic ray-sensitive or radiation-sensitive resin composition contains a compound (Q) represented by a specified formula (I-1) and a resin having a polarity that increases through decomposition by an action of an acid.

## Description

### BACKGROUND OF THE INVENTION

### 1. Field of the Invention

The present invention relates to an actinic ray-sensitive or radiation-sensitive resin composition, an actinic ray-sensitive or radiation-sensitive film, a pattern forming method, a method for producing an electronic device, and a compound.

### 2. Description of the Related Art

In the manufacturing process of semiconductor devices such as IC (Integrated Circuit, integrated circuits) and LSI (LargeScale Integrated circuit, large-scale integrated circuits), microfabrication by lithography using photosensitive compositions has been performed.

Examples of the lithography method include a method of using a photosensitive composition to form a resist film, subsequently exposing the obtained film, and subsequently developing the film. In particular, in recent years, the use of, in addition to the ArF excimer laser, EB (Electron Beam) or EUV (Extreme ultraviolet) light during exposure has been studied, and an actinic ray-sensitive or radiation-sensitive resin composition suitable for EUV exposure has been developed.

In the formation of a resist pattern using EUV (having a wavelength of 13.5 nm) or an electron beam for the purpose of forming a fine pattern, requirements for various performances are stricter than in the related-art cases of using ArF (having a wavelength of 193 nm) light or the like.

For example, JP2021-165824A describes a resist material including a base polymer and a specified onium salt compound having an anion having an iodine atom or a bromine atom at a specified site and having a -SO₂-N⁻-CO- structure.

JP2014-194534A describes an actinic ray-sensitive or radiation-sensitive resin composition containing a compound (A) including an anion having a specified structure having a -SO₂-N⁻-CO- or -CO-N⁻-CO- structure, a compound (B) that is different from (A) and generates an acid upon irradiation with an actinic ray or a radiation, and a polymer (P).

### SUMMARY OF THE INVENTION

In recent years, with the reduction in the sizes of patterns formed using EUV light or an electron beam, further improvements in various performances have been in demand. In particular, there remains room for studies on the roughness performance of resist compositions after being stored for a certain period of time (after a lapse of time).

Accordingly, an object of the present invention is to provide an actinic ray-sensitive or radiation-sensitive resin composition that has both of high roughness performance and high temporal roughness performance, an actinic ray-sensitive or radiation-sensitive resin film formed from the actinic ray-sensitive or radiation-sensitive resin composition, a pattern forming method and a method for producing an electronic device that use the actinic ray-sensitive or radiation-sensitive resin composition, and a compound that can be suitably used for the actinic ray-sensitive or radiation-sensitive resin composition.

The inventors of the present invention have found that the following features can address the above-described object.
[1] An actinic ray-sensitive or radiation-sensitive resin composition containing a compound (Q) represented by a formula (I-1) below and a resin having a polarity that increases through decomposition by an action of an acid, 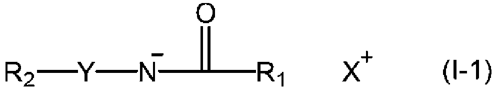 wherein, in the formula (I-1), R₁ represents a hydrocarbon group having at least one or more fluorine atoms, R₂ represents an aromatic group, Y represents -SO₂- or -CO-, and X⁺ represents a counter cation.
[2] The actinic ray-sensitive or radiation-sensitive resin composition according to [1], wherein R₁ is a group represented by a formula (II) or (III) below: 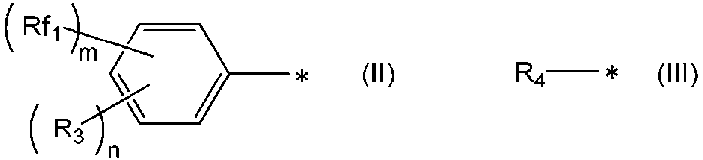 wherein, in the formula (II),
   Rf₁ represents a fluorine atom or a perfluoroalkyl group,
   R₃ represents a substituent,
   m represents an integer of 1 to 5,
   n represents an integer of 0 to 4,
   wherein 1 ≤ (m + n) ≤ 5 is satisfied,
   when m is an integer of 2 or more, a plurality of Rf₁'s may be the same or different,
   when n is an integer of 2 or more, a plurality of R₃'s may be the same or different and may be linked together to form a ring, and
   * represents a bonding site, and
   wherein, in the formula (III), R₄ represents an alkyl group or cycloalkyl group having at least one or more fluorine atoms, and * represents a bonding site.
[3] The actinic ray-sensitive or radiation-sensitive resin composition according to [1] or [2], wherein R₁ is a perfluoroalkyl group, a perfluorocycloalkyl group, or a perfluoroaryl group.
[4] The actinic ray-sensitive or radiation-sensitive resin composition according to any one of [1] to [3], wherein R₂ is an aromatic group having one or more substituents.
[5] The actinic ray-sensitive or radiation-sensitive resin composition according to any one of [1] to [4], wherein R₂ is an aromatic group having at least one substituent selected from the group consisting of a hydroxy group, an alkoxy group, an acyloxy group, an alkoxycarbonyl group, an aliphatic sulfonyl group, an aromatic sulfonyl group, a fluorine atom, a chlorine atom, a bromine atom, an iodine atom, an amino group, a nitro group, a cyano group, -NRₐ-C(=O)-R_{b}, and -NRₐ-C(=O)-O-R_{b} where Rₐ and R_{b} each independently represent a hydrogen atom or a hydrocarbon group.
[6] The actinic ray-sensitive or radiation-sensitive resin composition according to any one of [1] to [5], wherein R₂ is an aromatic group having at least one substituent selected from the group consisting of a hydroxy group, an alkoxy group, an acyloxy group, an alkoxycarbonyl group, an aliphatic sulfonyl group, an aromatic sulfonyl group, an amino group, a nitro group, a cyano group, -NRₐ-C(=O)-R_{b}, and -NRₐ-C(=O)-O-R_{b} where Rₐ and R_{b} each independently represent a hydrogen atom or a hydrocarbon group.
[7] The actinic ray-sensitive or radiation-sensitive resin composition according to any one of [1] to [6], wherein Y represents -SO₂-.
[8] The actinic ray-sensitive or radiation-sensitive resin composition according to any one of [1] to [7], wherein X⁺ is a sulfonium cation or an iodonium cation.
[9] The actinic ray-sensitive or radiation-sensitive resin composition according to any one of [1] to [8], further including a compound (P) that is different from the compound (Q) and generates an acid by irradiation with an actinic ray or a radiation.
[10] An actinic ray-sensitive or radiation-sensitive film formed from the actinic ray-sensitive or radiation-sensitive resin composition according to any one of [1] to [9].
[11] A pattern forming method including:
   a step of using the actinic ray-sensitive or radiation-sensitive resin composition according to any one of [1] to [9] to form an actinic ray-sensitive or radiation-sensitive film on a substrate;
   a step of exposing the actinic ray-sensitive or radiation-sensitive film; and
   a step of using a developer to develop the exposed actinic ray-sensitive or radiation-sensitive film to form a pattern.
[12] A method for producing an electronic device, the method including the pattern forming method according to [11].
[13] A compound represented by a formula (I-1) below: 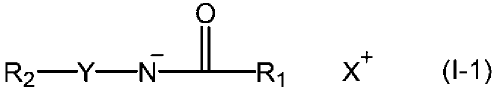 wherein, in the formula (I-1), R₁ represents a hydrocarbon group having at least one or more fluorine atoms, R₂ represents an aromatic group, Y represents -SO₂- or -CO-, and X⁺ represents a counter cation.
[14] The compound according to [13], wherein R₁ is a group represented by a formula (II) or (III) below: 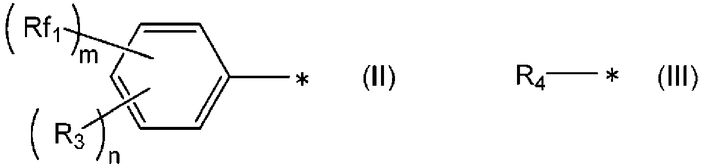 wherein, in the formula (II),
   Rf₁ represents a fluorine atom or a perfluoroalkyl group,
   R₃ represents a substituent,
   m represents an integer of 1 to 5,
   n represents an integer of 0 to 4,
   wherein 1 ≤ (m + n) ≤ 5 is satisfied,
   when m is an integer of 2 or more, a plurality of Rf₁'s may be the same or different,
   when n is an integer of 2 or more, a plurality of R₃'s may be the same or different and may be linked together to form a ring, and
   * represents a bonding site, and
   wherein, in the formula (III), R₄ represents an alkyl group or cycloalkyl group having at least one or more fluorine atoms, and * represents a bonding site.
[15] The compound according to [13] or [14], wherein R₁ is a perfluoroalkyl group, a perfluorocycloalkyl group, or a perfluoroaryl group.
[16] The compound according to any one of [13] to [15], wherein R₂ is an aromatic group having at least one substituent selected from the group consisting of a hydroxy group, an alkoxy group, an acyloxy group, an alkoxycarbonyl group, an aliphatic sulfonyl group, an aromatic sulfonyl group, a fluorine atom, a chlorine atom, a bromine atom, an iodine atom, an amino group, a nitro group, a cyano group, -NRₐ-C(=O)-R_{b}, and -NRₐ-C(=O)-O-R_{b} where Rₐ and R_{b} each independently represent a hydrogen atom or a hydrocarbon group.
[17] The compound according to any one of [13] to [16], wherein R₂ is an aromatic group having at least one substituent selected from the group consisting of a hydroxy group, an alkoxy group, an acyloxy group, an alkoxycarbonyl group, an aliphatic sulfonyl group, an aromatic sulfonyl group, an amino group, a nitro group, a cyano group, -NRₐ-C(=O)-R_{b}, and -NRₐ-C(=O)-O-R_{b} where Rₐ and R_{b} each independently represent a hydrogen atom or a hydrocarbon group.
[18] The compound according to any one of [13] to [17], wherein Y represents -SO₂-.
[19] The compound according to any one of [13] to [18], wherein X⁺ is a sulfonium cation or an iodonium cation.

The present invention can provide an actinic ray-sensitive or radiation-sensitive resin composition that has both of high roughness performance and high temporal roughness performance, an actinic ray-sensitive or radiation-sensitive resin film formed from the actinic ray-sensitive or radiation-sensitive resin composition, a pattern forming method and a method for producing an electronic device that use the actinic ray-sensitive or radiation-sensitive resin composition, and a compound that can be suitably used for the actinic ray-sensitive or radiation-sensitive resin composition.

### DESCRIPTION OF THE PREFERRED EMBODIMENTS

Hereinafter, the present invention will be described in detail.

Features may be described below on the basis of representative embodiments according to the present invention; however, the present invention is not limited to the embodiments.

In this Specification, for written forms of groups (atomic groups), written forms without referring to substituted or unsubstituted encompass, in addition to groups not having a substituent, groups including a substituent without departing from the spirit and scope of the present invention. For example, "alkyl group" encompasses not only alkyl groups not having a substituent (unsubstituted alkyl groups), but also alkyl groups having a substituent (substituted alkyl groups). In this Specification, "organic group" refers to a group including at least one carbon atom.

The substituent is preferably a monovalent substituent unless otherwise specified.

In this Specification, in the case of using a phrase "may have a substituent", the type of the substituent, the position of the substituent, and the number of such substituents are not particularly limited. The number of the substituents may be, for example, one, two, three, or more. Examples of the substituents include monovalent non-metallic atomic groups except for the hydrogen atom and, for example, can be selected from the group consisting of the following Substituents T.

### Substituents T

Examples of Substituents T include halogen atoms such as a fluorine atom, a chlorine atom, a bromine atom, and an iodine atom; alkoxy groups such as a methoxy group, an ethoxy group, and a tert-butoxy group; aryloxy groups such as a phenoxy group and a p-tolyloxy group; alkoxycarbonyl groups such as a methoxycarbonyl group, a butoxycarbonyl group, and a phenoxycarbonyl group; acyloxy groups such as an acetoxy group, a propionyloxy group, and a benzoyloxy group; acyl groups such as an acetyl group, a benzoyl group, an isobutyryl group, an acryloyl group, a methacryloyl group, and a methoxalyl group; alkylsulfanyl groups such as a methylsulfanyl group and a tert-butylsulfanyl group; arylsulfanyl groups such as a phenylsulfanyl group and a p-tolylsulfanyl group; alkyl groups; cycloalkyl groups; aryl groups; heteroaryl groups; a hydroxy group; a carboxy group; a formyl group; a sulfo group; a cyano group; alkylaminocarbonyl groups; arylaminocarbonyl groups; a sulfonamide group; a silyl group; an amino group; monoalkylamino groups; dialkylamino groups; arylamino groups; a nitro group; and combinations of the foregoing.

In this Specification, "actinic ray" or "radiation" means, for example, the emission line spectrum of a mercury lamp, far-ultraviolet rays represented by excimer lasers, extreme ultraviolet rays (EUV: Extreme Ultraviolet), X-rays, or an electron beam (EB: Electron Beam).

In this Specification, "light" means an actinic ray or a radiation.

In this Specification, "exposure" includes, unless otherwise specified, not only exposure using, for example, the emission line spectrum of a mercury lamp, far-ultraviolet rays represented by excimer lasers, extreme ultraviolet rays, or X-rays, but also patterning using a corpuscular beam such as an electron beam or an ion beam.

In this Specification, "a value 'to' another value" is used to mean that it includes the value and the other value as the lower limit value and the upper limit value.

In this Specification, the described bonding directions of divalent linking groups are not limited unless otherwise specified. For example, in a compound represented by a formula "X-Y-Z" where Y is -COO-, Y may be -CO-O- or -O-CO-. The compound may be "X-CO-O-Z" or may be "X-O-CO-Z".

In this Specification, (meth)acrylate represents acrylate and methacrylate, and (meth)acrylic represents acrylic and methacrylic.

In this Specification, the weight-average molecular weight (Mw), the number-average molecular weight (Mn), and the dispersity (hereafter, also referred to as "molecular weight distribution") (Mw/Mn) are defined as polystyrene-equivalent values measured using a GPC (Gel Permeation Chromatography) apparatus (HLC-8120GPC manufactured by Tosoh Corporation) by GPC measurement (solvent: tetrahydrofuran, flow rate (sample injection amount): 10 µL, column: TSK gel Multipore HXL-M manufactured by Tosoh Corporation, column temperature: 40°C, flow rate: 1.0 mL/min, detector: differential refractive index detector (Refractive Index Detector)).

In this Specification, the acid dissociation constant (pKa) represents pKa in an aqueous solution, specifically, a value determined using the following Software package 1, on the basis of the Hammett's substituent constant and the database of values in publicly known documents, by calculation.

### Software package 1: Advanced Chemistry Development (ACD/Labs) Software V8.14 for Solaris (1994-2007 ACD/Labs)

Alternatively, pKa can be determined by the molecular orbital calculation method. Specifically, this method may be a method of, on the basis of a thermodynamic cycle, calculating H⁺ dissociation free energy in an aqueous solution to achieve the determination. As the calculation method for H⁺ dissociation free energy, for example, DFT (density function theory) can be performed for calculation; however, other various methods have been reported in documents and the like and the calculation method is not limited to DFT. Note that there are a plurality of pieces of software for performing DFT, such as Gaussian 16.

In this Specification, as described above, pKa refers to a value determined using Software package 1, on the basis of the Hammett's substituent constant and the database of values in publicly known documents, by calculation; however, when use of this method cannot determine pKa, a value determined on the basis of DFT (density function theory) using Gaussian 16 is employed.

In this Specification, as described above, pKa refers to "pKa in an aqueous solution"; however, when pKa in an aqueous solution cannot be determined, "pKa in a dimethyl sulfoxide (DMSO) solution" is employed.

"Solid content" means components forming the resist film and does not include solvents. As long as a component forms the resist film, even when the component has the form of liquid, it is regarded as the solid content.

### Actinic ray-sensitive or radiation-sensitive resin composition

Hereinafter, an actinic ray-sensitive or radiation-sensitive resin composition of the present invention will be described.

The actinic ray-sensitive or radiation-sensitive resin composition of the present invention (hereafter, also referred to as "composition of the present invention") is typically a resist composition, and may be a positive resist composition or may be a negative resist composition. The resist composition may be a resist composition for alkali development or may be a resist composition for organic-solvent development. The resist composition may be a chemical amplification resist composition or may be a non-chemical amplification resist composition. The composition of the present invention is typically a chemical amplification resist composition.

The composition of the present invention contains a compound (Q) represented by a formula (I-1) below and a resin having a polarity that increases through decomposition by an action of an acid, 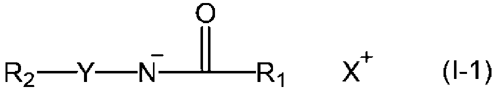 wherein, in the formula (I-1), R₁ represents a hydrocarbon group having at least one or more fluorine atoms, R₂ represents an aromatic group, Y represents -SO₂- or -CO-, and X⁺ represents a counter cation.

The reason why the composition of the present invention has both of high roughness performance and high temporal roughness performance has not been completely clarified, but is inferred by the inventors of the present invention as follows.

The compound (Q) used in the composition of the present invention has, as R₁ in the formula (I-1), a hydrocarbon group having at least one or more fluorine atoms. Such a fluorine atom facilitates absorption of light during EUV or electron-beam exposure to increase the efficiency of generation of secondary electrons, to thereby improve the efficiency of generation of an acid from the photoacid generator, which inferentially results in improvement in the roughness performance during pattern formation. For example, in a case where R₁ is a group having an iodine atom, the roughness performance is also improved for the same reason; however, the compound is likely to be decomposed due to the iodine atom during temporal storage of the composition, and the temporal roughness performance is poor. On the other hand, in the case of having a fluorine atom as in the compound (Q) in the present invention, decomposition due to temporal storage is suppressed and high temporal stability is provided, which inferentially results in high temporal roughness performance.

The compound (Q) further has, as R₂ in the formula (I-1), an aromatic group. When R₂ is an aliphatic group such as an alkyl group, the type of the substituent results in considerable variations in the basicity, so that, in some cases, the composition is adversely affected with time and the temporal roughness performance becomes poor. In addition, for this reason, it has been difficult to use the type of substituent to optimize various properties such as hydrophilicity and hydrophobicity. In the present invention, R₂ is an aromatic group, so that appropriate basicity is provided regardless of the presence or absence or type of the substituent, and temporal adverse effects are suppressed. In addition, it facilitates adjustment of the number or type of the substituents in order to optimize various properties such as hydrophilicity and hydrophobicity, which is also advantageous.

Hereinafter, first, various components of the actinic ray-sensitive or radiation-sensitive resin composition will be described in detail.

### Compound (Q)

The composition of the present invention includes a compound (Q) represented by a formula (I-1) below. Note that the present invention also relates to the compound represented by the formula (I-1) below.

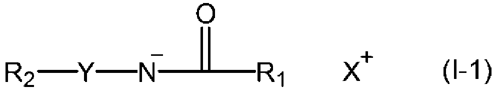

In the formula (I-1), R₁ represents a hydrocarbon group having at least one or more fluorine atoms. R₂ represents an aromatic group. Y represents -SO₂- or -CO-. X⁺ represents a counter cation.

In the formula (I-1), R₁ represents a hydrocarbon group having at least one or more fluorine atoms. R₁ may be an aromatic hydrocarbon group or aliphatic hydrocarbon group having at least one or more fluorine atoms. R₁ is preferably an aromatic hydrocarbon group or aliphatic hydrocarbon group having at least one or more fluorine atoms.

The aromatic hydrocarbon group may be an aryl group (monocyclic or polycyclic).

The aryl group is preferably an aryl group having 6 to 14 carbon atoms, and examples thereof include a phenyl group, a naphthyl group, and an anthryl group.

The aliphatic hydrocarbon group may be an alkyl group (linear or branched), a cycloalkyl group (monocyclic or polycyclic), an alkenyl group (linear or branched), or an alkynyl group (linear or branched).

Examples of the alkyl group include alkyl groups having 1 to 20 carbon atoms such as a methyl group, an ethyl group, an n-propyl group, an isopropyl group, an n-butyl group, an isobutyl group, and a t-butyl group.

Examples of the cycloalkyl group include monocyclic cycloalkyl groups having 3 to 10 carbon atoms such as a cyclopentyl group and a cyclohexyl group, and polycyclic cycloalkyl groups such as a norbornyl group, a tetracyclodecanyl group, a tetracyclododecanyl group, and an adamantyl group.

Examples of the alkenyl group include alkenyl groups having 2 to 20 carbon atoms such as a vinyl group.

Examples of the alkynyl group include alkynyl groups having 2 to 20 carbon atoms such as an ethynyl group.

For R₁, the hydrocarbon group having at least one or more fluorine atoms may be a group in which, in a group described above as an example, one or more hydrogen atoms are substituted with fluorine atoms or groups having a fluorine atom.

R₁ is preferably a group represented by a formula (II) or (III) below: 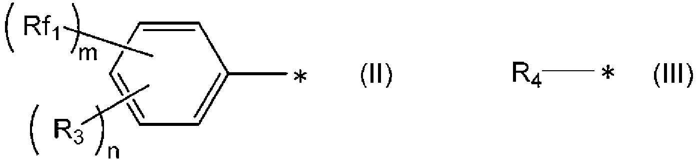
in the formula (II), Rf₁ represents a fluorine atom or a perfluoroalkyl group, R₃ represents a substituent, m represents an integer of 1 to 5, n represents an integer of 0 to 4, provided that 1 ≤ (m + n) ≤ 5 is satisfied; when m is an integer of 2 or more, a plurality of Rf₁'s may be the same or different; when n is an integer of 2 or more, a plurality of R₃'s may be the same or different, and may be linked together to form a ring; * represents a bonding site,
in the formula (III), R₄ represents an alkyl group or cycloalkyl group having at least one or more fluorine atoms, and * represents a bonding site.

In the formula (II), Rf₁ represents a fluorine atom or a perfluoroalkyl group.

For Rf₁, the perfluoroalkyl group may be a perfluoroalkyl group having 1 to 20 carbon atoms such as a trifluoromethyl group or a pentafluoroethyl group.

Rf₁ is preferably a fluorine atom.

In the formula (II), R₃ represents a substituent.

The substituent represented by R₃ is not particularly limited as long as it is a substituent other than the above-described groups corresponding to Rf₁; examples thereof include alkyl groups, cycloalkyl groups, aryl groups, a hydroxy group, alkoxy groups, acyloxy groups, alkylcarbonyloxy groups, alkylcarbonylamino groups, alkoxycarbonyl groups, aliphatic sulfonyl groups, aromatic sulfonyl groups, a chlorine atom, a bromine atom, an iodine atom, an amino group, a nitro group, a cyano group, -NRₐ-C(=O)-R_{b}, and -NRₐ-C(=O)-O-R_{b} (where Rₐ and R_{b} each independently represent a hydrogen atom or a hydrocarbon group).

The alkyl group may be an alkyl group having 1 to 20 carbon atoms, is preferably a methyl group or an ethyl group, and more preferably a methyl group.

In the alkoxy groups, the acyloxy groups, the alkylcarbonyloxy groups, the alkylcarbonylamino groups, and the alkoxycarbonyl groups, such an alkyl group may be an alkyl group having 1 to 20 carbon atoms, is preferably a methyl group or an ethyl group, and more preferably a methyl group.

Such a cycloalkyl group may be a monocyclic cycloalkyl group having 3 to 20 carbon atoms such as a cyclopentyl group or a cyclohexyl group, or a polycyclic cycloalkyl group such as a norbornyl group, a tetracyclodecanyl group, a tetracyclododecanyl group, or an adamantyl group, is preferably a cyclopentyl group or a cyclohexyl group, and more preferably a cyclohexyl group.

Such an aryl group is preferably an aryl group having 6 to 20 carbon atoms, may be, for example, a phenyl group, a naphthyl group, or an anthryl group, is preferably a phenyl group or a naphthyl group, and more preferably a phenyl group.

In such an aliphatic sulfonyl group, the aliphatic group may be, for example, an aliphatic hydrocarbon group. The aliphatic hydrocarbon group may be an aliphatic hydrocarbon group having 1 to 20 carbon atoms and may be, for example, an alkyl group having 1 to 20 carbon atoms. In particular, preferred is a methyl group or an ethyl group, and more preferred is a methyl group.

In such an aromatic sulfonyl group, the aromatic group is preferably an aryl group (monocyclic or polycyclic).

Such an aryl group is preferably an aryl group having 6 to 20 carbon atoms, may be, for example, a phenyl group, a naphthyl group, or an anthryl group, is preferably a phenyl group or a naphthyl group, and more preferably a phenyl group.

In the -NRₐ-C(=O)-R_{b} group and the -NRₐ-C(=O)-O-R_{b} group, for Rₐ and R_{b}, the hydrocarbon group may be a hydrocarbon group having 1 to 20 carbon atoms, may specifically be, for example, an alkyl group, alkenyl group, alkynyl group, cycloalkyl group, or aryl group having 1 to 20 carbon atoms; preferred is an alkyl group having 1 to 10 carbon atoms, and more preferred is a methyl group.

When a plurality of R₃'s are linked together to form a ring, the ring is preferably a ring having 5 or 6 ring members.

In the formula (II), m represents an integer of 1 to 5, n represents an integer of 0 to 4, provided that 1 ≤ (m + n) ≤ 5 is satisfied.
m is preferably an integer of 3 to 5, and more preferably 5.
n is preferably an integer of 0 to 2, and more preferably 0.

In the formula (III), R₄ represents an alkyl group or cycloalkyl group having at least one or more fluorine atoms.

The alkyl group may be an alkyl group having 1 to 20 carbon atoms such as a methyl group, an ethyl group, an n-propyl group, an isopropyl group, an n-butyl group, an isobutyl group, or a t-butyl group, is preferably an alkyl group having 1 to 3 carbon atoms, and more preferably a methyl group, an ethyl group, or an n-propyl group.

The alkyl group having at least one or more fluorine atoms may be a group in which, in an alkyl group described above as an example, one or more hydrogen atoms are substituted with fluorine atoms, and specific examples thereof include -CF₃, -CF₂-CF₃, -CF₂-CF₂-CF₃, -CH₂F, - CH₂-CH₂F, -CH₂-CHF₂, and -CH₂-CF₃.

The cycloalkyl group may be a monocyclic cycloalkyl group having 3 to 20 carbon atoms such as a cyclopentyl group and a cyclohexyl group, or a polycyclic cycloalkyl group such as a norbornyl group, a tetracyclodecanyl group, a tetracyclododecanyl group, or an adamantyl group, is preferably a cyclopentyl group or a cyclohexyl group, and more preferably a cyclohexyl group.

The cycloalkyl group having at least one or more fluorine atoms may be a group in which, in a cycloalkyl group described above as an example, one or more hydrogen atoms are substituted with fluorine atoms.

R₄ may further have a substituent, and examples of the substituent include the above-described substituents serving as R₃ in the formula (II).

R₁ is preferably a perfluoroalkyl group, a perfluorocycloalkyl group, or a perfluoroaryl group, and more preferably a perfluoroalkyl group having 1 to 3 carbon atoms, a perfluorocyclohexyl group, or a perfluorophenyl group.

In the formula (I-1), R₂ represents an aromatic group.

The aromatic group may be an aromatic hydrocarbon group such as an aryl group, or may be an aromatic heterocyclic group.

The aromatic group is preferably an aryl group (monocyclic or polycyclic).

The aryl group is preferably an aryl group having 6 to 20 carbon atoms, may be, for example, a phenyl group, a naphthyl group, or an anthryl group, is preferably a phenyl group or a naphthyl group, and more preferably a phenyl group.

The aromatic group represented by R₂ may have or may not have a substituent. R₂ is preferably an aromatic group having one or more substituents.

When the aromatic group represented by R₂ has a substituent, examples of the substituent include a hydroxy group, an alkoxy group, an acyloxy group, an alkylcarbonyloxy group, an alkylcarbonylamino group, an alkoxycarbonyl group, an aliphatic sulfonyl group, an aromatic sulfonyl group, a fluorine atom, a chlorine atom, a bromine atom, an iodine atom, an amino group, a nitro group, a cyano group, -NRₐ-C(=O)-R_{b}, and -NRₐ-C(=O)-O-R_{b} (where Rₐ and R_{b} each independently represent a hydrogen atom or a hydrocarbon group).

In the alkoxy group, the acyloxy group, the alkylcarbonyloxy group, the alkylcarbonylamino group, and the alkoxycarbonyl group, such an alkyl group may be an alkyl group having 1 to 20 carbon atoms, is preferably a methyl group or an ethyl group, and more preferably a methyl group.

The aliphatic group in the aliphatic sulfonyl group may be, for example, an aliphatic hydrocarbon group. The aliphatic hydrocarbon group may be an aliphatic hydrocarbon group having 1 to 20 carbon atoms and may be, for example, an alkyl group having 1 to 20 carbon atoms. In particular, preferred is a methyl group or an ethyl group, and more preferred is a methyl group.

The aromatic group in the aromatic sulfonyl group is preferably an aryl group (monocyclic or polycyclic).

The aryl group is preferably an aryl group having 6 to 20 carbon atoms, may be, for example, a phenyl group, a naphthyl group, or an anthryl group, is preferably a phenyl group or a naphthyl group, and more preferably a phenyl group.

In the -NRₐ-C(=O)-R_{b} group and the -NRₐ-C(=O)-O-R_{b} group, for Rₐ and R_{b}, the hydrocarbon group may be a hydrocarbon group having 1 to 20 carbon atoms, may specifically be an alkyl group, alkenyl group, alkynyl group, cycloalkyl group, or aryl group having 1 to 20 carbon atoms, is preferably an alkyl group having 1 to 10 carbon atoms, and more preferably a methyl group.

Rₐ preferably represents a hydrogen atom. R_{b} preferably represents an alkyl group having 1 to 10 carbon atoms, and is more preferably a methyl group.

In a preferred embodiment, R₂ is preferably an aromatic group having at least one substituent selected from the group consisting of a hydroxy group, an alkoxy group, an acyloxy group, an alkoxycarbonyl group, an aliphatic sulfonyl group, an aromatic sulfonyl group, a fluorine atom, a chlorine atom, a bromine atom, an iodine atom, an amino group, a nitro group, a cyano group, -NRₐ-C(=O)-R_{b}, and -NRₐ-C(=O)-O-R_{b} (where Rₐ and R_{b} each independently represent a hydrogen atom or a hydrocarbon group), more preferably an aromatic group having at least one substituent selected from the group consisting of a hydroxy group, an alkoxy group, an acyloxy group, an alkoxycarbonyl group, an aliphatic sulfonyl group, an aromatic sulfonyl group, an amino group, a nitro group, a cyano group, -NRₐ-C(=O)-R_{b}, and -NRₐ-C(=O)-O-R_{b}, and still more preferably an aromatic group having at least one substituent selected from the group consisting of a hydroxy group, an alkoxy group, an acyloxy group, an alkoxycarbonyl group, an aliphatic sulfonyl group, an aromatic sulfonyl group, a nitro group, a cyano group, and -NRₐ-C(=O)-R_{b}.

The number of substituents is not particularly limited, but is preferably 1 to 3, and more preferably 1 or 2. When a plurality of substituents are present, the substituents may be the same or different.

In the formula (I-1), Y represents -SO₂- or -CO-, and preferably represents -SO₂-.

The following are specific examples of the anionic moiety represented by R₂-Y-N⁻-C(=O)-R₁ in the formula (I-1); however, the present invention is not limited thereto. Me represent a methyl group.

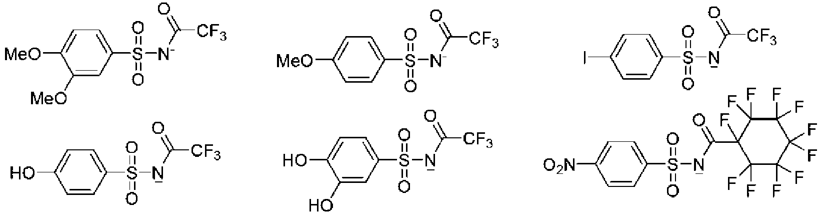

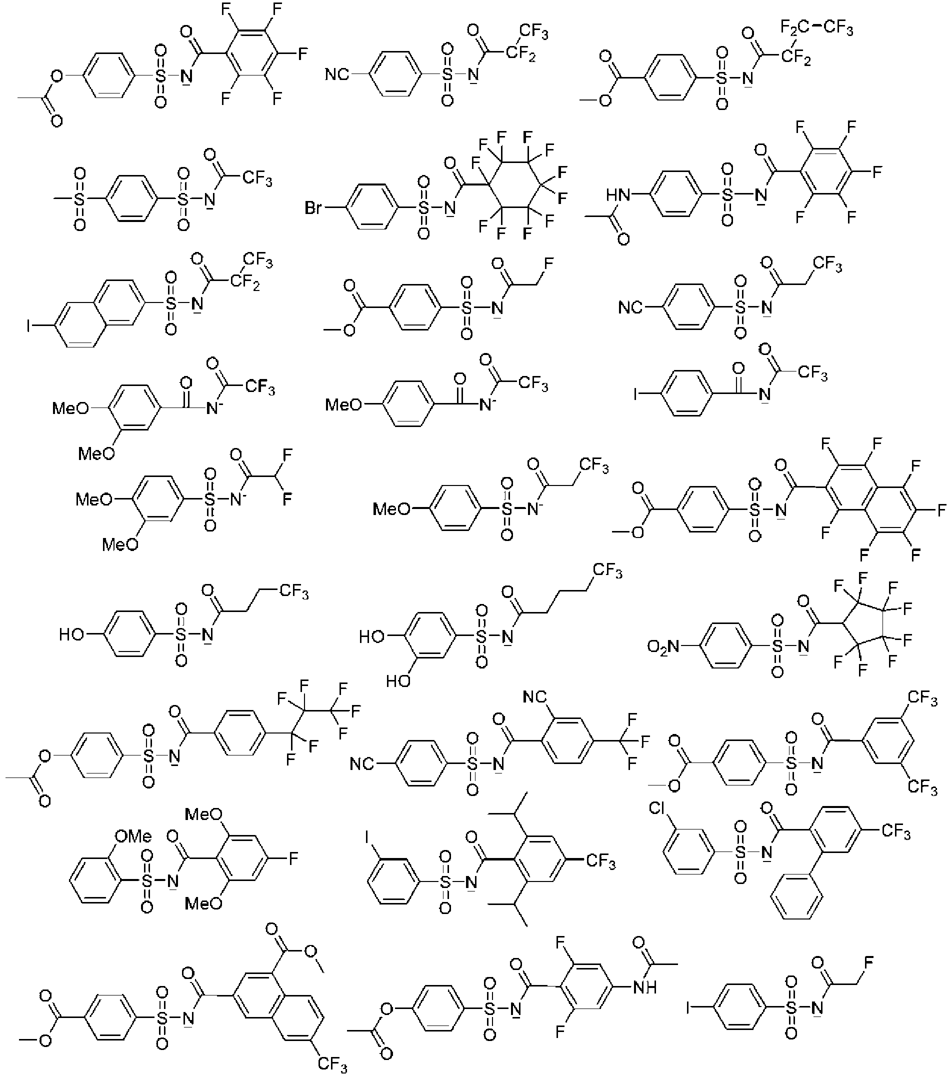

In the formula (I-1), X⁺ represents a counter cation.

The counter cation represented by X⁺ is not particularly limited, and is preferably an organic cation.

In particular, preferred is a sulfonium cation or an iodonium cation and preferred is a cation represented by formula (ZaI) (hereafter, also referred to as "cation (ZaI)") or a cation represented by a formula (ZaII) (hereafter, also referred to as "cation (ZaII)").

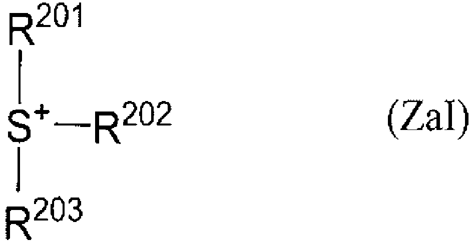

R²⁰⁴-I⁺-R²⁰⁵ (ZaII)

In the above-described formula (ZaI), R²⁰¹, R²⁰², and R²⁰³ each independently represent an organic group.

For R²⁰¹, R²⁰², and R²⁰³, such an organic group preferably has 1 to 30, and more preferably 1 to 20 carbon atoms. Among R²⁰¹ to R²⁰³, two may be bonded together to form a ring structure and the ring may include an oxygen atom, a sulfur atom, an ester group, an amide group, or a carbonyl group. Examples of the group formed by bonding together two among R²⁰¹ to R²⁰³ include alkylene groups (such as a butylene group and a pentylene group), and -CH₂-CH₂-O-CH₂-CH₂-.

Preferred examples of the organic cation in the formula (ZaI) include a cation (ZaI-1), a cation (ZaI-2), a cation (ZaI-3b), and a cation (ZaI-4b) described later.

First, the cation (ZaI-1) will be described.

The cation (ZaI-1) is an aryl sulfonium cation represented by the above-described formula (ZaI) where at least one of R²⁰¹ to R²⁰³ is an aryl group.

In the aryl sulfonium cation, all of R²⁰¹ to R²⁰¹ may be aryl groups, or a part of R²⁰¹ to R²⁰³ may be an aryl group and the other may be an alkyl group or a cycloalkyl group.

Alternatively, one among R²⁰¹ to R²⁰³ may be an aryl group and the other two among R²⁰¹ to R²⁰³ may be bonded together to form a ring structure in which the ring may include an oxygen atom, a sulfur atom, an ester group, an amide group, or a carbonyl group. Examples of the group formed by bonding together two among R²⁰¹ to R²⁰³ include alkylene groups in which one or more methylene groups may be substituted with an oxygen atom, a sulfur atom, an ester group, an amide group, and/or a carbonyl group (such as a butylene group, a pentylene group, and - CH₂-CH₂-O-CH₂-CH₂-).

Examples of the aryl sulfonium cation include triaryl sulfonium cations, diaryl alkyl sulfonium cations, aryl dialkyl sulfonium cations, diaryl cycloalkyl sulfonium cations, and aryl dicycloalkyl sulfonium cations.

The aryl group included in the aryl sulfonium cation is preferably a phenyl group or a naphthyl group, and more preferably a phenyl group. The aryl group may be an aryl group having a heterocyclic structure having an oxygen atom, a nitrogen atom, or a sulfur atom, for example. Examples of the heterocyclic structure include a pyrrole residue, a furan residue, a thiophene residue, an indole residue, a benzofuran residue, and a benzothiophene residue. When the aryl sulfonium cation has two or more aryl groups, the two or more aryl groups may be the same or different.

The alkyl group or cycloalkyl group that the aryl sulfonium cation optionally has is preferably a linear alkyl group having 1 to 15 carbon atoms, a branched alkyl group having 3 to 15 carbon atoms, or a cycloalkyl group having 3 to 15 carbon atoms, and more preferably a methyl group, an ethyl group, a propyl group, an n-butyl group, a sec-butyl group, a t-butyl group, a cyclopropyl group, a cyclobutyl group, or a cyclohexyl group.

For R²⁰¹ to R²⁰³, a substituent that the aryl group, the alkyl group, and the cycloalkyl group may have is preferably an alkyl group (having, for example, 1 to 15 carbon atoms), a cycloalkyl group (having, for example, 3 to 15 carbon atoms), an aryl group (having, for example, 6 to 14 carbon atoms), an alkoxy group (having, for example, 1 to 15 carbon atoms), a cycloalkylalkoxy group (having, for example, 1 to 15 carbon atoms), a halogen atom (for example, fluorine or iodine), a hydroxyl group, a carboxyl group, an ester group, a sulfinyl group, a sulfonyl group, an alkylthio group, or a phenylthio group.

The substituent may further have, when possible, a substituent; the alkyl group also preferably has, as a substituent, a halogen atom to serve as an alkyl halide group such as a trifluoromethyl group.

Such substituents are also preferably combined appropriately to form an acid-decomposable group.

Note that the acid-decomposable group means a group that is decomposed by the action of an acid to generate a polar group, and preferably has a structure in which a group that leaves by the action of an acid protects the polar group. The polar group and the leaving group are as described above.

Hereinafter, the cation (ZaI-2) will be described.

The cation (ZaI-2) is a cation represented by the formula (ZaI) where R²⁰¹ to R²⁰³ each independently represent an organic group not having an aromatic ring. The aromatic ring also encompasses aromatic rings including a heteroatom.

For R²⁰¹ to R²⁰³, the organic group not having an aromatic ring preferably has 1 to 30 carbon atoms and more preferably 1 to 20 carbon atoms.

R²⁰¹ to R²⁰³ are each independently preferably an alkyl group, a cycloalkyl group, an allyl group, or a vinyl group, more preferably a linear or branched 2-oxoalkyl group, a 2-oxocycloalkyl group, or an alkoxycarbonylmethyl group, and still more preferably a linear or branched 2-oxoalkyl group.

For R²⁰¹ to R²⁰³, the alkyl group and the cycloalkyl group may be, for example, a linear alkyl group having 1 to 10 carbon atoms or a branched alkyl group having 3 to 10 carbon atoms (for example, a methyl group, an ethyl group, a propyl group, a butyl group, or a pentyl group), or a cycloalkyl group having 3 to 10 carbon atoms (for example, a cyclopentyl group, a cyclohexyl group, or a norbornyl group).

R²⁰¹ to R²⁰³ may be further substituted with a halogen atom, an alkoxy group (having, for example, 1 to 5 carbon atoms), a hydroxy group, a cyano group, or a nitro group.

For R²⁰¹ to R²⁰³, substituents are also preferably provided independently as appropriate combinations of substituents to form acid-decomposable groups.

Hereinafter, the cation (ZaI-3b) will be described.

The cation (ZaI-3b) is a cation represented by the following formula (ZaI-3b).

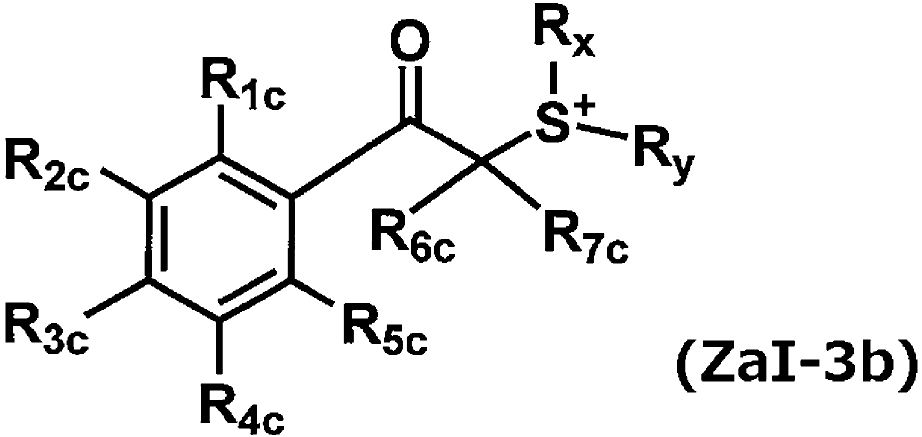

In the formula (ZaI-3b), R_{1c} to R_{5c} each independently represent a hydrogen atom, an alkyl group, a cycloalkyl group, an aryl group, an alkoxy group, an aryloxy group, an alkoxycarbonyl group, an alkylcarbonyloxy group, a cycloalkylcarbonyloxy group, a halogen atom, a hydroxy group, a nitro group, an alkylthio group, or an arylthio group.

R_{6c} and R_{7c} each independently represent a hydrogen atom, an alkyl group (for example, a t-butyl group), a cycloalkyl group, a halogen atom, a cyano group, or an aryl group.

Rₓ and R_{y} each independently represent an alkyl group, a cycloalkyl group, a 2-oxoalkyl group, a 2-oxocycloalkyl group, an alkoxycarbonylalkyl group, an allyl group, or a vinyl group.

For R_{1c} to R_{7c} and Rₓ and R_{y}, such substituents are also preferably provided independently as appropriate combinations of substituents to form acid-decomposable groups.

Any two or more among R_{1c} to R_{5c}, R_{5c} and R_{6c}, R_{6c} and R_{7c}, R_{5c} and Rₓ, and Rₓ and R_{y} may be individually bonded together to form rings; these rings may each independently include an oxygen atom, a sulfur atom, a ketone group, an ester bond, or an amide bond.

Such a ring may be an aromatic or non-aromatic hydrocarbon ring, an aromatic or non-aromatic heterocycle, or a polycyclic fused ring formed as a combination of two or more of these rings. The ring may be a 3- to 10-membered ring, and is preferably a 4- to 8-membered ring, and more preferably a 5- or 6-membered ring.

Examples of the groups formed by bonding together any two or more among R_{1c} to R_{5c}, R_{6c} and R_{7c}, and Rₓ and R_{y} include alkylene groups such as a butylene group and a pentylene group. In such an alkylene group, a methylene group may be substituted with a heteroatom such as an oxygen atom.

The groups formed by bonding together R_{5c} and R_{6c}, and R_{5c} and Rₓ are preferably single bonds or alkylene groups. Examples of the alkylene groups include a methylene group and an ethylene group.

R_{1c} to R_{5c}, R_{6c}, R_{7c}, Rₓ, R_{y}, and the rings formed by individually bonding together any two or more among R_{1c} to R_{5c}, R_{5c} and R_{6c}, R_{6c} and R_{7c}, R_{5c} and Rₓ, and Rₓ and R_{y} may have a substituent.

Hereinafter, the cation (ZaI-4b) will be described.

The cation (ZaI-4b) is a cation represented by the following formula (ZaI-4b).

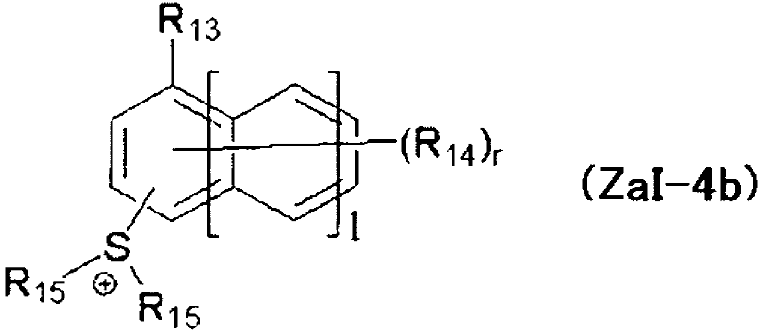

In the formula (ZaI-4b), l represents an integer of 0 to 2, and r represents an integer of 0 to 8.

R₁₃ represents a hydrogen atom, a halogen atom (for example, a fluorine atom or an iodine atom), a hydroxyl group, an alkyl group, an alkyl halide group, an alkoxy group, a carboxyl group, an alkoxycarbonyl group, or a group including a cycloalkyl group (may be the cycloalkyl group itself or a group including, as a part thereof, the cycloalkyl group). These groups may have a substituent.

R₁₄ represents a hydroxy group, a halogen atom (for example, a fluorine atom or an iodine atom), an alkyl group, an alkyl halide group, an alkoxy group, an alkoxycarbonyl group, an alkylcarbonyl group, an alkylsulfonyl group, a cycloalkylsulfonyl group, or a group including a cycloalkyl group (may be the cycloalkyl group itself or a group including, as a part thereof, the cycloalkyl group). These groups may have a substituent. When there are a plurality of R₁₄'s, R₁₄'s each independently represent such a group, for example, a hydroxy group.

R₁₅'s each independently represent an alkyl group, a cycloalkyl group, or a naphthyl group. Two R₁₅'s may be bonded together to form a ring. When two R₁₅'s are bonded together to form a ring, the ring skeleton may include a heteroatom such as an oxygen atom or a nitrogen atom.

In an example, two R₁₅'s are preferably alkylene groups and bonded together to form a ring structure. Note that the alkyl group, the cycloalkyl group, the naphthyl group, and the ring formed by bonding together two R₁₅'s may have a substituent.

In the formula (ZaI-4b), for R₁₃, R₁₄, and R₁₅, the alkyl groups may be linear or branched. Such an alkyl group preferably has 1 to 10 carbon atoms. Preferred examples of the alkyl group include a methyl group, an ethyl group, an n-butyl group, and a t-butyl group.

For R₁₃ to R₁₅, and Rₓ and R_{y}, such substituents are also preferably provided independently as appropriate combinations of substituents to form acid-decomposable groups.

Hereinafter, the formula (ZaII) will be described.

In the formula (ZaII), R²⁰⁴ and R²⁰⁵ each independently represent an aryl group, an alkyl group, or a cycloalkyl group.

For R²⁰⁴ and R²⁰⁵, the aryl group is preferably a phenyl group or a naphthyl group, and more preferably a phenyl group. For R²⁰⁴ and R²⁰⁵, the aryl group may be an aryl group having a heterocycle having an oxygen atom, a nitrogen atom, or a sulfur atom, for example. Examples of the skeleton of the aryl group having a heterocycle include pyrrole, furan, thiophene, indole, benzofuran, and benzothiophene.

For R²⁰⁴ and R²⁰⁵, the alkyl group and the cycloalkyl group are preferably a linear alkyl group having 1 to 10 carbon atoms, a branched alkyl group having 3 to 10 carbon atoms (for example, a methyl group, an ethyl group, a propyl group, a butyl group, or a pentyl group), or a cycloalkyl group having 3 to 10 carbon atoms (for example, a cyclopentyl group, a cyclohexyl group, or a norbornyl group).

For R²⁰⁴ and R²⁰⁵, the aryl group, the alkyl group, and the cycloalkyl group may each independently have a substituent. For R²⁰⁴ and R²⁰⁵, examples of the substituent that the aryl group, the alkyl group, and the cycloalkyl group may have include alkyl groups (having, for example, 1 to 15 carbon atoms), cycloalkyl groups (having, for example, 3 to 15 carbon atoms), aryl groups (having, for example, 6 to 15 carbon atoms), alkoxy groups (having, for example, 1 to 15 carbon atoms), halogen atoms, a hydroxy group, and a phenylthio group. For R²⁰⁴ and R²⁰⁵, substituents are also preferably provided independently as appropriate combinations of substituents to form acid-decomposable groups.

The following are specific examples of the counter cation represented by X⁺; however, the present invention is not limited thereto.

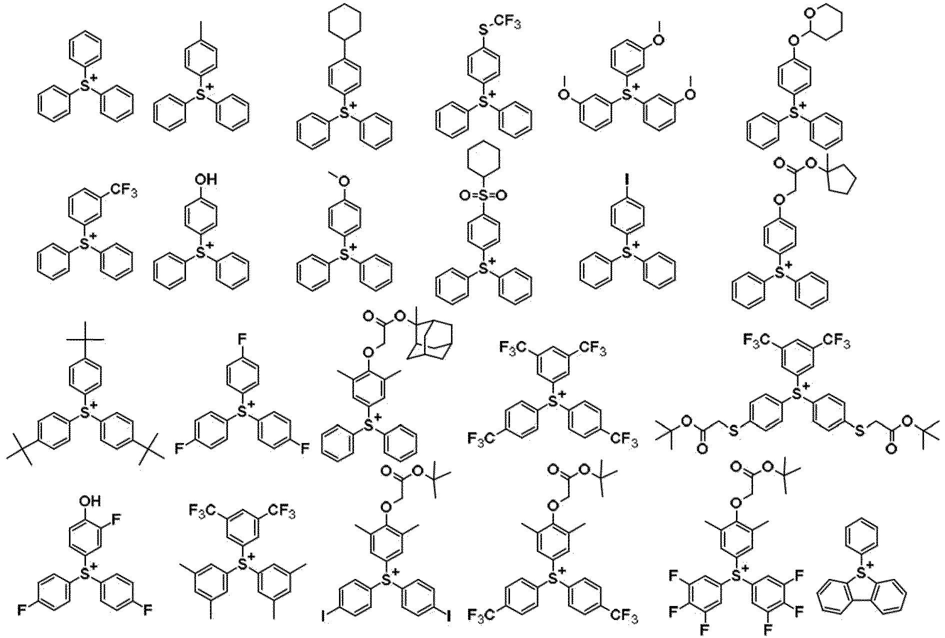

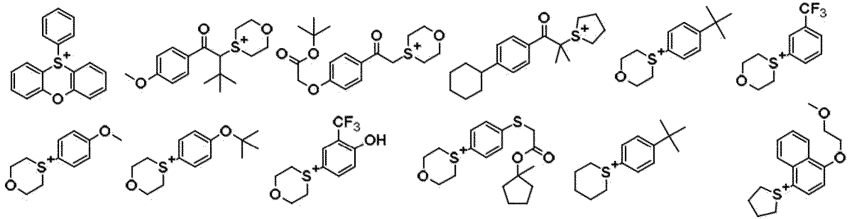

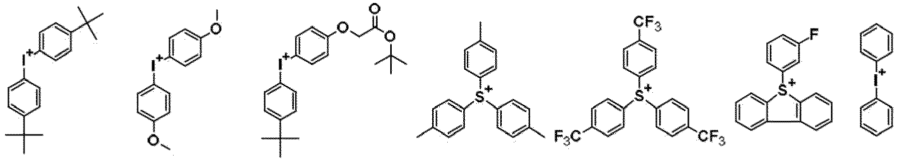

Preferred examples of the compound (Q) will be described below; however, the present invention is not limited thereto. Me represent a methyl group. In the following compounds, the combinations of a cation and an anion can be changed.

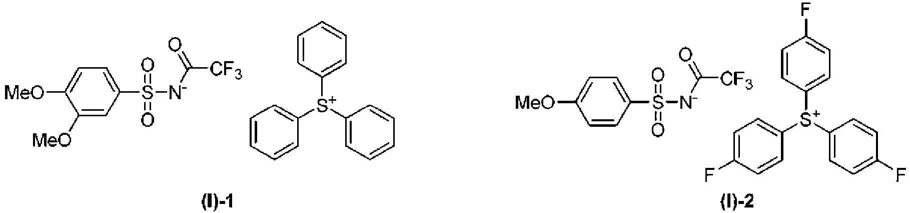

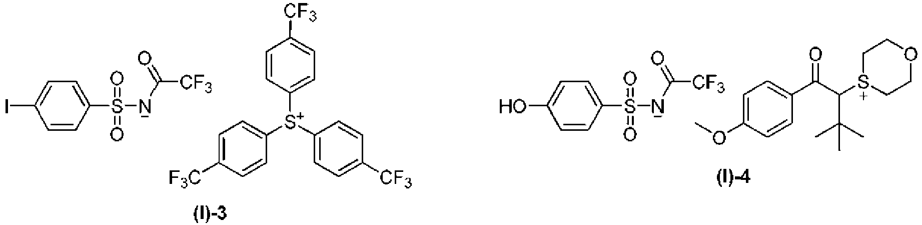

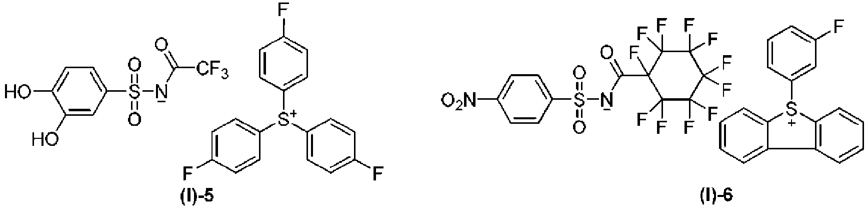

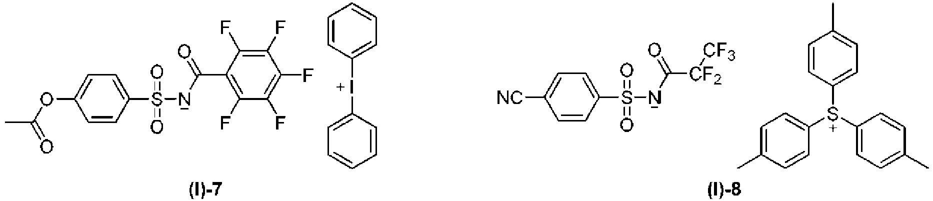

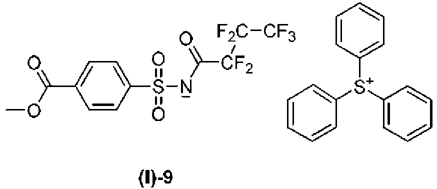

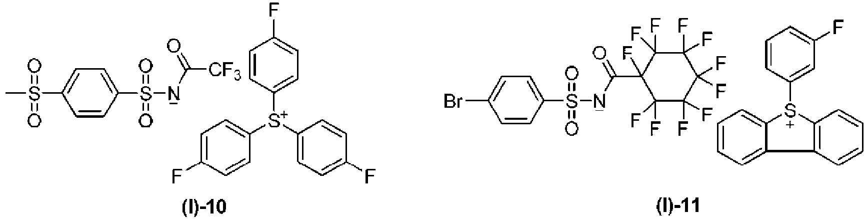

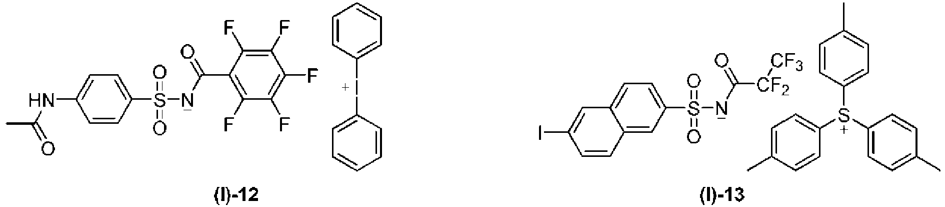

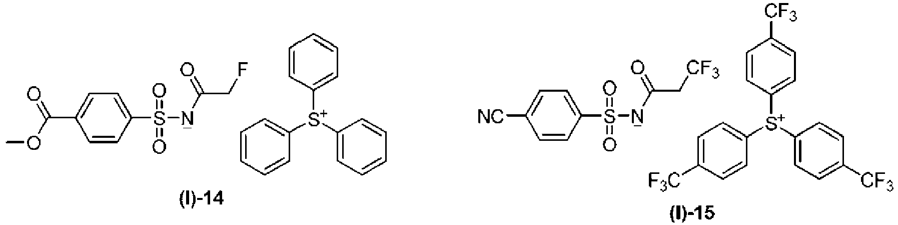

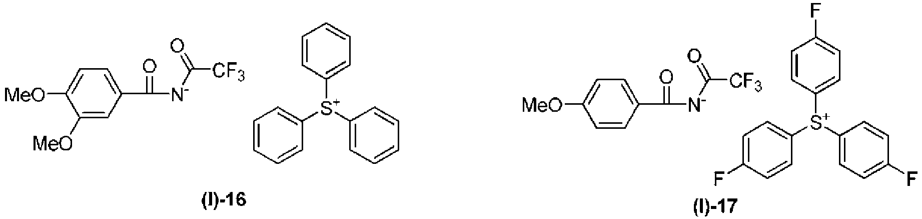

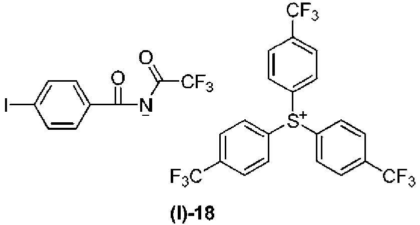

The compound (Q) is a compound that generates an acid upon irradiation with an actinic ray or a radiation (photoacid generator).

When the compound (Q) is used as an acid diffusion control agent, it is preferably used in combination with a photoacid generator that generates an acid necessary for the reaction of the resin in the exposed region and that generates a strong acid relative to the acid generated from the compound (Q).

The compound (Q) can be synthesized with reference to a publicly known method. Specific synthesis examples will be described later in EXAMPLES.

The compound (Q) has a molecular weight of preferably 300 to 3000, more preferably 300 to 2000, and still more preferably 300 to 1500.

Such compounds (Q) may be used alone or in combination of two or more thereof.

In the composition of the present invention, the content of the compound (Q) (when there are a plurality of compounds (Q), the total content thereof) is, on the basis of the total solid content of the composition, preferably 0.2 to 50 mass%, more preferably 0.5 to 40 mass%, and still more preferably 0.5 to 30 mass%.

Resin having a polarity that increases through decomposition by an action of an acid

The composition of the present invention includes a resin having a polarity that increases through decomposition by an action of an acid (hereafter, also referred to as "resin (A)").

The resin (A) ordinarily includes a group having a polarity that increases through decomposition by an action of an acid (hereafter, also referred to as an "acid-decomposable group") and preferably includes a repeating unit having an acid-decomposable group.

When the resin (A) has an acid-decomposable group, in a pattern forming method in this Specification, typically, in the case of employing a developer that is an alkali developer, a positive-type pattern is suitably formed or, in the case of employing a developer that is an organic-based developer, a negative-type pattern is suitably formed.

Preferred examples of the repeating unit having an acid-decomposable group include, in addition to a repeating unit having an acid-decomposable group described later, a repeating unit having an acid-decomposable group including an unsaturated bond.

### Repeating unit having acid-decomposable group

The acid-decomposable group refers to a group that is decomposed by the action of an acid to generate a polar group. The acid-decomposable group preferably has a structure in which the polar group is protected with a group (leaving group) that leaves by the action of an acid. Thus, the resin (A) has a repeating unit having a group that is decomposed by the action of an acid to generate a polar group. The resin having the repeating unit is subjected to the action of an acid to have increased polarity to have an increased degree of solubility in the alkali developer, but have a decreased degree of solubility in organic solvents.

The polar group is preferably an alkali-soluble group; examples thereof include acidic groups such as a carboxyl group, a phenolic hydroxyl group, fluorinated alcohol groups, a sulfonic acid group, a phosphoric acid group, a sulfonamide group, a sulfonylimide group, (alkylsulfonyl)(alkylcarbonyl)methylene groups, (alkylsulfonyl)(alkylcarbonyl)imide groups, bis(alkylcarbonyl)methylene groups, bis(alkylcarbonyl)imide groups, bis(alkylsulfonyl)methylene groups, bis(alkylsulfonyl)imide groups, tris(alkylcarbonyl)methylene groups, and tris(alkylsulfonyl)methylene groups, and an alcoholic hydroxyl group.

In particular, the polar group is preferably a carboxyl group, a phenolic hydroxyl group, a fluorinated alcohol group (preferably a hexafluoroisopropanol group), or a sulfonic acid group.

Examples of the group that leaves by the action of an acid include groups represented by formulas (Y1) to (Y4).

formula (Y1) : -C(Rx₁)(Rx₂)(Rx₃)

formula (Y2) : -C(=O)OC(Rx₁)(Rx₂)(Rx₃)

formula (Y3) : -C(R₃₆)(R₃₇)(OR₃₈)

formula (Y4) : -C(Rn)(H)(Ar)

In the formula (Y1) and the formula (Y2), Rx₁ to Rx₃ each independently represent an alkyl group (linear or branched), a cycloalkyl group (monocyclic or polycyclic), an alkenyl group (linear or branched), or an aryl group (monocyclic or polycyclic). Note that, when Rx₁ to Rx₃ are all alkyl groups (linear or branched), at least two among Rx₁ to Rx₃ are preferably methyl groups.

In particular, Rx₁ to Rx₃ preferably each independently represent a linear or branched alkyl group, and Rx₁ to Rx₃ more preferably each independently represent a linear alkyl group.

Two among Rx₁ to Rx₃ may be bonded together to form a monocycle or a polycycle.

For Rx₁ to Rx₃, the alkyl group is preferably an alkyl group having 1 to 5 carbon atoms such as a methyl group, an ethyl group, an n-propyl group, an isopropyl group, an n-butyl group, an isobutyl group, or a t-butyl group.

For Rx₁ to Rx₃, the cycloalkyl group is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group.

For Rx₁ to Rx₃, the aryl group is preferably an aryl group having 6 to 10 carbon atoms and may be, for example, a phenyl group, a naphthyl group, or an anthryl group.

For Rx₁ to Rx₃, the alkenyl group is preferably a vinyl group.

The ring formed by bonding together two among Rx₁ to Rx₃ is preferably a cycloalkyl group. The cycloalkyl group formed by bonding together two among Rx₁ to Rx₃ is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group, and more preferably a monocyclic cycloalkyl group having 5 to 6 carbon atoms.

In the cycloalkyl group formed by bonding together two among Rx₁ to Rx₃, one of methylene groups constituting the ring may be replaced by a heteroatom such as an oxygen atom, a group including a heteroatom such as a carbonyl group, or a vinylidene group. In the cycloalkyl group, one or more ethylene groups constituting the cycloalkane ring may be replaced by vinylene groups.

The group represented by the formula (Y1) or the formula (Y2) preferably has a form in which, for example, Rx₁ is a methyl group or an ethyl group, and Rx₂ and Rx₃ are bonded together to form the above-described cycloalkyl group.

When the composition of the present invention is, for example, a resist composition used for EUV exposure, the alkyl groups, the cycloalkyl groups, the alkenyl groups, and the aryl groups represented by Rx₁ to Rx₃ and the ring formed by bonding together two among Rx₁ to Rx₃ also preferably further have, as a substituent, a fluorine atom or an iodine atom.

In the formula (Y3), R₃₆ to R₃₈ each independently represent a hydrogen atom or a monovalent organic group. R₃₇ and R₃₈ may be bonded together to form a ring. The monovalent organic group may be an alkyl group, a cycloalkyl group, an aryl group, an aralkyl group, or an alkenyl group. R₃₆ is also preferably a hydrogen atom.

Note that the alkyl group, the cycloalkyl group, the aryl group, and the aralkyl group may include a heteroatom such as an oxygen atom and/or a group including a heteroatom such as a carbonyl group. For example, in the alkyl group, the cycloalkyl group, the aryl group, and the aralkyl group, one or more methylene groups may be replaced by a heteroatom such as an oxygen atom and/or a group including a heteroatom such as a carbonyl group.

R₃₈ and another substituent of the main chain of the repeating unit may be bonded together to form a ring. The group formed by bonding together R₃₈ and another substituent of the main chain of the repeating unit is preferably an alkylene group such as a methylene group.

When the composition of the present invention is, for example, a resist composition used for EUV exposure, the monovalent organic groups represented by R₃₆ to R₃₈ and the ring formed by bonding together R₃₇ and R₃₈ also preferably further have, as a substituent, a fluorine atom or an iodine atom.

The formula (Y3) is preferably a group represented by the following formula (Y3-1).

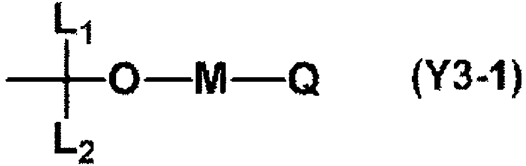

L₁ and L₂ each independently represent a hydrogen atom, an alkyl group, a cycloalkyl group, an aryl group, or a group that is a combination of the foregoing (for example, a group that is a combination of an alkyl group and an aryl group).

M represents a single bond or a divalent linking group.

Q represents an alkyl group that may include a heteroatom, a cycloalkyl group that may include a heteroatom, an aryl group that may include a heteroatom, an amino group, an ammonium group, a mercapto group, a cyano group, an aldehyde group, or a group that is a combination of the foregoing (for example, a group that is a combination of an alkyl group and a cycloalkyl group).

In the alkyl group and the cycloalkyl group, for example, one of methylene groups may be replaced by a heteroatom such as an oxygen atom or a group including a heteroatom such as a carbonyl group.

Note that one of L₁ and L₂ is preferably a hydrogen atom and the other is preferably an alkyl group, a cycloalkyl group, an aryl group, or a group that is a combination of an alkylene group and an aryl group.

At least two among Q, M, and L₁ may be bonded together to form a ring (preferably a 5-membered or 6-membered ring).

From the viewpoint of forming finer patterns, L₂ is preferably a secondary or tertiary alkyl group, and more preferably a tertiary alkyl group. Examples of the secondary alkyl group include an isopropyl group, a cyclohexyl group, and a norbornyl group; examples of the tertiary alkyl group include a tert-butyl group and an adamantane group. In such examples, Tg (glass transition temperature) and activation energy are increased, so that film hardness is ensured and fogging can be suppressed.

When the composition of the present invention is, for example, a resist composition used for EUV exposure, the alkyl groups, the cycloalkyl groups, the aryl groups, and the groups that are combinations of the foregoing represented by L₁ and L₂ also preferably further have, as a substituent, a fluorine atom or an iodine atom. The alkyl groups, the cycloalkyl groups, the aryl groups, and the aralkyl groups also preferably include, in addition to a fluorine atom and an iodine atom, a heteroatom such as an oxygen atom. Specifically, in the alkyl groups, the cycloalkyl groups, the aryl groups, and the aralkyl groups, for example, one of methylene groups may be replaced by a heteroatom such as an oxygen atom or a group including a heteroatom such as a carbonyl group.

When the resist composition is, for example, a resist composition used for EUV exposure, in the alkyl group that may include a heteroatom, the cycloalkyl group that may include a heteroatom, the aryl group that may include a heteroatom, the amino group, the ammonium group, the mercapto group, the cyano group, the aldehyde group, and the group that is a combination of the foregoing represented by Q, such a heteroatom is also preferably a heteroatom selected from the group consisting of a fluorine atom, an iodine atom, and an oxygen atom.

In the formula (Y4), Ar represents an aromatic ring group. Rn represents an alkyl group, a cycloalkyl group, or an aryl group. Rn and Ar may be bonded together to form a non-aromatic ring. Ar is preferably an aryl group.

When the resist composition is, for example, a resist composition used for EUV exposure, the aromatic ring group represented by Ar and the alkyl group, the cycloalkyl group, and the aryl group represented by Rn also preferably have, as a substituent, a fluorine atom or an iodine atom.

From the viewpoint of providing a repeating unit having high acid-decomposability, in the leaving group protecting the polar group, when a non-aromatic ring is directly bonded to the polar group (or its residue), in the non-aromatic ring, a ring-member atom adjacent to a ring-member atom directly bonded to the polar group (or its residue) also preferably does not have, as a substituent, a halogen atom such as a fluorine atom.

Alternatively, the group that leaves by the action of an acid may be a 2-cyclopentenyl group having a substituent (such as an alkyl group) such as a 3-methyl-2-cyclopentenyl group, or a cyclohexyl group having a substituent (such as an alkyl group) such as a 1,1,4,4-tetramethylcyclohexyl group.

The repeating unit having an acid-decomposable group is also preferably a repeating unit represented by a formula (A).

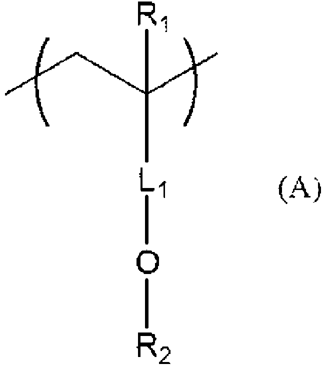

L₁ represents a divalent linking group that may have a fluorine atom or an iodine atom; R₁ represents a hydrogen atom, a fluorine atom, an iodine atom, an alkyl group that may have a fluorine atom or an iodine atom, or an aryl group that may have a fluorine atom or an iodine atom; R₂ represents a leaving group that leaves by the action of an acid and that may have a fluorine atom or an iodine atom. Note that at least one of L₁, R₁, or R₂ has a fluorine atom or an iodine atom.

Examples of the divalent linking group that is represented by L₁ and may have a fluorine atom or an iodine atom include -CO-, -O-, -S-, -SO-, -SO₂-, hydrocarbon groups that may have a fluorine atom or an iodine atom (for example, alkylene groups, cycloalkylene groups, alkenylene groups, and arylene groups), and linking groups provided by linking together a plurality of the foregoing. In particular, L₁ is preferably -CO-, an arylene group, or an -arylene group-alkylene group having a fluorine atom or an iodine atom-, and more preferably -CO- or an -arylene group-alkylene group having a fluorine atom or an iodine atom-.

The arylene group is preferably a phenylene group.

The alkylene group may be linear or may be branched. The number of carbon atoms of the alkylene group is not particularly limited, but is preferably 1 to 10, and more preferably 1 to 3.

In the alkylene group having a fluorine atom or an iodine atom, the total number of fluorine atoms and iodine atoms included is not particularly limited, but is preferably 2 or more, more preferably 2 to 10, and still more preferably 3 to 6.

The alkyl group represented by R₁ may be linear or may be branched. The number of carbon atoms of the alkyl group is not particularly limited, but is preferably 1 to 10, and more preferably 1 to 3.

In the alkyl group represented by R₁ and having a fluorine atom or an iodine atom, the total number of fluorine atoms and iodine atoms included is not particularly limited, but is preferably 1 or more, more preferably 1 to 5, and still more preferably 1 to 3.

The alkyl group represented by R₁ may include a heteroatom other than halogen atoms such as an oxygen atom.

Examples of the leaving group that is represented by R₂ and may have a fluorine atom or an iodine atom include leaving groups that are represented by the above-described formulas (Y1) to (Y4) and that have a fluorine atom or an iodine atom.

The repeating unit having an acid-decomposable group is also preferably a repeating unit represented by a formula (AI).

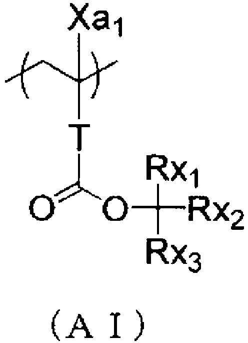

In the formula (AI), Xa₁ represents a hydrogen atom or an alkyl group that may have a substituent. T represents a single bond or a divalent linking group. Rx₁ to Rx₃ each independently represent an alkyl group (linear or branched), a cycloalkyl group (monocyclic or polycyclic), an alkenyl group (linear or branched), or an aryl (monocyclic or polycyclic) group. Note that, when Rx₁ to Rx₃ are all alkyl groups (linear or branched), at least two among Rx₁ to Rx₃ are preferably methyl groups.

Two among Rx₁ to Rx₃ may be bonded together to form a monocycle or polycycle (such as a monocyclic or polycyclic cycloalkyl group).

The alkyl group that is represented by Xa₁ and may have a substituent may be, for example, a methyl group or a group represented by -CH₂-R₁₁. R₁₁ represents a halogen atom (such as a fluorine atom), a hydroxy group, or a monovalent organic group. The monovalent organic group represented by R₁₁ is, for example, an alkyl group that has 5 or less carbon atoms and that may be substituted with a halogen atom, an acyl group that has 5 or less carbon atoms and that may be substituted with a halogen atom, or an alkoxy group that has 5 or less carbon atoms and that may be substituted with a halogen atom, and is preferably an alkyl group having 3 or less carbon atoms, and more preferably a methyl group. Xa₁ is preferably a hydrogen atom, a methyl group, a trifluoromethyl group, or a hydroxymethyl group.

For T, the divalent linking group may be an alkylene group, an aromatic ring group, a -COO-Rt- group, or an -O-Rt- group. In the formulas, Rt represent an alkylene group or a cycloalkylene group.

T is preferably a single bond or a -COO-Rt- group. When T represents a -COO-Rt- group, Rt is preferably an alkylene group having 1 to 5 carbon atoms, and more preferably a -CH₂-group, a -(CH₂)₂- group, or a -(CH₂)₃- group.

For Rx₁ to Rx₃, the alkyl group is preferably an alkyl group having 1 to 4 carbon atoms such as a methyl group, an ethyl group, an n-propyl group, an isopropyl group, an n-butyl group, an isobutyl group, or a t-butyl group.

For Rx₁ to Rx₃, the cycloalkyl group is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group.

For Rx₁ to Rx₃, the aryl group is preferably an aryl group having 6 to 10 carbon atoms and may be, for example, a phenyl group, a naphthyl group, or an anthryl group.

For Rx₁ to Rx₃, the alkenyl group is preferably a vinyl group.

The cycloalkyl group formed by bonding together two among Rx₁ to Rx₃ is preferably a monocyclic cycloalkyl group such as a cyclopentyl group or a cyclohexyl group. Also preferred are polycyclic cycloalkyl groups such as a norbornyl group, a tetracyclodecanyl group, a tetracyclododecanyl group, and an adamantyl group. In particular, preferred is a monocyclic cycloalkyl group having 5 to 6 carbon atoms.

In the cycloalkyl group formed by bonding together two among Rx₁ to Rx₃, for example, one of methylene groups constituting the ring may be replaced by a heteroatom such as an oxygen atom, a group including a heteroatom such as a carbonyl group, or a vinylidene group. In the cycloalkyl group, one or more of the ethylene groups constituting the cycloalkane ring may be replaced by vinylene groups.

The repeating unit represented by the formula (AI) preferably has a form in which, for example, Rx₁ is a methyl group or an ethyl group, and Rx₂ and Rx₃ are bonded together to form the above-described cycloalkyl group.

When the above-described groups each have a substituent, examples of the substituent include alkyl groups (having 1 to 4 carbon atoms), halogen atoms, a hydroxyl group, alkoxy groups (having 1 to 4 carbon atoms), a carboxyl group, and alkoxycarbonyl groups (having 2 to 6 carbon atoms). The substituent preferably has 8 or less carbon atoms.

The repeating unit represented by the formula (AI) is preferably an acid-decomposable (meth)acrylic acid tertiary alkyl ester-based repeating unit (the repeating unit where Xa₁ represents a hydrogen atom or a methyl group and T represents a single bond).

The resin (A) may have, as a repeating unit having an acid-decomposable group, a repeating unit having an acid-decomposable group including an unsaturated bond.

The repeating unit having an acid-decomposable group including an unsaturated bond is preferably a repeating unit represented by a formula (B).

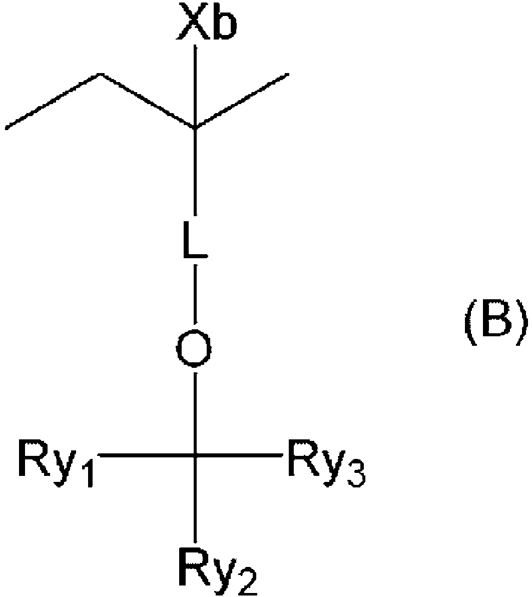

In the formula (B), Xb represents a hydrogen atom, a halogen atom, or an alkyl group that may have a substituent. L represents a single bond or a divalent linking group that may have a substituent. Ry₁ to Ry₃ each independently represent a linear or branched alkyl group, a monocyclic or polycyclic cycloalkyl group, an alkenyl group, an alkynyl group, or a monocyclic or polycyclic aryl group. Note that at least one of Ry₁ to Ry₃ represents an alkenyl group, an alkynyl group, a monocyclic or polycyclic cycloalkenyl group, or a monocyclic or polycyclic aryl group.

Two among Ry₁ to Ry₃ may be bonded together to form a monocycle or polycycle (such as a monocyclic or polycyclic cycloalkyl group or cycloalkenyl group).

For Xb, the alkyl group that may have a substituent may be, for example, a methyl group or a group represented by -CH₂-R₁₁. R₁₁ represents a halogen atom (such as a fluorine atom), a hydroxy group, or a monovalent organic group, may be, for example, an alkyl group that has 5 or less carbon atoms and that may be substituted with a halogen atom, an acyl group that has 5 or less carbon atoms and that may be substituted with a halogen atom, or an alkoxy group that has 5 or less carbon atoms and that may be substituted with a halogen atom, is preferably an alkyl group having 3 or less carbon atoms, and more preferably a methyl group. Xb is preferably a hydrogen atom, a fluorine atom, a methyl group, a trifluoromethyl group, or a hydroxymethyl group.

For L, the divalent linking group may be an -Rt- group, a -CO- group, a -COO-Rt-group, a -COO-Rt-CO- group, an -Rt-CO- group, or an -O-Rt- group. In the formulas, Rt represent an alkylene group, a cycloalkylene group, or an aromatic ring group, and is preferably an aromatic ring group.

L is preferably an -Rt- group, a -CO- group, a -COO-Rt-CO- group, or an -Rt-CO- group. Rt may have a substituent such as a halogen atom, a hydroxy group, or an alkoxy group.

For Ry₁ to Ry₃, the alkyl group is preferably an alkyl group having 1 to 4 carbon atoms such as a methyl group, an ethyl group, an n-propyl group, an isopropyl group, an n-butyl group, an isobutyl group, or a t-butyl group.

For Ry₁ to Ry₃, the cycloalkyl group is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group.

For Ry₁ to Ry₃, the aryl group is preferably an aryl group having 6 to 10 carbon atoms, and may be, for example, a phenyl group, a naphthyl group, or an anthryl group.

For Ry₁ to Ry₃, the alkenyl group is preferably a vinyl group.

For Ry₁ to Ry₃, the alkynyl group is preferably an ethynyl group.

For Ry₁ to Ry₃, the cycloalkenyl group is preferably a structure in which a monocyclic cycloalkyl group such as a cyclopentyl group or a cyclohexyl group includes partially a double bond.

The cycloalkyl group formed by bonding together two among Ry₁ to Ry₃ is preferably a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group. In particular, more preferred is a monocyclic cycloalkyl group having 5 to 6 carbon atoms.

In the cycloalkyl group or the cycloalkenyl group formed by bonding together two among Ry₁ to Ry₃, for example, one of methylene groups constituting the ring may be replaced by a heteroatom such as an oxygen atom, a group including a heteroatom such as a carbonyl group, a -SO₂- group, or a -SO₃- group, a vinylidene group, or a combination of the foregoing. In the cycloalkyl group or the cycloalkenyl group, one or more ethylene groups constituting the cycloalkane ring or the cycloalkene ring may be replaced by vinylene groups.

The repeating unit represented by the formula (B) preferably has a form in which, for example, Ry₁ is a methyl group, an ethyl group, a vinyl group, an allyl group, or an aryl group, and Ry₂ and Ry₃ are bonded together to form the above-described cycloalkyl group or cycloalkenyl group.

When the above-described groups each have a substituent, examples of the substituent include alkyl groups (having 1 to 4 carbon atoms), halogen atoms, a hydroxyl group, alkoxy groups (having 1 to 4 carbon atoms), a carboxyl group, and alkoxycarbonyl groups (having 2 to 6 carbon atoms). The substituent preferably has 8 or less carbon atoms.

The repeating unit represented by the formula (B) is preferably an acid-decomposable (meth)acrylic acid tertiary ester-based repeating unit (the repeating unit where Xb represents a hydrogen atom or a methyl group, and L represents a -CO- group), an acid-decomposable hydroxystyrene tertiary alkyl ether-based repeating unit (the repeating unit where Xb represents a hydrogen atom or a methyl group, and L represents a phenyl group), or an acid-decomposable styrenecarboxylic acid tertiary ester-based repeating unit (the repeating unit where Xb represents a hydrogen atom or a methyl group, and L represents an -Rt-CO- group (where Rt is an aromatic group)).

The content of the repeating unit having an acid-decomposable group including an unsaturated bond relative to all the repeating units in the resin (A) is preferably 15 mol% or more, more preferably 20 mol% or more, and still more preferably 30 mol% or more. The upper limit value relative to all the repeating units in the resin (A) is preferably 80 mol% or less, more preferably 70 mol% or less, and still more preferably 60 mol% or less.

The content of the repeating unit having an acid-decomposable group relative to all the repeating units in the resin (A) is preferably 15 mol% or more, more preferably 20 mol% or more, and still more preferably 30 mol% or more. The upper limit value relative to all the repeating units in the resin (A) is preferably 90 mol% or less, more preferably 80 mol% or less, still more preferably 70 mol% or less, and particularly preferably 60 mol% or less.

The resin (A) may include at least one repeating unit species selected from the group consisting of the following Group A and/or at least one repeating unit species selected from the group consisting of the following Group B.

Group A: a group consisting of the following repeating units (20) to (25)
(20) a repeating unit (described later) having an acid group
(21) a repeating unit (described later) not having an acid-decomposable group or an acid group, but having a fluorine atom, a bromine atom, or an iodine atom
(22) a repeating unit (described later) having a lactone group, a sultone group, or a carbonate group
(23) a repeating unit (described later) having a photoacid generation group
(24) a repeating unit (described later) represented by a formula (V-1) or a formula (V-2) below
(25) a repeating unit for lowering the mobility of the main chain

Note that repeating units represented by a formula (A) to a formula (E) described later correspond to the repeating unit (25) for lowering the mobility of the main chain.

Group B: a group consisting of the following repeating units (30) to (32)
(30) a repeating unit (described later) having at least one group species selected from the group consisting of a lactone group, a sultone group, a carbonate group, a hydroxy group, a cyano group, and an alkali-soluble group
(31) a repeating unit (described later) having an alicyclic hydrocarbon structure and not exhibiting acid-decomposability
(32) a repeating unit (described later) not having a hydroxy group or a cyano group and represented by a formula (III)

The resin (A) preferably has an acid group and preferably includes a repeating unit having an acid group as described later. Note that the definition of the acid group will be described in a later part together with preferred examples of the repeating unit having an acid group. When the resin (A) has an acid group, a better interaction between the resin (A) and the acid generated from the photoacid generator is provided. This results in further suppression of diffusion of the acid to form a pattern having a more square profile.

The resin (A) may have at least one repeating unit species selected from the group consisting of Group A above. When the composition of the present invention is used as an actinic ray-sensitive or radiation-sensitive resin composition used for EUV exposure, the resin (A) preferably has at least one repeating unit species selected from the group consisting of Group A above.

The resin (A) may include at least one of a fluorine atom or an iodine atom. When the resist composition is used as an actinic ray-sensitive or radiation-sensitive resin composition used for EUV exposure, the resin (A) preferably includes at least one of a fluorine atom or an iodine atom. When the resin (A) includes both of a fluorine atom and an iodine atom, the resin (A) may have a repeating unit including both of a fluorine atom and an iodine atom, or the resin (A) may include two species that are a repeating unit having a fluorine atom and a repeating unit including an iodine atom.

The resin (A) may have a repeating unit having an aromatic group. When the resist composition is used as an actinic ray-sensitive or radiation-sensitive resin composition used for EUV exposure, the resin (A) also preferably has a repeating unit having an aromatic group.

The resin (A) may also have at least one repeating unit species selected from the group consisting of Group B above. When the resist composition is used as an actinic ray-sensitive or radiation-sensitive resin composition used for ArF, the resin (A) preferably has at least one repeating unit species selected from the group consisting of Group B above.

Note that, when the composition of the present invention is used as an actinic ray-sensitive or radiation-sensitive resin composition used for ArF, the resin (A) preferably does not include a fluorine atom or a silicon atom.

When the composition of the present invention is used as an actinic ray-sensitive or radiation-sensitive resin composition used for ArF, the resin (A) preferably does not have an aromatic group.

### Repeating unit having acid group

The resin (A) may have a repeating unit having an acid group.

The acid group is preferably an acid group having a pKa of 13 or less. The acid group preferably has an acid dissociation constant of 13 or less, more preferably 3 to 13, and still more preferably 5 to 10.

When the resin (A) has an acid group having a pKa of 13 or less, the content of the acid group in the resin (A) is not particularly limited, but is often 0.2 to 6.0 mmol/g. In particular, preferred is 0.8 to 6.0 mmol/g, more preferred is 1.2 to 5.0 mmol/g, and still more preferred is 1.6 to 4.0 mmol/g. When the content of the acid group is within such a range, development suitably proceeds to form a pattern having a good profile at high resolution.

The acid group is preferably, for example, a carboxyl group, a phenolic hydroxyl group, a fluoroalcohol group (preferably a hexafluoroisopropanol group), a sulfonic acid group, a sulfonamide group, or an isopropanol group.

In the hexafluoroisopropanol group, one or more (preferably one to two) of the fluorine atoms may be substituted with groups other than fluorine atoms (such as alkoxycarbonyl groups).

The acid group is also preferably -C(CF₃)(OH)-CF₂- formed in this manner. Alternatively, one or more of the fluorine atoms may be substituted with groups other than fluorine atoms, to form a ring including -C(CF₃)(OH)-CF₂-.

The repeating unit having an acid group is preferably a repeating unit different from the above-described repeating unit having a structure in which a polar group is protected with a group that leaves by the action of an acid and repeating units described later and having a lactone group, a sultone group, or a carbonate group.

The repeating unit having an acid group may have a fluorine atom or an iodine atom.

Examples of the repeating unit having an acid group include the following repeating units.

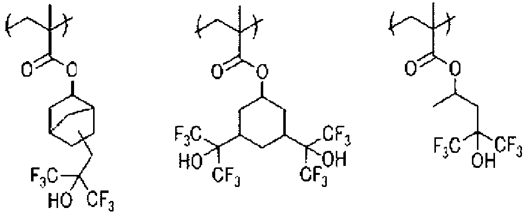

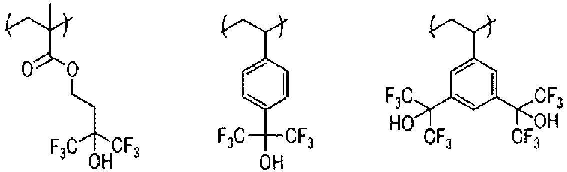

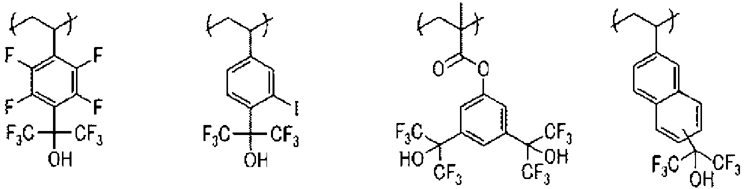

The repeating unit having an acid group is preferably a repeating unit represented by the following formula (1).

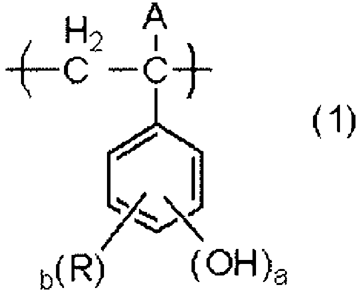

In the formula (1), A represents a hydrogen atom, an alkyl group, a cycloalkyl group, a halogen atom, or a cyano group. R represents a halogen atom, an alkyl group, a cycloalkyl group, an aryl group, an alkenyl group, an aralkyl group, an alkoxy group, an alkylcarbonyloxy group, an alkylsulfonyloxy group, an alkyloxycarbonyl group, or an aryloxycarbonyl group; when there are a plurality of R's, they may be the same or different. When there are a plurality of R's, they may together form a ring. R is preferably a hydrogen atom. a represents an integer of 1 to 3. b represents an integer of 0 to (5 - a).

The content of the repeating unit having an acid group relative to all the repeating units in the resin (A) is preferably 10 mol% or more, and more preferably 15 mol% or more. The upper limit value relative to all the repeating units in the resin (A) is preferably 70 mol% or less, more preferably 65 mol% or less, and still more preferably 60 mol% or less.

### Repeating unit not having acid-decomposable group or acid group, but having fluorine atom, bromine atom, or iodine atom

The resin (A) may have, in addition to the above-described <repeating unit having an acid-decomposable group> and <repeating unit having an acid group>, a repeating unit not having an acid-decomposable group or an acid group, but having a fluorine atom, a bromine atom, or an iodine atom (hereafter, also referred to as unit X). This <repeating unit not having an acid-decomposable group or an acid group, but having a fluorine atom, a bromine atom, or an iodine atom> is preferably different from other repeating unit species belonging to Group A such as the <repeating unit having a lactone group, a sultone group, or a carbonate group> and the <repeating unit having a photoacid generation group> described later.

The unit X is preferably a repeating unit represented by a formula (C).

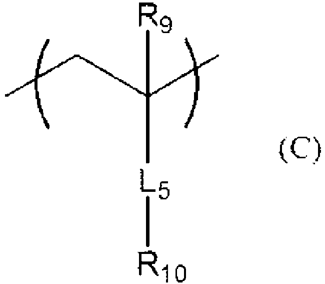

L₅ represents a single bond or an ester group. R₉ represents a hydrogen atom or an alkyl group that may have a fluorine atom or an iodine atom. R₁₀ represents a hydrogen atom, an alkyl group that may have a fluorine atom or an iodine atom, a cycloalkyl group that may have a fluorine atom or an iodine atom, an aryl group that may have a fluorine atom or an iodine atom, or a group that is a combination of the foregoing.

The following are examples of the repeating unit having a fluorine atom or an iodine atom.

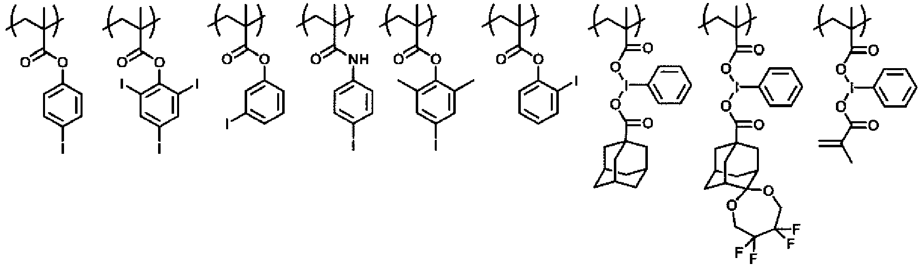

The unit X content relative to all the repeating units in the resin (A) is preferably 0 mol% or more, more preferably 5 mol% or more, and still more preferably 10 mol% or more. The upper limit value relative to all the repeating units in the resin (A) is preferably 50 mol% or less, more preferably 45 mol% or less, and still more preferably 40 mol% or less.

Of the repeating units of the resin (A), the total content of the repeating unit including at least one of a fluorine atom, a bromine atom, or an iodine atom relative to all the repeating units of the resin (A) is preferably 10 mol% or more, more preferably 20 mol% or more, still more preferably 30 mol% or more, and particularly preferably 40 mol% or more. The upper limit value is not particularly limited, but is, for example, relative to all the repeating units of the resin (A), 100 mol% or less.

Note that examples of the repeating unit including at least one of a fluorine atom, a bromine atom, or an iodine atom include a repeating unit having a fluorine atom, a bromine atom, or an iodine atom and having an acid-decomposable group, a repeating unit having a fluorine atom, a bromine atom, or an iodine atom and having an acid group, and a repeating unit having a fluorine atom, a bromine atom, or an iodine atom.

### Repeating unit having lactone group, sultone group, or carbonate group

The resin (A) may have a repeating unit having at least one species selected from the group consisting of a lactone group, a sultone group, and a carbonate group (hereafter, also referred to as "unit Y").

The unit Y also preferably does not have acid groups such as a hydroxy group and a hexafluoropropanol group.

The lactone group or the sultone group has a lactone structure or a sultone structure. The lactone structure or the sultone structure is preferably a 5- to 7-membered lactone structure or a 5- to 7-membered sultone structure. In particular, more preferred is a 5- to 7-membered lactone structure to which another ring structure is fused so as to form a bicyclo structure or a spiro structure, or a 5- to 7-membered sultone structure to which another ring structure is fused so as to form a bicyclo structure or a spiro structure.

The resin (A) preferably has a repeating unit having a lactone group or a sultone group provided by withdrawing, from ring-member atoms of the lactone structure represented by any one of formulas (LC1-1) to (LC1-21) below or the sultone structure represented by any one of formulas (SL1-1) to (SL1-3) below, one or more hydrogen atoms, and a lactone group or a sultone group may be directly bonded to the main chain. For example, ring-member atoms of a lactone group or a sultone group may constitute the main chain of the resin (A).

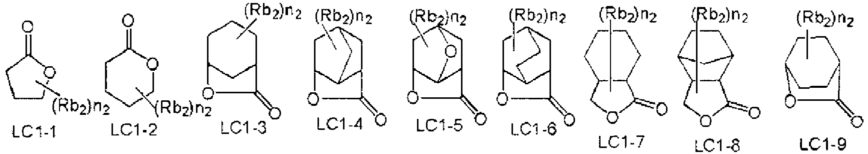

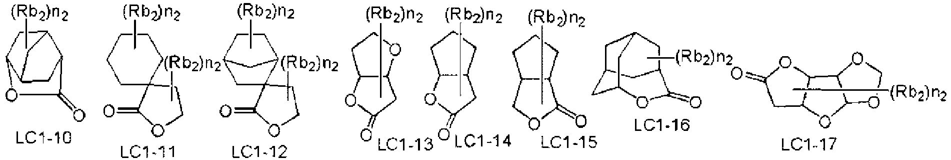

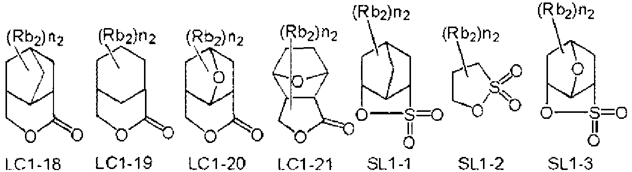

The lactone structure or the sultone structure may have a substituent (Rb₂). Preferred examples of the substituent (Rb₂) include an alkyl group having 1 to 8 carbon atoms, a cycloalkyl group having 4 to 7 carbon atoms, an alkoxy group having 1 to 8 carbon atoms, an alkoxycarbonyl group having 1 to 8 carbon atoms, a carboxyl group, a halogen atom, a cyano group, and an acid-decomposable group. n₂ represent an integer of 0 to 4. When n₂ is 2 or more, the plurality of Rb₂'s present may be different, and the plurality of Rb₂'s present may be bonded together to form a ring.

The repeating unit having a group including the lactone structure represented by any one of the formulas (LC1-1) to (LC1-21) or the sultone structure represented by any one of the formulas (SL1-1) to (SL1-3) may be, for example, a repeating unit represented by the following formula (AI).

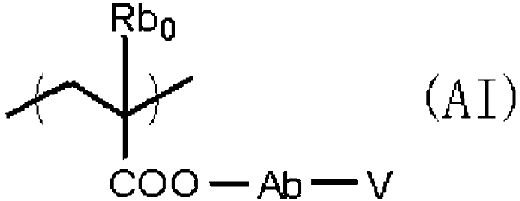

In the formula (AI), Rb₀ represents a hydrogen atom, a halogen atom, or an alkyl group having 1 to 4 carbon atoms. Preferred examples of the substituent that the alkyl group of Rb₀ may have include a hydroxy group and a halogen atom.

Examples of the halogen atom of Rb₀ include a fluorine atom, a chlorine atom, a bromine atom, and an iodine atom. Rb₀ is preferably a hydrogen atom or a methyl group.

Ab represents a single bond, an alkylene group, a divalent linking group having a monocyclic or polycyclic alicyclic hydrocarbon structure, an ether group, an ester group, a carbonyl group, a carboxyl group, or a divalent linking group that is a combination of the foregoing. In particular, Ab is preferably a single bond or a linking group represented by -Ab₁-CO₂-. Ab₁ is a linear or branched alkylene group or a monocyclic or polycyclic cycloalkylene group, and preferably a methylene group, an ethylene group, a cyclohexylene group, an adamantylene group, or a norbornylene group.

V represents a group formed by withdrawing, from a ring-member atom of the lactone structure represented by any one of the formulas (LC1-1) to (LC1-21), a single hydrogen atom, or a group formed by withdrawing, from a ring-member atom of the sultone structure represented by any one of the formulas (SL1-1) to (SL1-3), a single hydrogen atom.

When the repeating unit having a lactone group or a sultone group has an optical isomer, any optical isomer may be used. A single optical isomer may be used alone, or a plurality of optical isomers may be used in combination. In the case of mainly using one of the optical isomers, its optical purity (ee) is preferably 90 or more, and more preferably 95 or more.

The carbonate group is preferably a cyclic carbonic acid ester group.

The repeating unit having a cyclic carbonic acid ester group is preferably a repeating unit represented by the following formula (A-1).

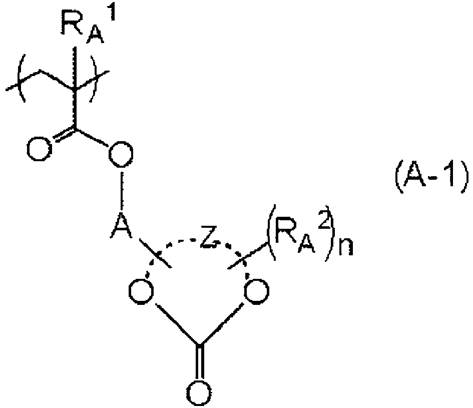

In the formula (A-1), R_{A}¹ represents a hydrogen atom, a halogen atom, or a monovalent organic group (preferably a methyl group). n represents an integer of 0 or more. R_{A}² represents a substituent. When n is 2 or more, the plurality of R_{A}²'s present may be the same or different. A represents a single bond or a divalent linking group. The divalent linking group is preferably an alkylene group, a divalent linking group having a monocyclic or polycyclic alicyclic hydrocarbon structure, an ether group, an ester group, a carbonyl group, a carboxyl group, or a divalent linking group that is a combination of the foregoing. Z represents an atomic group that forms, together with the group represented by -O-CO-O- in the formula, a monocycle or a polycycle.

The unit Y content relative to all the repeating units in the resin (A) is preferably 1 mol% or more, and more preferably 10 mol% or more. The upper limit value relative to all the repeating units in the resin (A) is preferably 85 mol% or less, more preferably 80 mol% or less, still more preferably 70 mol% or less, and particularly preferably 60 mol% or less.

### Repeating unit having photoacid generation group

The resin (A) may have, as a repeating unit other than the above-described repeating units, a repeating unit having a group that generates an acid upon irradiation with an actinic ray or a radiation (hereafter, also referred to as "photoacid generation group").

The repeating unit having a photoacid generation group may be a repeating unit represented by a formula (4).

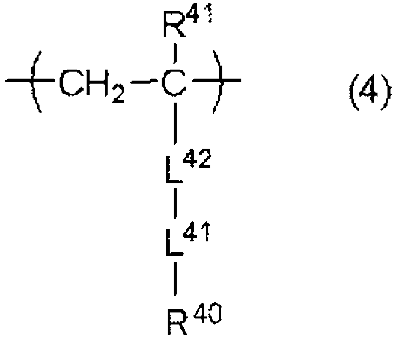

R⁴¹ represents a hydrogen atom or a methyl group. L⁴¹ represents a single bond or a divalent linking group. L⁴² represents a divalent linking group. R⁴⁰ represents a structural moiety that is decomposed upon irradiation with an actinic ray or a radiation to generate an acid in the side chain.

The following are examples of the repeating unit having a photoacid generation group.

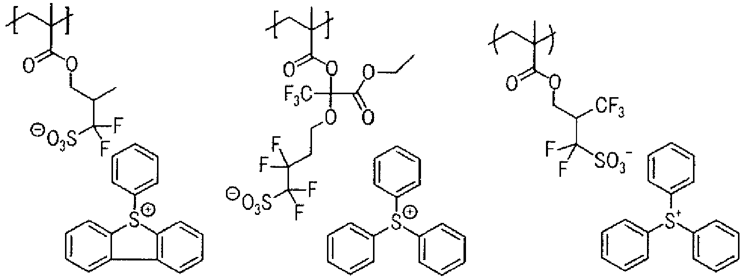

Other examples of the repeating unit represented by the formula (4) include the repeating units described in Paragraphs [0094] to [0105] of JP2014-041327A and the repeating units described in Paragraph [0094] of WO2018/193954A.

The content of the repeating unit having a photoacid generation group relative to all the repeating units in the resin (A) is preferably 1 mol% or more, and more preferably 5 mol% or more. The upper limit value relative to all the repeating units in the resin (A) is preferably 40 mol% or less, more preferably 35 mol% or less, and still more preferably 30 mol% or less.

### Repeating unit represented by formula (V-1) or formula (V-2) below

The resin (A) may have a repeating unit represented by a formula (V-1) below or a formula (V-2) below.

The repeating unit represented by the formula (V-1) below or the formula (V-2) below is preferably a repeating unit different from the above-described repeating units.

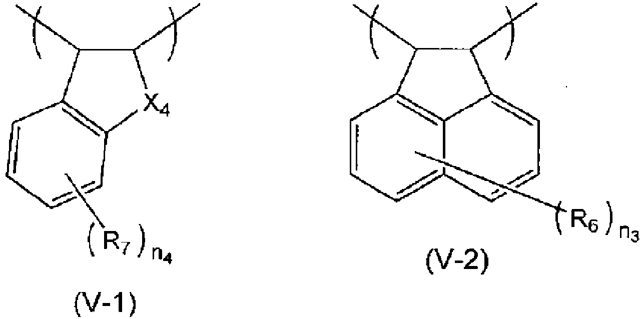

In the formulas,
R₆ and R₇ each independently represent a hydrogen atom, a hydroxyl group, an alkyl group, an alkoxy group, an acyloxy group, a cyano group, a nitro group, an amino group, a halogen atom, an ester group (-OCOR or -COOR: R is an alkyl group or fluorinated alkyl group having 1 to 6 carbon atoms), or a carboxyl group. The alkyl group is preferably a linear, branched, or cyclic alkyl group having 1 to 10 carbon atoms.
n₃ represents an integer of 0 to 6.
n₄ represents an integer of 0 to 4.
X₄ is a methylene group, an oxygen atom, or a sulfur atom.

The following are examples of the repeating unit represented by the formula (V-1) or (V-2).

Examples of the repeating unit represented by the formula (V-1) or (V-2) include the repeating units described in Paragraph [0100] of WO2018/193954A.

### Repeating unit for lowering mobility of main chain

The resin (A) preferably has, from the viewpoint of suppressing excessive diffusion of the generated acid or pattern collapse during development, a relatively high glass transition temperature (Tg). Tg is preferably more than 90°C, more preferably more than 100°C, still more preferably more than 110°C, and particularly preferably more than 125°C. Note that, from the viewpoint of having a high dissolution rate in developers, Tg is preferably 400°C or less, and more preferably 350°C or less.

Note that, in this Specification, the glass transition temperatures (Tg) of polymers such as the resin (A) (hereafter, "Tg's of repeating units") are calculated in the following manner. First, for the repeating units included in a polymer, the Tg's of homopolymers composed only of the repeating units are individually calculated by the Bicerano method. Subsequently, the mass ratios (%) of the repeating units relative to all the repeating units in the polymer are calculated. Subsequently, the Fox equation (described in Materials Letters 62 (2008) 3152, for example) is used to calculate Tg's for the mass ratios and the Tg's are summed up to determine the Tg(°C) of the polymer.

The Bicerano method is described in Prediction of polymer properties, Marcel Dekker Inc, New York (1993). The calculation of Tg by the Bicerano method can be performed using a software for estimating properties of polymers, MDL Polymer (MDL Information Systems, Inc.).

In order to increase the Tg of the resin (A) (preferably, making Tg be more than 90°C), the mobility of the main chain of the resin (A) is preferably lowered. Examples of the method for lowering the mobility of the main chain of the resin (A) include the following methods (a) to (e):
(a) introduction of a bulky substituent to the main chain;
(b) introduction of a plurality of substituents to the main chain;
(c) introduction of a substituent that induces interaction between the resins (A), to the vicinity of the main chain;
(d) formation of the main chain using a ring structure; and
(e) linkage of a ring structure to the main chain.

Note that the resin (A) preferably has a repeating unit whose homopolymer has a Tg of 130°C or more.

Note that the repeating unit species whose homopolymer has a Tg of 130°C or more is not particularly limited and is a repeating unit whose homopolymer has a Tg of 130°C or more calculated by the Bicerano method. Note that the repeating units represented by a formula (A) to a formula (E) described later may, depending on the functional group species, belong to the repeating unit whose homopolymer has a Tg of 130°C or more.

An example of specific means for achieving (a) above is a method of introducing, into the resin (A), a repeating unit represented by a formula (A).

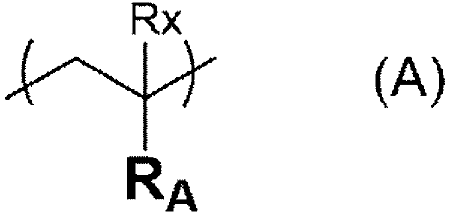

For the formula (A), R_{A} represents a group including a polycyclic structure. Rₓ represents a hydrogen atom, a methyl group, or an ethyl group. The group including a polycyclic structure is a group including a plurality of cyclic structures; the plurality of cyclic structures may be fused together or may not be fused together.

Specific examples of the repeating unit represented by the formula (A) include those described in Paragraphs [0107] to [0119] of WO2018/193954A.

An example of specific means for achieving (b) above is a method of introducing, into the resin (A), a repeating unit represented by a formula (B).

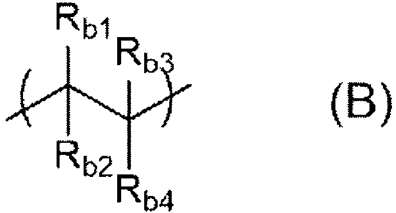

In the formula (B), R_{b1} to R_{b4} each independently represent a hydrogen atom or an organic group; at least two or more among R_{b1} to R_{b4} represent organic groups.

When at least one of the organic groups is a group whose cyclic structure is directly linked to the main chain in the repeating unit, the other organic group species is not particularly limited.

When none of the organic groups is a group whose cyclic structure is directly linked to the main chain in the repeating unit, at least two or more among the organic groups are substituents having three or more constituent atoms (except for hydrogen atoms).

Specific examples of the repeating unit represented by the formula (B) include those described in Paragraphs [0113] to [0115] of WO2018/193954A.

An example of specific means for achieving (c) above is a method of introducing, into the resin (A), a repeating unit represented by a formula (C).

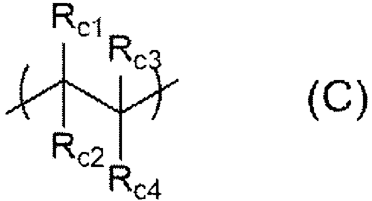

In the formula (C), R_{c1} to R_{c4} each independently represent a hydrogen atom or an organic group; at least one of R_{c1} to R_{c4} is a group including a hydrogen-bond-forming hydrogen atom positioned within three atoms from the carbon atom in the main chain. In particular, from the viewpoint of inducing the interaction between the main chains of the resin (A), it preferably has a hydrogen-bond-forming hydrogen atom positioned within two atoms (closer to the main chain side).

Specific examples of the repeating unit represented by the formula (C) include those described in Paragraphs [0119] to [0121] of WO2018/193954A.

An example of specific means for achieving (d) above is a method of introducing, into the resin (A), a repeating unit represented by a formula (D).

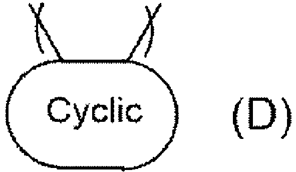

In the formula (D), "Cyclic" denotes a group having a ring structure and forming the main chain. The number of atoms constituting the ring is not particularly limited.

Specific examples of the repeating unit represented by the formula (D) include those described in Paragraphs [0126] to [0127] of WO2018/193954A.

An example of specific means for achieving (e) above is a method of introducing, into the resin (A), a repeating unit represented by a formula (E).

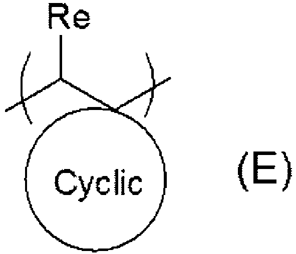

In the formula (E), Re each independently represent a hydrogen atom or an organic group. Examples of the organic group include alkyl groups, cycloalkyl groups, aryl groups, aralkyl groups, and alkenyl groups that may have substituents.

"Cyclic" is a cyclic group including a carbon atom of the main chain. The number of atoms included in the cyclic group is not particularly limited.

Specific examples of the repeating unit represented by the formula (E) include those described in Paragraphs [0131] to [0133] of WO2018/193954A.

### Repeating unit having at least one group species selected from the group consisting of lactone group, sultone group, carbonate group, hydroxy group, cyano group, and alkali-soluble group

The resin (A) may have a repeating unit having at least one group species selected from the group consisting of a lactone group, a sultone group, a carbonate group, a hydroxy group, a cyano group, and an alkali-soluble group.

In the resin (A), the repeating unit having a lactone group, a sultone group, or a carbonate group may be the repeating unit having been described in <Repeating unit having lactone group, sultone group, or carbonate group>. Preferred contents are also the same as those having been described in <Repeating unit having lactone group, sultone group, or carbonate group>.

The resin (A) may have a repeating unit having a hydroxy group or a cyano group. This results in improvement in adhesiveness to the substrate and affinity for the developer.

The repeating unit having a hydroxy group or a cyano group is preferably a repeating unit having an alicyclic hydrocarbon structure substituted with a hydroxy group or a cyano group.

The repeating unit having a hydroxy group or a cyano group preferably does not have an acid-decomposable group. Examples of the repeating unit having a hydroxy group or a cyano group include those described in Paragraphs [0081] to [0084] of JP2014-098921A.

The resin (A) may have a repeating unit having an alkali-soluble group.

The alkali-soluble group may be a carboxyl group, a sulfonamide group, a sulfonylimide group, a bissulfonylimide group, or an aliphatic alcohol group substituted, at the α position, with an electron-withdrawing group (for example, a hexafluoroisopropanol group), and is preferably a carboxyl group. When the resin (A) includes the repeating unit having an alkali-soluble group, increased resolution is provided in the contact hole application. Examples of the repeating unit having an alkali-soluble group include those described in Paragraphs [0085] and [0086] of JP2014-098921A.

### Repeating unit having alicyclic hydrocarbon structure and not exhibiting acid-decomposability

The resin (A) may have a repeating unit having an alicyclic hydrocarbon structure and not exhibiting acid-decomposability. This results in, during liquid immersion exposure, a reduction in leaching of, from the resist film to the immersion liquid, low-molecular-weight components. Examples of the repeating unit having an alicyclic hydrocarbon structure and not exhibiting acid-decomposability include a repeating unit derived from 1-adamantyl (meth)acrylate, diadamantyl (meth)acrylate, tricyclodecanyl (meth)acrylate, or cyclohexyl (meth)acrylate.

### Repeating unit not having hydroxy group or cyano group and represented by formula (III)

The resin (A) may have a repeating unit not having a hydroxy group or a cyano group and represented by a formula (III).

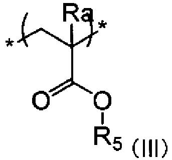

In the formula (III), R₅ represents a hydrocarbon group having at least one ring structure and not having a hydroxy group or a cyano group.

Ra represents a hydrogen atom, an alkyl group, or a -CH₂-O-Ra₂ group. In the formula, Ra₂ represents a hydrogen atom, an alkyl group, or an acyl group.

Examples of the repeating unit not having a hydroxy group or a cyano group and represented by the formula (III) include those described in Paragraphs [0087] to [0094] of JP2014-098921A.

### Other repeating unit

Furthermore, the resin (A) may have another repeating unit other than the above-described repeating units.

For example, the resin (A) may have a repeating unit selected from the group consisting of a repeating unit having an oxathiane ring group, a repeating unit having an oxazolone ring group, a repeating unit having a dioxane ring group, and a repeating unit having a hydantoin ring group.

The resin (A) may have, in addition to the above-described repeating structural units, various repeating structural units for the purpose of adjusting, for example, dry etching resistance, standard developer droplet property, substrate adhesiveness, resist profile, resolution, heat resistance, and sensitivity.

For the resin (A), particularly when the composition is used as an actinic ray-sensitive or radiation-sensitive resin composition used for ArF, all the repeating units are preferably constituted by a repeating unit derived from a compound having an ethylenically unsaturated bond. In particular, all the repeating units are also preferably constituted by a (meth)acrylate-based repeating unit. In the case in which all the repeating units are constituted by a (meth)acrylate-based repeating unit, all the repeating units may be a methacrylate-based repeating unit, all the repeating units may be an acrylate-based repeating unit, or all the repeating units may be a methacrylate-based repeating unit and an acrylate-based repeating unit; the acrylate-based repeating unit content relative to all the repeating units is preferably 50 mol% or less.

The resin (A) can be synthesized by standard procedures (for example, radical polymerization).

The resin (A) has a weight-average molecular weight of, as a polystyrene-equivalent value determined by GPC method, preferably 30,000 or less, more preferably 1,000 to 30,000, still more preferably 3,000 to 30,000, and particularly preferably 5,000 to 15,000.

The resin (A) has a dispersity (molecular weight distribution) of preferably 1 to 5, more preferably 1 to 3, still more preferably 1.2 to 3.0, and particularly preferably 1.2 to 2.0. As the dispersity lowers, the resolution becomes higher, the resist profile becomes better, the sidewalls of the resist pattern become smoother, and the roughness performance becomes higher.

In the composition of the present invention, the content of the resin (A) is, relative to the total solid content of the composition, preferably 20.0 to 99.9 mass%, and more preferably 30.0 to 90.0 mass%.

### Such resins (A) may be used alone or in combination of two or more thereof. Photoacid generator

The composition of the present invention may include a compound (P) that is different from the compound (Q) and generates an acid upon irradiation with an actinic ray or a radiation (hereafter, also referred to as a photoacid generator, a photoacid generator (P), or a compound (P)).

The compound (P) is a compound not corresponding to the compound (Q), and preferably generates, upon irradiation with an actinic ray or a radiation, an acid stronger than an acid generated by the compound (Q).

The phrase "generate an acid stronger than an acid generated by the compound (Q)" means that the acid strength of the acid generated by the compound (P) is higher than the acid strength of the acid generated by the compound (Q), and typically means that the acid dissociation constant (pKa) of the acid generated by the compound (P) is lower than the pKa of the acid generated by the compound (Q).

The acid generated from the compound (P) ordinarily reacts with the acid-decomposable group in the resin (A).

The photoacid generator (P) may have the form of a low-molecular-weight compound, or the form of being incorporated into a portion of a polymer (for example, the above-described resin (A)). Alternatively, the form of a low-molecular-weight compound and the form of being incorporated into a portion of a polymer (for example, the above-described resin (A)) may be used in combination.

When the photoacid generator (P) has the form of a low-molecular-weight compound, the photoacid generator preferably has a molecular weight of 3000 or less, more preferably 2000 or less, and still more preferably 1000 or less. The lower limit is not particularly limited, but is preferably 100 or more.

When the photoacid generator (P) has the form of being incorporated into a portion of a polymer, it may be incorporated into a portion of the resin (A) or may be incorporated into a resin different from the resin (A).

In this Specification, the photoacid generator (P) preferably has the form of a low-molecular-weight compound.

The photoacid generator (P) may be, for example, a compound represented by "M⁺ X⁻ " (onium salt), and is preferably a compound that generates an organic acid by exposure.

Examples of the organic acid include sulfonic acids (such as aliphatic sulfonic acids, aromatic sulfonic acids, and camphorsulfonic acid), carboxylic acids (such as aliphatic carboxylic acids, aromatic carboxylic acids, and aralkyl carboxylic acids), carbonylsulfonylimidic acid, bis(alkylsulfonyl)imidic acids, and tris(alkylsulfonyl)methide acids.

In the compound represented by "M⁺ X'", M⁺ represents an organic cation.

The organic cation is not particularly limited. For the valence, the organic cation may be mono-, di-, or higher valent.

In particular, the organic cation is preferably a cation represented by the above-described formula (ZaI) (hereafter, also referred to as "cation (ZaI)") or a cation represented by the above-described formula (ZaII) (hereafter, also referred to as "cation (ZaII)").

In the compound represented by "M⁺ X⁻", X⁻ represents an organic anion.

The organic anion is not particularly limited, but may be a mono-, di-, or higher valent organic anion.

The organic anion is preferably an anion that has a very low capability of causing a nucleophilic reaction, and more preferably a non-nucleophilic anion.

Examples of the non-nucleophilic anion include sulfonate anions (such as aliphatic sulfonate anions, aromatic sulfonate anions, and a camphorsulfonate anion), carboxylate anions (such as aliphatic carboxylate anions, aromatic carboxylate anions, and aralkyl carboxylate anions), a sulfonylimide anion, bis(alkylsulfonyl)imide anions, and tris(alkylsulfonyl)methide anions.

In such an aliphatic sulfonate anion or aliphatic carboxylate anion, the aliphatic moiety may be a linear or branched alkyl group or may be a cycloalkyl group, and is preferably a linear or branched alkyl group having 1 to 30 carbon atoms, or a cycloalkyl group having 3 to 30 carbon atoms.

The alkyl group may be, for example, a fluoroalkyl group (that may have a substituent other than a fluorine atom, or may be a perfluoroalkyl group).

In such an aromatic sulfonate anion or aromatic carboxylate anion, the aryl group is preferably an aryl group having 6 to 14 carbon atoms, and may be, for example, a phenyl group, a tolyl group, or a naphthyl group.

The above-described alkyl group, cycloalkyl group, and aryl group may have a substituent. The substituent is not particularly limited; examples include a nitro group, halogen atoms such as a fluorine atom and a chlorine atom, a carboxyl group, a hydroxyl group, an amino group, a cyano group, alkoxy groups (preferably having 1 to 15 carbon atoms), alkyl groups (preferably having 1 to 10 carbon atoms), cycloalkyl groups (preferably having 3 to 15 carbon atoms), aryl groups (preferably having 6 to 14 carbon atoms), alkoxycarbonyl groups (preferably having 2 to 7 carbon atoms), acyl groups (preferably having 2 to 12 carbon atoms), alkoxycarbonyloxy groups (preferably having 2 to 7 carbon atoms), alkylthio groups (preferably having 1 to 15 carbon atoms), alkylsulfonyl groups (preferably having 1 to 15 carbon atoms), alkyliminosulfonyl groups (preferably having 1 to 15 carbon atoms), and aryloxysulfonyl groups (preferably having 6 to 20 carbon atoms).

In such an aralkyl carboxylate anion, the aralkyl group is preferably an aralkyl group having 7 to 14 carbon atoms.

Examples of the aralkyl group having 7 to 14 carbon atoms include a benzyl group, a phenethyl group, a naphthylmethyl group, a naphthylethyl group, and a naphthylbutyl group.

The sulfonylimide anion may be, for example, a saccharin anion.

In such a bis(alkylsulfonyl)imide anion or a tris(alkylsulfonyl)methide anion, the alkyl groups are preferably an alkyl group having 1 to 5 carbon atoms. In the alkyl group, a substituent may be a halogen atom, an alkyl group substituted with a halogen atom, an alkoxy group, an alkylthio group, an alkyloxysulfonyl group, an aryloxysulfonyl group, or a cycloalkylaryloxysulfonyl group, and is preferably a fluorine atom or an alkyl group substituted with a fluorine atom.

In the bis(alkylsulfonyl)imide anion, the alkyl groups may be bonded together to form a ring structure. This results in an increase in the acid strength.

Other examples of the non-nucleophilic anion include phosphorus fluoride (for example, PF₆⁻), boron fluoride (for example, BF₄⁻), and antimony fluoride (for example, SbF₆⁻).

The non-nucleophilic anion is preferably an aliphatic sulfonate anion in which at least the α position of sulfonic acid is substituted with a fluorine atom, an aromatic sulfonate anion substituted with a fluorine atom or a group having a fluorine atom, a bis(alkylsulfonyl)imide anion in which the alkyl groups are substituted with fluorine atoms, or a tris(alkylsulfonyl)methide anion in which the alkyl groups are substituted with fluorine atoms. In particular, the anion is more preferably a perfluoroaliphatic sulfonate anion (preferably having 4 to 8 carbon atoms) or a benzenesulfonate anion having a fluorine atom, and still more preferably a nonafluorobutanesulfonate anion, a perfluorooctanesulfonate anion, a pentafluorobenzenesulfonate anion, or a 3,5-bis(trifluoromethyl)benzenesulfonate anion.

The non-nucleophilic anion is also preferably an anion represented by the following formula (AN1).

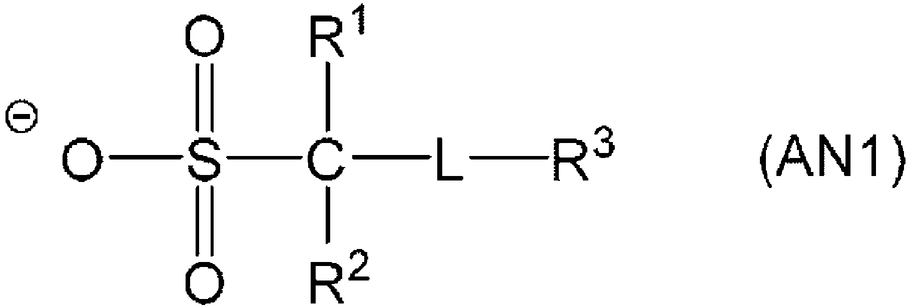

In the formula (AN1), R¹ and R² each independently represent a hydrogen atom or a substituent.

The substituent is not particularly limited, but is preferably a group that is not electron-withdrawing groups. Examples of the group that is not electron-withdrawing groups include hydrocarbon groups, a hydroxy group, oxyhydrocarbon groups, oxycarbonylhydrocarbon groups, an amino group, hydrocarbon-substituted amino groups, and hydrocarbon-substituted amide groups.

Such groups that are not electron-withdrawing groups are each independently preferably -R', -OH, -OR', -OCOR', -NH₂, -NR'₂, -NHR', or -NHCOR'. R' are monovalent hydrocarbon groups.

Examples of the monovalent hydrocarbon groups represented by R' above include monovalent linear or branched hydrocarbon groups such as alkyl groups such as a methyl group, an ethyl group, a propyl group, and a butyl group; alkenyl groups such as an ethenyl group, a propenyl group, and a butenyl group; and alkynyl groups such as an ethynyl group, a propynyl group, and a butynyl group; monovalent alicyclic hydrocarbon groups such as cycloalkyl groups such as a cyclopropyl group, a cyclobutyl group, a cyclopentyl group, a cyclohexyl group, a norbornyl group, and an adamantyl group; and cycloalkenyl groups such as a cyclopropenyl group, a cyclobutenyl group, a cyclopentenyl group, and a norbornenyl group; and monovalent aromatic hydrocarbon groups such as aryl groups such as a phenyl group, a tolyl group, a xylyl group, a mesityl group, a naphthyl group, a methylnaphthyl group, an anthryl group, and methylanthryl group; and aralkyl groups such as a benzyl group, a phenethyl group, a phenylpropyl group, a naphthylmethyl group, and an anthrylmethyl group.

In particular, R¹ and R² are each independently preferably a hydrocarbon group (preferably a cycloalkyl group) or a hydrogen atom.

L represents a divalent linking group.

When a plurality of L's are present, L's may be the same or different.

The divalent linking group may be, for example, -O-CO-O-, -COO-, -CONH-, -CO-, - O-, -S-, -SO-, -SO₂-, an alkylene group (preferably having 1 to 6 carbon atoms), a cycloalkylene group (preferably having 3 to 15 carbon atoms), an alkenylene group (preferably having 2 to 6 carbon atoms), or a divalent linking group that is a combination of a plurality of the foregoing. In particular, the divalent linking group is preferably -O-CO-O-, -COO-, -CONH-, -CO-, -O-, - SO₂-, -O-CO-O-alkylene group-, -COO-alkylene group-, or -CONH-alkylene group-, and more preferably -O-CO-O-, -O-CO-O-alkylene group-, -COO-, -CONH-, -SO₂-, or -COO-alkylene group-.

L is preferably, for example, a group represented by the following formula (AN1-1). *^{a}-(CR^{2a}₂)_{X}-Q-(CR^{2b}₂)_{Y}-*^{b} (AN1-1)

In the formula (AN1-1), *^{a} represents the bonding site to R³ in the formula (AN1). *^{b} represents the bonding site to -C(R¹)(R²)- in the formula (AN1).

X and Y each independently represent an integer of 0 to 10, and is preferably an integer of 0 to 3.

R^{2a} and R^{2b} each independently represent a hydrogen atom or a substituent.

When a plurality of R^{2a}'s and a plurality of R^{2b}'s are present, the plurality of R^{2a}'s and the plurality of R^{2b}'s present may be individually the same or different.

Note that, when Y is 1 or more, in the formula (AN1), in CR^{2b}₂ directly bonded to - C(R¹)(R²)-, R^{2b}'s are not fluorine atoms.

Q represents *^{A}-O-CO-O-*^{B}, *^{A}-CO-*^{B}, *^{A}-CO-O-*^{B}, *^{A}-O-CO-*^{B}, *^{A}-O-*^{B}, *^{A}-S-*^{B}, or *^{A}-SO₂-*^{B}.

Note that, when X + Y in the formula (AN1-1) is 1 or more, and R^{2a}'s and R^{2b}'s in the formula (AN1-1) are all hydrogen atoms, Q represents *^{A}-O-CO-O-*^{B}, *^{A}-CO-*^{B}, *^{A}-O-CO-*^{B}, *^{A}-O-*^{B}, *^{A}-S-*^{B}, or *^{A}-SO₂-*^{B}.
*^{A} represent a bonding site on the R³ side in the formula (AN1) and *^{B} represent a bonding site on the -SO₃⁻ side in the formula (AN1).

In the formula (AN1), R³ represents an organic group.

The organic group is not particularly limited as long as it has 1 or more carbon atoms, and may be a linear group (for example, a linear alkyl group) or a branched group (for example, a branched alkyl group such as a t-butyl group), or may be a cyclic group. The organic group may have or may not have a substituent. The organic group may have or may not have a heteroatom (such as an oxygen atom, a sulfur atom, and/or a nitrogen atom).

In particular, R³ is preferably an organic group having a ring structure. The ring structure may be monocyclic or polycyclic, and may have a substituent. In the organic group including a ring structure, the ring is preferably directly bonded to L in the formula (AN1).

The organic group having a ring structure, for example, may have or may not have a heteroatom (such as an oxygen atom, a sulfur atom, and/or a nitrogen atom). The heteroatom may substitute one or more carbon atoms forming the ring structure.

The organic group having a ring structure is preferably, for example, a hydrocarbon group having a ring structure, a lactone ring group, or a sultone ring group. In particular, the organic group having a ring structure is preferably a hydrocarbon group having a ring structure.

The hydrocarbon group having a ring structure is preferably a monocyclic or polycyclic cycloalkyl group. Such groups may have a substituent.

The cycloalkyl group may be monocyclic (such as a cyclohexyl group) or polycyclic (such as an adamantyl group), and preferably has 5 to 12 carbon atoms.

The lactone group and the sultone group are, for example, preferably a group provided by removing, in any one of the above-described structures represented by the formulas (LC1-1) to (LC1-21) and the above-described structures represented by the formulas (SL1-1) to (SL1-3), a single hydrogen atom from a ring-member atom constituting the lactone structure or the sultone structure.

The non-nucleophilic anion may be a benzenesulfonate anion, and is preferably a benzenesulfonate anion substituted with a branched alkyl group or a cycloalkyl group.

The non-nucleophilic anion is also preferably an anion represented by the following formula (AN2).

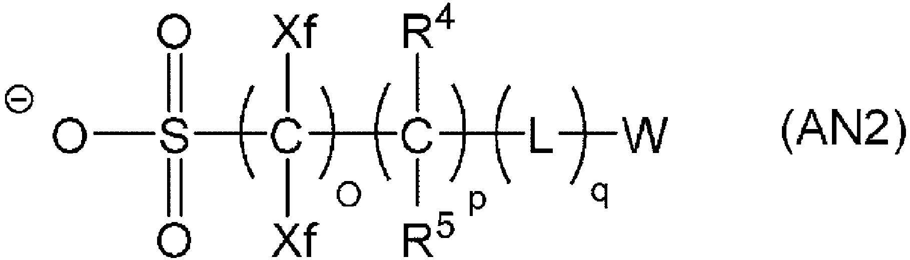

In the formula (AN2), o represents an integer of 1 to 3. p represents an integer of 0 to 10. q represents an integer of 0 to 10.

Xf's represent a hydrogen atom, a fluorine atom, an alkyl group substituted with at least one fluorine atom, or an organic group not having fluorine atoms. The alkyl group preferably has 1 to 10 carbon atoms, and more preferably 1 to 4 carbon atoms. The alkyl group substituted with at least one fluorine atom is preferably a perfluoroalkyl group.

Xf's are preferably a fluorine atom or a perfluoroalkyl group having 1 to 4 carbon atoms, and more preferably a fluorine atom or CF₃; still more preferably, both of Xf's are fluorine atoms.

R⁴ and R⁵ each independently represent a hydrogen atom, a fluorine atom, an alkyl group, or an alkyl group substituted with at least one fluorine atom. When a plurality of R⁴'s and a plurality of R⁵'s are present, R⁴'s and R⁵'s may be individually the same or different.

For R⁴ and R⁵, the alkyl group preferably has 1 to 4 carbon atoms. The alkyl group may have a substituent. R⁴ and R⁵ are preferably a hydrogen atom.

L represents a divalent linking group. L has the same definition as L in the formula (AN1).

W represents an organic group, and preferably represents an organic group including a ring structure. In particular, preferred is a cyclic organic group.

The cyclic organic group may be, for example, an alicyclic group, an aryl group, or a heterocyclic group.

The alicyclic group may be monocyclic or may be polycyclic. Examples of the monocyclic alicyclic group include monocyclic cycloalkyl groups such as a cyclopentyl group, a cyclohexyl group, and a cyclooctyl group. Examples of the polycyclic alicyclic group include polycyclic cycloalkyl groups such as a norbornyl group, a tricyclodecanyl group, a tetracyclodecanyl group, a tetracyclododecanyl group, and an adamantyl group. In particular, preferred are alicyclic groups having a bulky structure having 7 or more carbon atoms such as a norbornyl group, a tricyclodecanyl group, a tetracyclodecanyl group, a tetracyclododecanyl group, and an adamantyl group.

The aryl group may be monocyclic or polycyclic. Examples of the aryl group include a phenyl group, a naphthyl group, a phenanthryl group, and an anthryl group.

The heterocyclic group may be monocyclic or polycyclic. In particular, in the case of a polycyclic heterocyclic group, diffusion of acid can be further suppressed. The heterocyclic group may have aromaticity or may not have aromaticity. Examples of the heterocycle having aromaticity include a furan ring, a thiophene ring, a benzofuran ring, a benzothiophene ring, a dibenzofuran ring, a dibenzothiophene ring, and a pyridine ring. Examples of the heterocycle not having aromaticity include a tetrahydropyran ring, a lactone ring, a sultone ring, and a decahydroisoquinoline ring. In the heterocyclic group, the heterocycle is preferably a furan ring, a thiophene ring, a pyridine ring, or a decahydroisoquinoline ring.

The cyclic organic group may have a substituent. The substituent may be, for example, an alkyl group (that may be linear or branched and preferably has 1 to 12 carbon atoms), a cycloalkyl group (that may have a monocycle, a polycycle, or a spiro ring, and preferably has 3 to 20 carbon atoms), an aryl group (preferably having 6 to 14 carbon atoms), a hydroxy group, an alkoxy group, an ester group, an amide group, a urethane group, a ureido group, a thioether group, a sulfonamide group, or a sulfonic acid ester group. Note that a carbon constituting the cyclic organic group (carbon contributing to formation of the ring) may be a carbonyl carbon.

The anion represented by the formula (AN2) is preferably SO₃⁻-CF₂-CH₂-OCO-(L)_{q'}-W, SO₃⁻-CF₂-CHF-CH₂-OCO-(L)_{q'}-W, SO₃⁻-CF₂-COO-(L)_{q'}-W, SO₃⁻-CF₂-CF₂-CH₂-CH₂-(L)_{q}-W, or SO₃⁻-CF₂-CH(CF₃)-OCO-(L)_{q'}-W, Here, L, q, and W are the same as those in the formula (AN2). q' represents an integer of 0 to 10.

The non-nucleophilic anion is also preferably an aromatic sulfonate anion represented by the following formula (AN3).

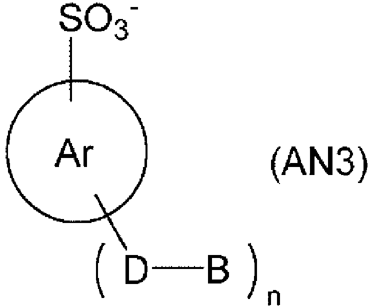

In the formula (AN3), Ar represents an aryl group (such as a phenyl group), and may further have a substituent other than the sulfonate anion and the -(D-B) group. Examples of the substituent that Ar may further have include a fluorine atom and a hydroxy group.

n represents an integer of 0 or more. n is preferably 1 to 4, more preferably 2 to 3, and still more preferably 3.

D represents a single bond or a divalent linking group. The divalent linking group may be an ether group, a thioether group, a carbonyl group, a sulfoxide group, a sulfo group, a sulfonic acid ester group, an ester group, or a group that is a combination of two or more of the foregoing.

B represents a hydrocarbon group.

B is preferably an aliphatic hydrocarbon group, and more preferably an isopropyl group, a cyclohexyl group, or an aryl group that may further have a substituent (such as a tricyclohexylphenyl group).

The non-nucleophilic anion is also preferably a disulfonamide anion.

The disulfonamide anion is, for example, an anion represented by N⁻(SO₂-R^{q})₂.

R^{q}'s represent an alkyl group that may have a substituent, and are preferably a fluoroalkyl group, and more preferably a perfluoroalkyl group. Two R^{q}'s may be bonded together to form a ring. The group formed by bonding together two R^{q}'s is preferably an alkylene group that may have a substituent, preferably a fluoroalkylene group, and more preferably a perfluoroalkylene group. The alkylene group preferably has 2 to 4 carbon atoms.

Other examples of the non-nucleophilic anion include anions represented by the following formulas (d1-1) to (d1-4).

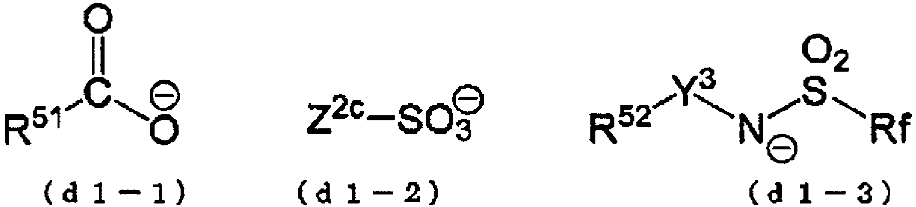

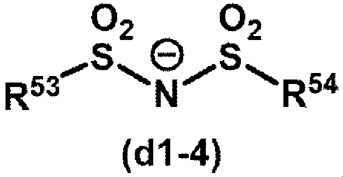

In the formula (d1-1), R⁵¹ represents a hydrocarbon group that may have a substituent (such as a hydroxy group) (for example, an aryl group such as a phenyl group).

In the formula (d1-2), Z^{2c} represents a hydrocarbon group that has 1 to 30 carbon atoms and that may have a substituent (provided that the carbon atom adjacent to S is not substituted with a fluorine atom).

In Z^{2c}, the hydrocarbon group may be linear or branched, and may have a ring structure. In the hydrocarbon group, a carbon atom (preferably, in a case where the hydrocarbon group has a ring structure, a carbon atom serving as a ring-member atom) may be a carbonyl carbon (-CO-). The hydrocarbon group may be, for example, a group that has a norbornyl group that may have a substituent. A carbon atom forming the norbornyl group may be a carbonyl carbon.

In the formula (d1-2), "Z^{2c}-SO₃⁻" is preferably different from the anions represented by the above-described formulas (AN1) to (AN3). For example, Z^{2c} is preferably not aryl groups. For example, in Z^{2c}, the atoms at the α position and the β position with respect to -SO₃⁻ are preferably atoms other than carbon atoms having, as a substituent, a fluorine atom. For example, in Z^{2c}, the atom at the α position and/or the atom at the β position with respect to -SO₃⁻ is preferably a ring-member atom in a ring group.

In the formula (d1-3), R⁵² represents an organic group (preferably a hydrocarbon group having a fluorine atom); Y³ represents a linear, branched, or cyclic alkylene group, an arylene group, or a carbonyl group; and Rf represents a hydrocarbon group.

In the formula (d1-4), R⁵³ and R⁵⁴ each independently represent an organic group (preferably a hydrocarbon group having a fluorine atom). R⁵³ and R⁵⁴ may be bonded together to form a ring.

Such organic anions may be used alone or in combination of two or more thereof.

The photoacid generator is also preferably at least one selected from the group consisting of compounds (I) to (II).

### Compound (I)

The compound (I) is a compound having one or more structural moieties X described below and one or more structural moieties Y described below, and is a compound that generates, upon irradiation with an actinic ray or a radiation, an acid including a first acidic moiety described below derived from the structural moiety X described below and a second acidic moiety described below derived from the structural moiety Y described below.

Structural moiety X: a structural moiety that is constituted by an anionic moiety A₁⁻ and a cationic moiety M₁⁺ and that forms, upon irradiation with an actinic ray or a radiation, the first acidic moiety represented by HA₁

Structural moiety Y: a structural moiety that is constituted by an anionic moiety A₂⁻ and a cationic moiety M₂⁺ and that forms, upon irradiation with an actinic ray or a radiation, the second acidic moiety represented by HA₂

The compound (I) satisfies the following condition I.

Condition I: A compound PI in which the cationic moiety M₁⁺ in the structural moiety X and the cationic moiety M₂⁺ in the structural moiety Y in the compound (I) are replaced by H⁺ has an acid dissociation constant a1 derived from an acidic moiety represented by HA₁ in which the cationic moiety M₁⁺ in the structural moiety X is replaced by H⁺, and an acid dissociation constant a2 derived from an acidic moiety represented by HA₂ in which the cationic moiety M₂⁺ in the structural moiety Y is replaced by H⁺, and the acid dissociation constant a2 is larger than the acid dissociation constant a1.

Hereinafter, the condition I will be more specifically described.

When the compound (I) is, for example, a compound that generates an acid having one first acidic moiety derived from the structural moiety X and one second acidic moiety derived from the structural moiety Y, the compound PI corresponds to a "compound having HA₁ and HA₂".

The acid dissociation constant a1 and the acid dissociation constant a2 of the compound PI will be more specifically described as follows: in determination of the acid dissociation constants of the compound PI, the pKa at the time when the compound PI turns into a "compound having A₁⁻ and HA₂" is the acid dissociation constant a1, and the pKa at the time when the "compound having A₁⁻ and HA₂" turns into a "compound having A₁⁻ and A₂⁻" is the acid dissociation constant a2.

When the compound (I) is, for example, a compound that generates an acid having two first acidic moieties derived from the structural moieties X and one second acidic moiety derived from the structural moiety Y, the compound PI corresponds to a "compound having two HA₁ and one HA₂" .

In determination of the acid dissociation constants of the compound PI, the acid dissociation constant at the time when the compound PI turns into a "compound having one A₁⁻, one HA₁, and one HA₂" and the acid dissociation constant at the time when the "compound having one A₁⁻, one HA₁, and one HA₂" turns into a "compound having two A₁⁻ and one HA₂" correspond to the above-described acid dissociation constant a1. The acid dissociation constant at the time when the "compound having two A₁⁻ and one HA₂" turns into a "compound having two A₁⁻ and A₂⁻" corresponds to the acid dissociation constant a2. In other words, when the compound PI has a plurality of acid dissociation constants derived from the acidic moieties represented by HA₁ in which the cationic moiety M₁⁺ in the structural moiety X is replaced by H⁺, the value of the acid dissociation constant a2 is larger than the largest value among the plurality of the acid dissociation constants a1. Note that, in a case where the acid dissociation constant at the time when the compound PI turns into the "compound having one A₁⁻, one HA₁, and one HA₂" is defined as aa, and the acid dissociation constant at the time when the "compound having one A₁⁻, one HA₁, and one HA₂" turns into the "compound having two A₁⁻ and one HA₂" is defined as ab, the relationship between aa and ab satisfies aa < ab.

The acid dissociation constant a1 and the acid dissociation constant a2 can be determined by the above-described method of measuring an acid dissociation constant.

The compound PI corresponds to an acid generated upon irradiation of the compound (I) with an actinic ray or a radiation.

When the compound (I) has two or more structural moieties X, the structural moieties X may be the same or different. The two or more A₁⁻ and the two or more M₁⁺ may be individually the same or different.

In the compound (I), A₁⁻ above and A₂⁻ above, and M₁⁺ above and M₂⁺ above may be individually the same or different, but A₁⁻ above and A₂⁻ above are preferably different from each other.

In the compound PI, the difference (absolute value) between the acid dissociation constant a1 (when a plurality of acid dissociation constants a1 are present, the maximum value thereof) and the acid dissociation constant a2 is preferably 0.1 or more, more preferably 0.5 or more, and still more preferably 1.0 or more. Note that the upper limit value of the difference (absolute value) between the acid dissociation constant a1 (when a plurality of acid dissociation constants a1 are present, the maximum value thereof) and the acid dissociation constant a2 is not particularly limited, but is, for example, 16 or less.

In the compound PI, the acid dissociation constant a2 is preferably 20 or less, and more preferably 15 or less. Note that the lower limit value of the acid dissociation constant a2 is preferably -4.0 or more.

In the compound PI, the acid dissociation constant a1 is preferably 2.0 or less, and more preferably 0 or less. Note that the lower limit value of the acid dissociation constant a1 is preferably -20.0 or more.

The anionic moiety A₁⁻ and the anionic moiety A₂⁻ are structural moieties including a negatively charged atom or atomic group and are, for example, structural moieties selected from the group consisting of the following formulas (AA-1) to (AA-3) and formulas (BB-1) to (BB-6).

The anionic moiety A₁⁻ is preferably an anionic moiety that can form an acidic moiety having a small acid dissociation constant, in particular, more preferably any one of the formulas (AA-1) to (AA-3), and still more preferably any one of the formulas (AA-1) and (AA-3).

The anionic moiety A₂⁻ is preferably an anionic moiety that can form an acidic moiety having a larger acid dissociation constant than the anionic moiety A₁⁻, more preferably any one of the formulas (BB-1) to (BB-6), and still more preferably any one of the formulas (BB-1) and (BB-4).

Note that, in the formulas (AA-1) to (AA-3) and the formulas (BB-1) to (BB-6) below, * represent a bonding site.

In the formula (AA-2), R^{A} represent a monovalent organic group. The monovalent organic groups represented by R^{A} are not particularly limited, but may be, for example, a cyano group, a trifluoromethyl group, or a methanesulfonyl group.

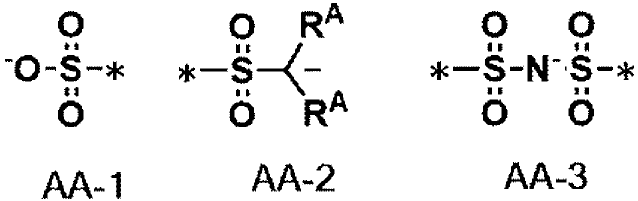

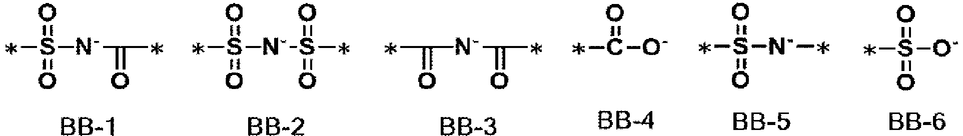

The cationic moiety M₁⁺ and the cationic moiety M₂⁺ are structural moieties including a positively charged atom or atomic group and may be, for example, singly charged organic cations. Note that such an organic cation may be, for example, the above-described organic cation represented by M⁺.

The compound (I) is not particularly limited in terms of specific structures; examples include compounds represented by a formula (Ia-1) to a formula (Ia-5) described later. Compound represented by formula (Ia-1)
Hereinafter, first, the compound represented by the formula (Ia-1) will be described.

M₁₁⁺ A₁₁⁻-L₁-A₁₂⁻ M₁₂⁺ (Ia-1)

The compound represented by the formula (Ia-1) generates, upon irradiation with an actinic ray or a radiation, an acid represented by HA₁₁-L₁-A₁₂H.

In the formula (Ia-1), M₁₁⁺ and M₁₂⁺ each independently represent an organic cation. A₁₁⁻ and A₁₂⁻ each independently represent a monovalent anionic functional group.

L₁ represents a divalent linking group.

M₁₁⁺ and M₁₂⁺ may be the same or different.

A₁₁⁻ and A₁₂⁻ may be the same or different, but are preferably different from each other.

Note that, in the formula (Ia-1), in a compound PIa (HA₁₁-L₁-A₁₂H) in which the cations represented by M₁₁⁺ and M₁₂⁺ are replaced by H⁺, the acid dissociation constant a2 derived from the acidic moiety represented by A₁₂H is larger than the acid dissociation constant a1 derived from the acidic moiety represented by HA₁₁. Note that preferred values of the acid dissociation constant a1 and the acid dissociation constant a2 are the same as those described above. The compound PIa is the same as the acid generated from the compound represented by the formula (Ia-1) upon irradiation with an actinic ray or a radiation.

At least one of M₁₁⁺, M₁₂⁺, An', A₁₂⁻, or L₁ may have, as a substituent, an acid-decomposable group.

In the formula (Ia-1), the organic cations represented by M₁₁⁺ and M₁₂⁺ are the same as those having been described for M⁺.

The monovalent anionic functional group represented by A₁₁⁻ means a monovalent group including the above-described anionic moiety A₁⁻. The monovalent anionic functional group represented by A₁₂⁻ means a monovalent group including the above-described anionic moiety A₂⁻.

The monovalent anionic functional groups represented by An' and A₁₂⁻ are preferably monovalent anionic functional groups including the anionic moiety of any one of the above-described formulas (AA-1) to (AA-3) and formulas (BB-1) to (BB-6), and more preferably monovalent anionic functional groups selected from the group consisting of formulas (AX-1) to (AX-3) and formulas (BX-1) to (BX-7). In particular, the monovalent anionic functional group represented by An' is preferably the monovalent anionic functional group represented by any one of the formulas (AX-1) to (AX-3). In particular, the monovalent anionic functional group represented by A₁₂⁻ is preferably the monovalent anionic functional group represented by any one of the formulas (BX-1) to (BX-7), and more preferably the monovalent anionic functional group represented by any one of the formulas (BX-1) to (BX-6).

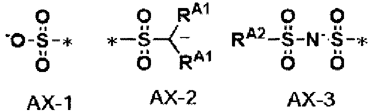

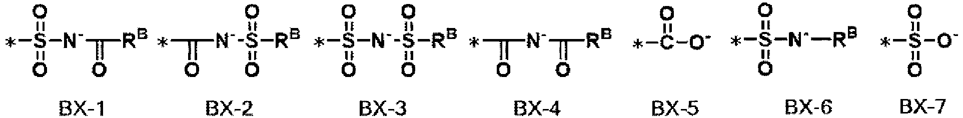

In the formulas (AX-1) to (AX-3), R^{A1} and R^{A2} each independently represent a monovalent organic group. * represent a bonding site.

The monovalent organic groups represented by R^{A1} are not particularly limited, but may be, for example, a cyano group, a trifluoromethyl group, or a methanesulfonyl group.

The monovalent organic group represented by R^{A2} is preferably a linear, branched, or cyclic alkyl group, or an aryl group.

The number of carbon atoms of the alkyl group is preferably 1 to 15, more preferably 1 to 10, and still more preferably 1 to 6.

The alkyl group may have a substituent. The substituent is preferably a fluorine atom or a cyano group, and more preferably a fluorine atom. When the alkyl group has, as a substituent, a fluorine atom, it may be a perfluoroalkyl group.

The aryl group is preferably a phenyl group or a naphthyl group, and more preferably a phenyl group.

The aryl group may have a substituent. The substituent is preferably a fluorine atom, an iodine atom, a perfluoroalkyl group (for example, preferably having 1 to 10 carbon atoms, and more preferably 1 to 6 carbon atoms), or a cyano group, and more preferably a fluorine atom, an iodine atom, or a perfluoroalkyl group.

In the formulas (BX-1) to (BX-4) and the formula (BX-6), R^{B} represent a monovalent organic group. * represent a bonding site.

The monovalent organic groups represented by R^{B} are preferably a linear, branched, or cyclic alkyl group, or an aryl group.

The number of carbon atoms of the alkyl group is preferably 1 to 15, more preferably 1 to 10, and still more preferably 1 to 6.

The alkyl group may have a substituent. The substituent is not particularly limited, but the substituent is preferably a fluorine atom or a cyano group, and more preferably a fluorine atom. When the alkyl group has, as a substituent, a fluorine atom, it may be a perfluoroalkyl group.

Note that, in the alkyl group, when a carbon atom serving as a bonding site has a substituent, it is also preferably a substituent other than a fluorine atom and a cyano group. In the alkyl group, the carbon atom serving as the bonding site corresponds to, for example, in the case of the formulas (BX-1) and (BX-4), a carbon atom (in such an alkyl group) directly bonded to -CO- clearly described in the formula; in the case of the formulas (BX-2) and (BX-3), a carbon atom (in such an alkyl group) directly bonded to -SO₂- clearly described in the formula; in the case of the formula (BX-6), a carbon atom (in the alkyl group) directly bonded to N' clearly described in the formula.

In the alkyl group, a carbon atom may be substituted with a carbonyl carbon.

The aryl group is preferably a phenyl group or a naphthyl group, and more preferably a phenyl group.

The aryl group may have a substituent. The substituent is preferably a fluorine atom, an iodine atom, a perfluoroalkyl group (for example, preferably having 1 to 10 carbon atoms, and more preferably having 1 to 6 carbon atoms), a cyano group, an alkyl group (for example, preferably having 1 to 10 carbon atoms, and more preferably having 1 to 6 carbon atoms), an alkoxy group (for example, preferably having 1 to 10 carbon atoms, and more preferably having 1 to 6 carbon atoms), or an alkoxycarbonyl group (for example, preferably having 2 to 10 carbon atoms, and more preferably having 2 to 6 carbon atoms), and more preferably a fluorine atom, an iodine atom, a perfluoroalkyl group, an alkyl group, an alkoxy group, or an alkoxycarbonyl group.

In the formula (Ia-1), the divalent linking group represented by L₁ is not particularly limited, and examples thereof include -CO-, -NR-, -O-, -S-, -SO-, -SO₂-, an alkylene group (that preferably has 1 to 6 carbon atoms and may be linear or branched), a cycloalkylene group (preferably having 3 to 15 carbon atoms), an alkenylene group (preferably having 2 to 6 carbon atoms), a divalent aliphatic heterocyclic group (having preferably a 5- to 10-membered ring, more preferably a 5- to 7-membered ring, and still more preferably a 5- to 6-membered ring that have at least one N atom, O atom, S atom, or Se atom in the ring structure), a divalent aromatic heterocyclic group (having preferably a 5- to 10-membered ring, more preferably a 5- to 7-membered ring, and still more preferably a 5- to 6-membered ring that have at least one N atom, O atom, S atom, or Se atom in the ring structure), a divalent aromatic hydrocarbon cyclic group (having preferably a 6- to 10-membered ring, and more preferably a 6-membered ring), and divalent linking groups that are combinations of a plurality of the foregoing. R above may be a hydrogen atom or a monovalent organic group. The monovalent organic group is not particularly limited, but is preferably, for example, an alkyl group (preferably having 1 to 6 carbon atoms).

The alkylene group, the cycloalkylene group, the alkenylene group, the divalent aliphatic heterocyclic group, the divalent aromatic heterocyclic group, and the divalent aromatic hydrocarbon cyclic group may have a substituent. Examples of the substituent include halogen atoms (preferably a fluorine atom).

In particular, the divalent linking group represented by L₁ is preferably a divalent linking group represented by a formula (L1).

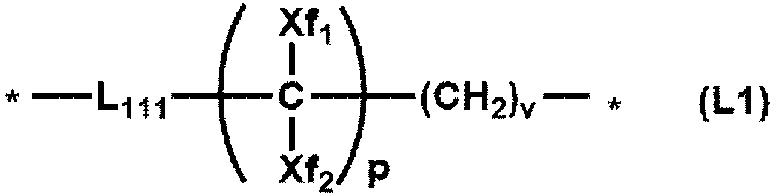

In the formula (L1), L₁₁₁ represents a single bond or a divalent linking group.

The divalent linking group represented by L₁₁₁ is not particularly limited, and examples thereof include -CO-, -NH-, -O-, -SO-, -SO₂-, an alkylene group that may have a substituent (that more preferably has 1 to 6 carbon atoms and may be linear or branched), a cycloalkylene group that may have a substituent (that preferably has 3 to 15 carbon atoms), an aryl group that may have a substituent (that preferably has 6 to 10 carbon atoms), and divalent linking groups that are combinations of a plurality of the foregoing. The substituent is not particularly limited, and may be, for example, a halogen atom.
p represents an integer of 0 to 3, and preferably represents an integer of 1 to 3.
v represents an integer of 0 or 1.
Xf₁ each independently represent a fluorine atom or an alkyl group substituted with at least one fluorine atom. The alkyl group preferably has 1 to 10 carbon atoms, and more preferably 1 to 4 carbon atoms. The alkyl group substituted with at least one fluorine atom is preferably a perfluoroalkyl group.
Xf₂ each independently represent a hydrogen atom, an alkyl group that may have, as a substituent, a fluorine atom, or a fluorine atom. The alkyl group preferably has 1 to 10 carbon atoms, and more preferably 1 to 4 carbon atoms. In particular, Xf₂ preferably represent a fluorine atom or an alkyl group substituted with at least one fluorine atom, and more preferably a fluorine atom or a perfluoroalkyl group.

In particular, Xf₁ and Xf₂ are each independently preferably a fluorine atom or a perfluoroalkyl group having 1 to 4 carbon atoms, and more preferably a fluorine atom or CF₃. In particular, Xf₁ and Xf₂ are each still more preferably a fluorine atom.
* represent a bonding site.

When, in the formula (Ia-1), L₁ represents a divalent linking group represented by the formula (L1), the L₁₁₁-side direct bond (*) in the formula (L1) is preferably bonded to A₁₂⁻ in the formula (Ia-1).

### Compounds represented by formulas (Ia-2) to (Ia-4)

Hereinafter, compounds represented by formulas (Ia-2) to (Ia-4) will be described.

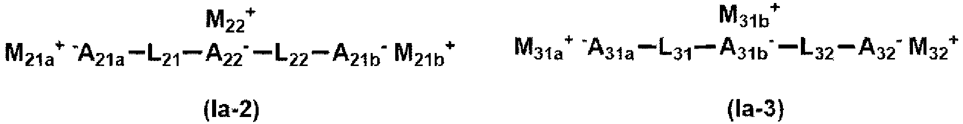

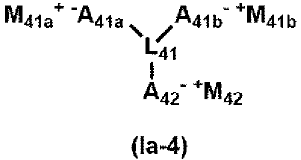

In the formula (Ia-2), A₂₁ₐ⁻ and A_{21b}⁻ each independently represent a monovalent anionic functional group. For A₂₁ₐ⁻ and A_{21b}⁻, the monovalent anionic functional group means a monovalent group including the above-described anionic moiety A₁⁻. For A₂₁ₐ⁻ and A_{21b}⁻, the monovalent anionic functional group is not particularly limited, but may be, for example, a monovalent anionic functional group selected from the group consisting of the above-described formulas (AX-1) to (AX-3).

A₂₂⁻ represents a divalent anionic functional group. The divalent anionic functional group represented by A₂₂⁻ means a divalent linking group including the above-described anionic moiety A₂⁻. For the divalent anionic functional group represented by A₂₂⁻, examples include divalent anionic functional groups represented by the following formulas (BX-8) to (BX-11).

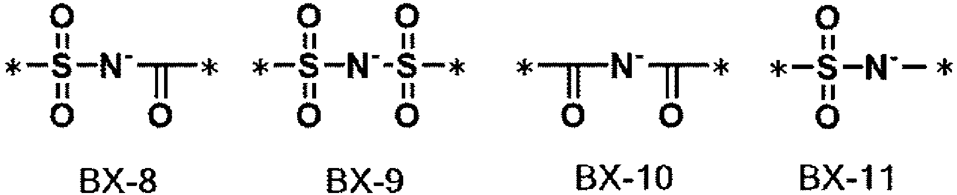

M₂₁ₐ⁺, M_{21b}⁺, and M₂₂⁺ each independently represent an organic cation. The organic cations represented by M₂₁ₐ⁺, M_{21b}⁺, and M₂₂⁺ have the same definitions and preferred examples as those of M₁₁⁺ described above.

L₂₁ and L₂₂ each independently represent a divalent organic group.

In a compound PIa-2 in which organic cations represented by M₂₁ₐ⁺, M_{21b}⁺, and M₂₂⁺ in the formula (Ia-2) are replaced by H⁺, the acid dissociation constant a2 derived from the acidic moiety represented by A₂₂H is larger than the acid dissociation constant a1-1 derived from the acidic moiety represented by A₂₁ₐH and the acid dissociation constant a1-2 derived from the acidic moiety represented by A_{21b}H. Note that the acid dissociation constant a1-1 and the acid dissociation constant a1-2 correspond to the above-described acid dissociation constant a1.

Note that A₂₁ₐ⁻ and A_{21b}⁻ may be the same or different. M₂₁ₐ⁺, M_{21b}⁺, and M₂₂⁺ may be the same or different.

At least one of M₂₁ₐ⁺, M_{21b}⁺, M₂₂⁺, A₂₁ₐ⁻, A_{21b}⁻, L₂₁, or L₂₂ may have, as a substituent, an acid-decomposable group.

In the formula (Ia-3), A₃₁ₐ⁻ and A₃₂⁻ each independently represent a monovalent anionic functional group. Note that the monovalent anionic functional group represented by A₃₁ₐ⁻ has the same definition and preferred examples as the above-described A₂₁ₐ⁻ and A_{21b}⁻ in the formula (Ia-2).

The monovalent anionic functional group represented by A₃₂⁻ means a monovalent group including the above-described anionic moiety A₂⁻. The monovalent anionic functional group represented by A₃₂⁻ is not particularly limited, but may be, for example, a monovalent anionic functional group selected from the group consisting of the above-described formulas (BX-1) to (BX-7).

A_{31b}⁻ represents a divalent anionic functional group. The divalent anionic functional group represented by A_{31b}⁻ means a divalent linking group including the above-described anionic moiety A₁⁻. The divalent anionic functional group represented by A_{31b}⁻ may be, for example, a divalent anionic functional group represented by the following formula (AX-4).

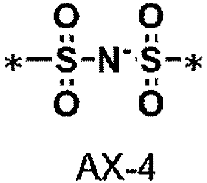

M₃₁ₐ⁺, M_{31b}⁺, and M₃₂⁺ each independently represent a monovalent organic cation. The organic cations represented by M₃₁ₐ⁺, M_{31b}⁺, and M₃₂⁺ have the same definitions and preferred examples as M₁₁⁺ described above.

L₃₁ and L₃₂ each independently represent a divalent organic group.

In a compound PIa-3 in which organic cations represented by M₃₁ₐ⁺, M_{31b}⁺, and M₃₂⁺ are replaced by H⁺ in the above-described formula (Ia-3), the acid dissociation constant a2 derived from the acidic moiety represented by A₃₂H is larger than the acid dissociation constant a1-3 derived from the acidic moiety represented by A₃₁ₐH and the acid dissociation constant a1-4 derived from the acidic moiety represented by A_{31b}H. Note that the acid dissociation constant a1-3 and the acid dissociation constant a1-4 correspond to the above-described acid dissociation constant a1.

Note that A₃₁ₐ⁻ and A₃₂⁻ may be the same or different. M₃₁ₐ⁺, M_{31b}⁺, and M₃₂⁺ may be the same or different.

At least one of M₃₁ₐ⁺, M_{31b}⁺, M₃₂⁺, A₃₁ₐ⁻, A₃₂⁻, L₃₁, or L₃₂ may have, as a substituent, an acid-decomposable group.

In the formula (Ia-4), A₄₁ₐ⁻, A_{41b}⁻, and A₄₂⁻ each independently represent a monovalent anionic functional group. Note that, for A₄₁ₐ⁻ and A_{41b}⁻, the monovalent anionic functional group has the same definition as the above-described A₂₁ₐ⁻ and A_{21b}⁻ in the formula (Ia-2). The monovalent anionic functional group represented by A₄₂⁻ has the same definition and preferred examples as the above-described A₃₂⁻ in the formula (Ia-3).

M₄₁ₐ⁺, M_{41b}⁺, and M₄₂⁺ each independently represent an organic cation. The organic cations represented by M₄₁ₐ⁺, M_{41b}⁺, and M₄₂⁺ have the same definitions and preferred examples as M₁₁⁺ described above.

L₄₁ represents a trivalent organic group.

In a compound PIa-4 in which the organic cations represented by M₄₁ₐ⁺, M_{41b}⁺, and M₄₂⁺ in the above-described formula (Ia-4) are replaced by H⁺, the acid dissociation constant a2 derived from the acidic moiety represented by A₄₂H is larger than the acid dissociation constant a1-5 derived from the acidic moiety represented by A₄₁ₐH and the acid dissociation constant a1-6 derived from the acidic moiety represented by A_{41b}H. Note that the acid dissociation constant a1-5 and the acid dissociation constant a1-6 correspond to the above-described acid dissociation constant a1.

Note that A₄₁ₐ⁻, A_{41b}⁻, and A₄₂⁻ may be the same or different. M₄₁ₐ⁺, M_{41b}⁺, and M₄₂⁺ may be the same or different.

At least one of M₄₁ₐ⁺, M_{41b}⁺, M₄₂⁺, A₄₁ₐ⁻, A_{41b}⁻, A₄₂⁻, or L₄₁ may have, as a substituent, an acid-decomposable group.

For L₂₁ and L₂₂ in the formula (Ia-2) and L₃₁ and L₃₂ in the formula (Ia-3), the divalent organic group is not particularly limited, and examples thereof include -CO-, -NR-, -O-, -S-, - SO-, -SO₂-, an alkylene group (that preferably has 1 to 6 carbon atoms and may be linear or branched), a cycloalkylene group (preferably having 3 to 15 carbon atoms), an alkenylene group (preferably having 2 to 6 carbon atoms), a divalent aliphatic heterocyclic group (having preferably a 5- to 10-membered ring, more preferably a 5- to 7-membered ring, and still more preferably a 5- to 6-membered ring that have at least one N atom, O atom, S atom, or Se atom in the ring structure), a divalent aromatic heterocyclic group (having preferably a 5- to 10-membered ring, more preferably a 5- to 7-membered ring, and still more preferably a 5- to 6-membered ring that have at least one N atom, O atom, S atom, or Se atom in the ring structure), a divalent aromatic hydrocarbon cyclic group (having preferably a 6- to 10-membered ring, and still more preferably a 6-membered ring), and divalent organic groups that are combinations of a plurality of the foregoing. In -NR- above, R may be a hydrogen atom or a monovalent organic group. The monovalent organic group is not particularly limited, but is preferably, for example, an alkyl group (preferably having 1 to 6 carbon atoms).

The alkylene group, the cycloalkylene group, the alkenylene group, the divalent aliphatic heterocyclic group, the divalent aromatic heterocyclic group, and the divalent aromatic hydrocarbon cyclic group may have a substituent. Examples of the substituent include halogen atoms (preferably a fluorine atom).

For L₂₁ and L₂₂ in the formula (Ia-2) and L₃₁ and L₃₂ in the formula (Ia-3), the divalent organic group is also preferably, for example, a divalent organic group represented by the following formula (L2).

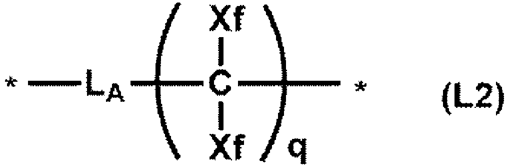

In the formula (L2), q represents an integer of 1 to 3. * represent a bonding site.

Xf's each independently represent a fluorine atom or an alkyl group substituted with at least one fluorine atom. The alkyl group preferably has 1 to 10 carbon atoms, and more preferably 1 to 4 carbon atoms. The alkyl group substituted with at least one fluorine atom is preferably a perfluoroalkyl group.

Xf's are preferably a fluorine atom or a perfluoroalkyl group having 1 to 4 carbon atoms, and more preferably a fluorine atom or CF₃. In particular, still more preferably, both of Xf's are fluorine atoms.

L_{A} represents a single bond or a divalent linking group.

The divalent linking group represented by L_{A} is not particularly limited, and examples thereof include -CO-, -O-, -SO-, -SO₂-, an alkylene group (that preferably has 1 to 6 carbon atoms and may be linear or branched), a cycloalkylene group (preferably having 3 to 15 carbon atoms), a divalent aromatic hydrocarbon cyclic group (preferably a 6- to 10-membered ring, and more preferably a 6-membered ring), and divalent linking groups that are combinations of a plurality of the foregoing.

The alkylene group, the cycloalkylene group, and the divalent aromatic hydrocarbon cyclic group may have a substituent. Examples of the substituent include halogen atoms (preferably a fluorine atom).

Examples of the divalent organic group represented by the formula (L2) include *-CF₂-*, *-CF₂-CF₂-*, *-CF₂-CF₂-CF₂-*, *-Ph-O-SO₂-CF₂-*, *-Ph-O-SO₂-CF₂-CF₂-*, *-Ph-O-SO₂-CF₂-CF₂-CF₂-*, and *-Ph-OCO-CF₂-*. Note that Ph are a phenylene group that may have a substituent, and preferably a 1,4-phenylene group. The substituent is not particularly limited, but is preferably an alkyl group (for example, preferably having 1 to 10 carbon atoms, and more preferably having 1 to 6 carbon atoms), an alkoxy group (for example, preferably having 1 to 10 carbon atoms, and more preferably having 1 to 6 carbon atoms), or an alkoxycarbonyl group (for example, preferably having 2 to 10 carbon atoms, and more preferably having 2 to 6 carbon atoms).

When L₂₁ and L₂₂ in the formula (Ia-2) represent the divalent organic group represented by the formula (L2), the L_{A}-side direct bond (*) in the formula (L2) is preferably bonded to A₂₁ₐ⁻ and A_{21b}⁻ in the formula (Ia-2).

When L₃₁ and L₃₂ in the formula (Ia-3) represent the divalent organic group represented by the formula (L2), the L_{A}-side direct bond (*) in the formula (L2) is preferably bonded to A₃₁ₐ⁻ and A₃₂⁻ in the formula (Ia-3).

### Compound represented by formula (Ia-5)

Hereinafter, the formula (Ia-5) will be described.

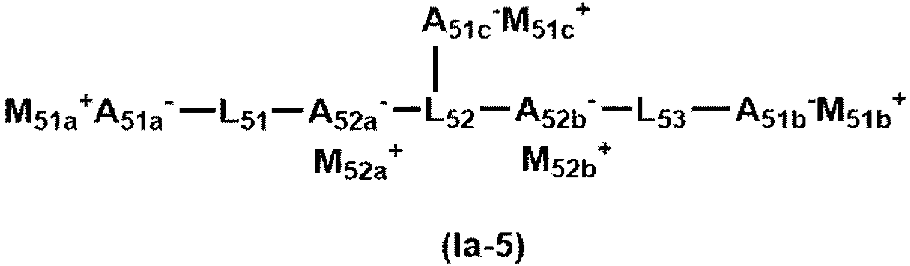

In the formula (Ia-5), A₅₁ₐ⁻, A_{51b}⁻, and A_{51c}⁻ each independently represent a monovalent anionic functional group. For A₅₁ₐ⁻, A_{51b}⁻, and A_{51c}⁻, the monovalent anionic functional group means a monovalent group including the above-described anionic moiety A₁⁻. For A₅₁ₐ⁻, A_{51b}⁻, and A_{51c}⁻, the monovalent anionic functional group is not particularly limited, but may be, for example, a monovalent anionic functional group selected from the group consisting of the above-described formulas (AX-1) to (AX-3).

A₅₂ₐ⁻ and A_{52b}⁻ represent a divalent anionic functional group. For A₅₂ₐ⁻ and A_{52b}⁻, the divalent anionic functional group means a divalent linking group including the above-described anionic moiety A₂⁻. The divalent anionic functional group represented by A₂₂⁻ may be, for example, a divalent anionic functional group selected from the group consisting of the above-described formulas (BX-8) to (BX-11).

M₅₁ₐ⁺, M_{51b}⁺, M_{51c}⁺, M₅₂ₐ⁺, and M_{52b}⁺ each independently represent an organic cation. The organic cations represented by M₅₁ₐ⁺, M_{51b}⁺, M_{51c}⁺, M₅₂ₐ⁺, and M_{52b}⁺ have the same definitions and preferred examples as M₁₁⁺ described above.

L₅₁ and L₅₃ each independently represent a divalent organic group. For L₅₁ and L₅₃, the divalent organic group has the same definition and preferred examples as the above-described L₂₁ and L₂₂ in the formula (Ia-2).

L₅₂ represents a trivalent organic group. The trivalent organic group represented by L₅₂ has the same definition and preferred examples as the above-described L₄₁ in the formula (Ia-4).

In a compound PIa-5 in which organic cations represented by M₅₁ₐ⁺, M_{51b}⁺, M_{51c}⁺, M₅₂ₐ⁺, and M_{52b}⁺ in the above-described formula (Ia-5) are replaced by H⁺, the acid dissociation constant a2-1 derived from the acidic moiety represented by A₅₂ₐH and the acid dissociation constant a2-2 derived from the acidic moiety represented by A_{52b}H are larger than the acid dissociation constant a1-1 derived from the acidic moiety represented by A₅₁ₐH , the acid dissociation constant a1-2 derived from the acidic moiety represented by A_{51b}H, and the acid dissociation constant a1-3 derived from the acidic moiety represented by A_{51c}H. Note that the acid dissociation constants a1-1 to a1-3 correspond to the above-described acid dissociation constant a1, and the acid dissociation constants a2-1 and a2-2 correspond to the above-described acid dissociation constant a2.

Note that A₅₁ₐ⁻, A_{51b}⁻, and A_{51c}⁻ may be the same or different. A₅₂ₐ⁻ and A_{52b}⁻ may be the same or different.

M₅₁ₐ⁺, M_{51b}⁺, M_{51c}⁺, M₅₂ₐ⁺, and M_{52b}⁺ may be the same or different.

At least one of M_{51b}⁺, M_{51c}⁺, M₅₂ₐ⁺, M_{52b}⁺, A₅₁ₐ⁻, A_{51b}⁻, A_{51c}⁻, L₅₁, L₅₂, or L₅₃ may have, as a substituent, an acid-decomposable group.

### Compound (II)

The compound (II) is a compound having two or more structural moieties X described above and one or more structural moieties Z described below, and is a compound that generates, upon irradiation with an actinic ray or a radiation, an acid including two or more first acidic moieties derived from the above-described structural moieties X and the above-described structural moiety Z.

### Structural moiety Z: a nonionic moiety that can neutralize acid

In the compound (II), the definition of the structural moiety X and the definitions of A₁⁻ and M₁⁺ are the same as the definition of the structural moiety X and the definitions of A₁⁻ and M₁⁺ in the above-described compound (I), and preferred examples are also the same.

In a compound PII in which the cationic moiety M₁⁺ in the structural moiety X in the compound (II) is replaced by H⁺, the preferred range of the acid dissociation constant a1 derived from the acidic moiety represented by HA₁ in which the cationic moiety M₁⁺ in the structural moiety X is replaced by H⁺ is the same as in the acid dissociation constant a1 in the compound PI.

Note that, when the compound (II) is, for example, a compound that generates an acid having two first acidic moieties derived from the structural moiety X and the structural moiety Z, the compound PII corresponds to a "compound having two HA₁". In determination of the acid dissociation constants of this compound PII, the acid dissociation constant at the time when the compound PII turns into a "compound having one A⁻ and one HA₁" and the acid dissociation constant at the time when the "compound having one A₁⁻ and one HA₁" turns into a "compound having two A₁⁻" correspond to the acid dissociation constant a1.

The acid dissociation constant a1 can be determined by the above-described method of measuring an acid dissociation constant.

The compound PII corresponds to an acid generated upon irradiation of the compound (II) with an actinic ray or a radiation.

Note that the two or more structural moieties X may be the same or different. The two or more A₁⁻ and the two or more M₁⁺ may be individually the same or different.

The nonionic moiety that can neutralize acid in the structural moiety Z is not particularly limited, and is preferably, for example, a moiety including a group that can electrostatically interact with a proton or a functional group having an electron.

Examples of the group that can electrostatically interact with a proton or the functional group having an electron include a functional group having a macrocyclic structure such as cyclic polyether, and a functional group having a nitrogen atom having an unshared electron pair that does not contribute to π-conjugation. Examples of the nitrogen atom having an unshared electron pair that does not contribute to π-conjugation include nitrogen atoms having partial structures represented by the following formulas.

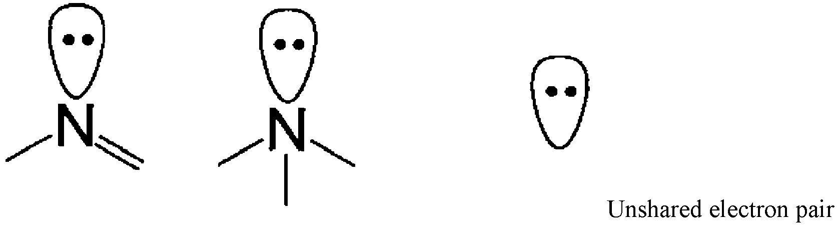

The partial structure of the group that can electrostatically interact with a proton or the functional group having an electron may be, for example, a crown ether structure, an azacrown ether structure, a primary to tertiary amine structure, a pyridine structure, an imidazole structure, or a pyrazine structure; in particular, preferred are primary to tertiary amine structures.

The compound (II) is not particularly limited, but examples thereof include compounds represented by the following formula (IIa-1) and the following formula (IIa-2).

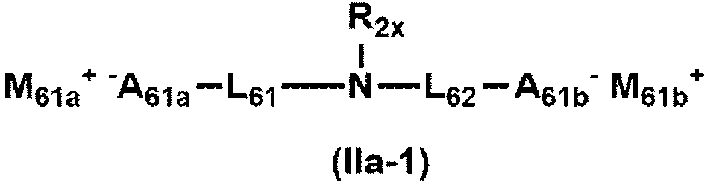

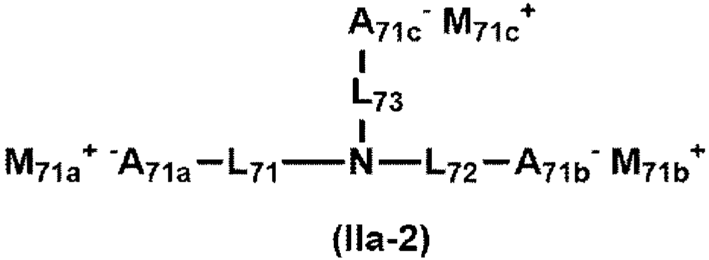

In the above-described formula (IIa-1), A₆₁ₐ⁻ and A_{61b}⁻ have the same definitions and preferred examples as An' in the above-described formula (Ia-1). M₆₁ₐ⁺ and M_{61b}⁺ have the same definitions and preferred examples as M₁₁⁺ in the above-described formula (Ia-1).

In the above-described formula (IIa-1), L₆₁ and L₆₂ have the same definitions and preferred examples as L₁ in the above-described formula (Ia-1).

In the formula (IIa-1), R_{2X} represents a monovalent organic group. The monovalent organic group represented by R_{2X} is not particularly limited and may be an alkyl group (that preferably has 1 to 10 carbon atoms and may be linear or branched), a cycloalkyl group (preferably having 3 to 15 carbon atoms), or an alkenyl group (preferably having 2 to 6 carbon atoms). In the monovalent organic group represented by R_{2X}, -CH₂- included in the alkyl group, cycloalkyl group, or alkenyl group may be substituted with one or a combination of two or more selected from the group consisting of -CO-, -NH-, -O-, -S-, -SO-, and -SO₂-.

The alkylene group, the cycloalkylene group, and the alkenylene group may have a substituent. The substituent is not particularly limited, but may be, for example, a halogen atom (preferably a fluorine atom).

In a compound PIIa-1 in which the organic cations represented by M₆₁ₐ⁺ and M_{61b}⁺ in the above-described formula (IIa-1) are replaced by H⁺, the acid dissociation constant a1-7 derived from the acidic moiety represented by A₆₁ₐH and the acid dissociation constant a1-8 derived from the acidic moiety represented by A_{61b}H correspond to the above-described acid dissociation constant a1.

Note that, in the above-described formula (IIa-1), the compound PIIa-1 in which the cationic moieties M₆₁ₐ⁺ and M_{61b}⁺ in the structural moieties X are replaced by H⁺ corresponds to HA₆₁ₐ-L₆₁-N(R_{2X})-L₆₂-A_{61b}H. The compound PIIa-1 is the same as the acid generated from the compound represented by the formula (IIa-1) upon irradiation with an actinic ray or a radiation.

At least one of M₆₁ₐ⁺, M_{61b}⁺, A₆₁ₐ⁻, A_{61b}⁻, L₆₁, L₆₂, or R_{2X} may have, as a substituent, an acid-decomposable group.

In the above-described formula (IIa-2), A₇₁ₐ⁻, A_{71b}⁻, and A_{71c}⁻ individually have the same definitions and preferred examples as A₁₁⁻ in the above-described formula (Ia-1). M₇₁ₐ⁺, M_{71b}⁺, and M_{71c}⁺ individually have the same definitions and preferred examples as M₁₁⁺ in the above-described formula (Ia-1).

In the above-described formula (IIa-2), L₇₁, L₇₂, and L₇₃ individually have the same definitions and preferred examples as L₁ in the above-described formula (Ia-1).

In a compound PIIa-2 in which the organic cations represented by M₇₁ₐ⁺, M_{71b}⁺, and M_{71c}⁺ in the above-described formula (IIa-2) are replaced by H⁺, the acid dissociation constant a1-9 derived from the acidic moiety represented by A₇₁ₐH, the acid dissociation constant a1-10 derived from the acidic moiety represented by A_{71b}H, and the acid dissociation constant a1-11 derived from the acidic moiety represented by A_{71c}H correspond to the above-described acid dissociation constant a1.

Note that, in the formula (IIa-2), the compound PIIa-2 in which the cationic moieties M₇₁ₐ⁺, M_{71b}⁺, and M_{71c}⁺ in the structural moieties X are replaced by H⁺ corresponds to HA₇₁ₐ-L₇₁-N(L₇₃-A_{71c}H)-L₇₂-A_{71b}H. The compound PIIa-2 is the same as the acid generated from the compound represented by the formula (IIa-2) upon irradiation with an actinic ray or a radiation.

At least one of M₇₁ₐ⁺, M_{71b}⁺, M_{71c}⁺, A₇₁ₐ⁻, A_{71b}⁻, A_{71c}⁻, L₇₁, L₇₂, or L₇₃ may have, as a substituent, an acid-decomposable group.

Examples of the non-cationic moieties that the compound (I) and the compound (II) can have are as follows.

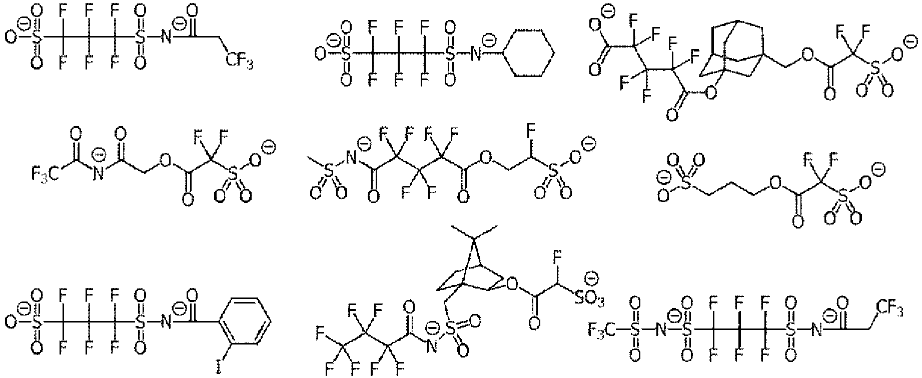

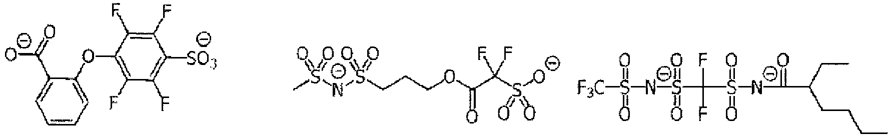

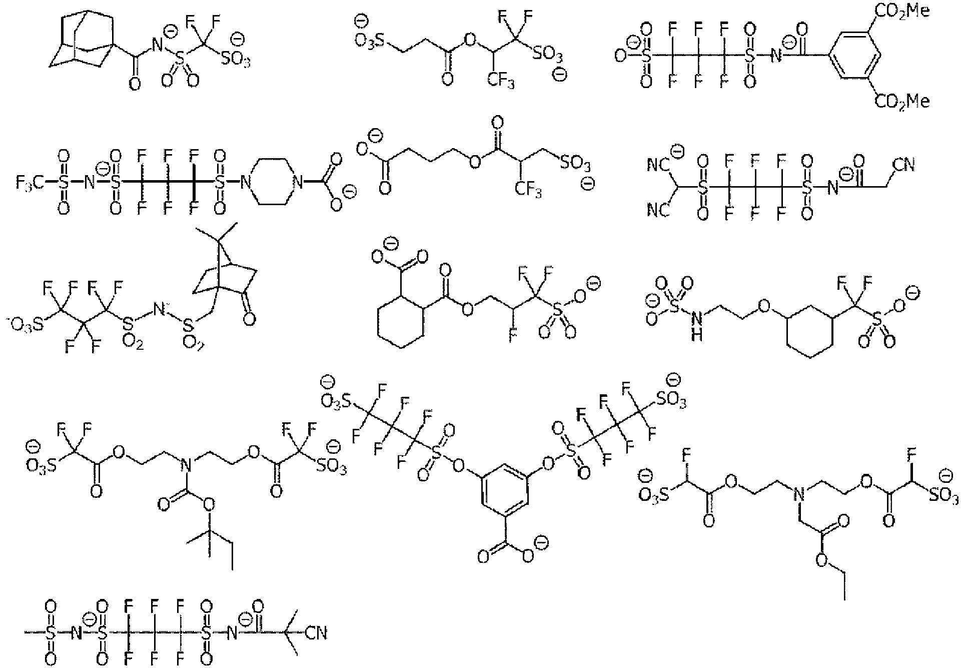

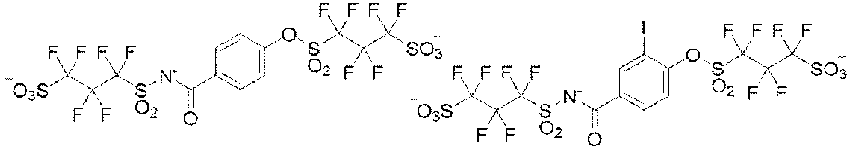

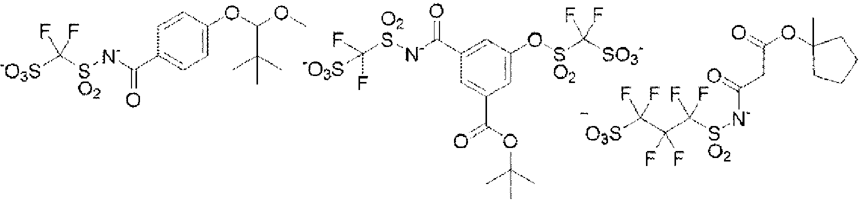

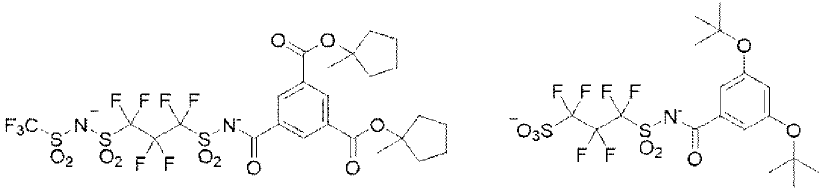

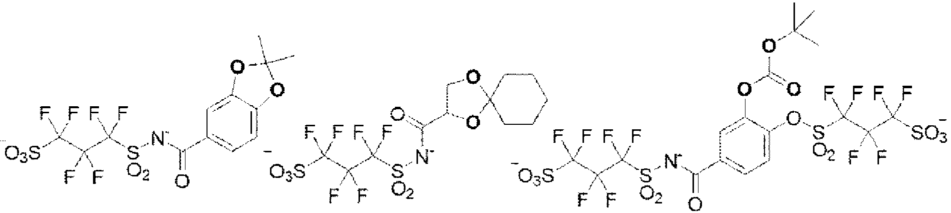

The following are non-limiting specific examples of the photoacid generator.

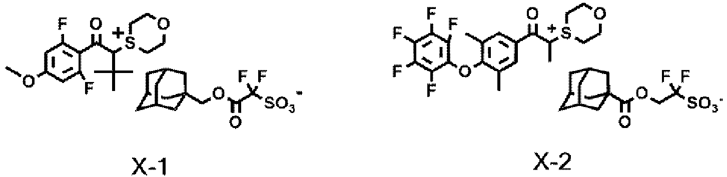

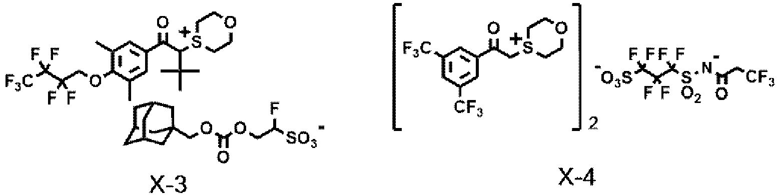

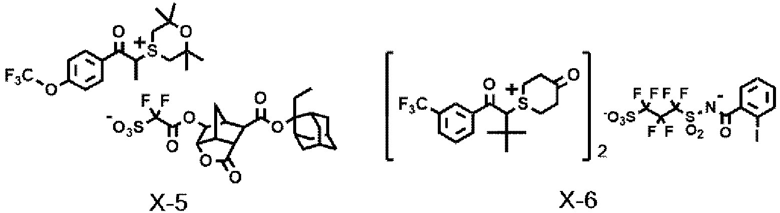

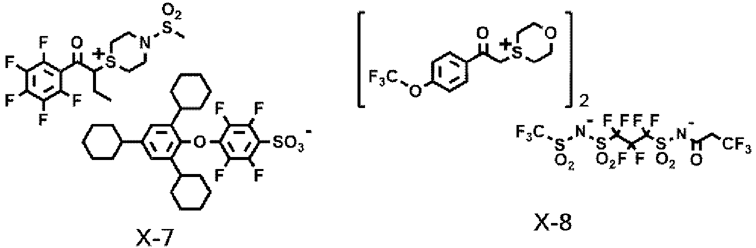

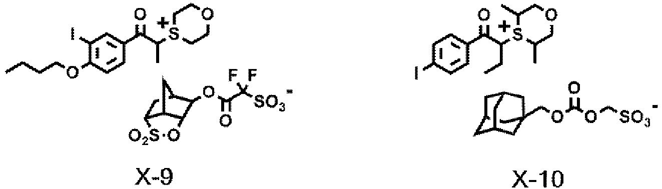

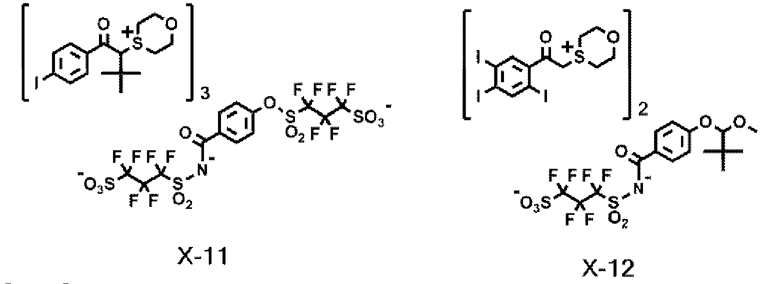

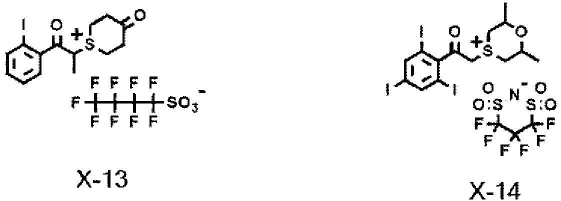

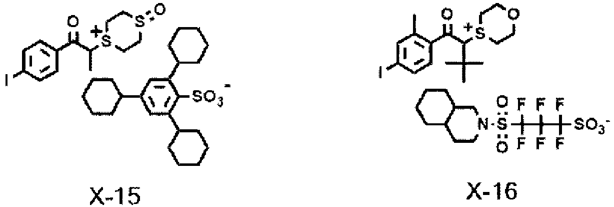

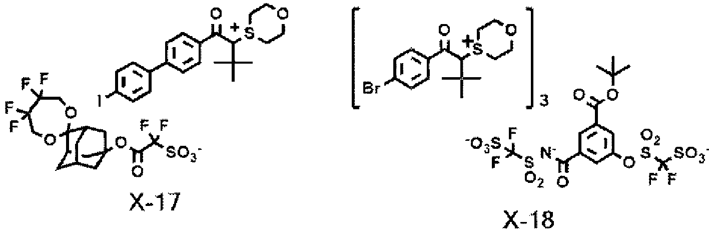

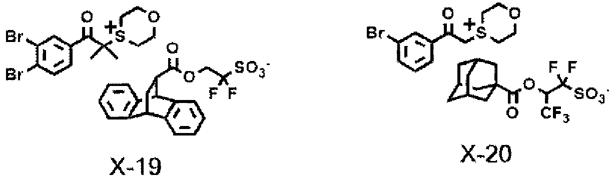

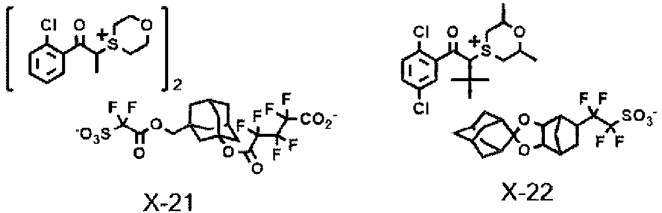

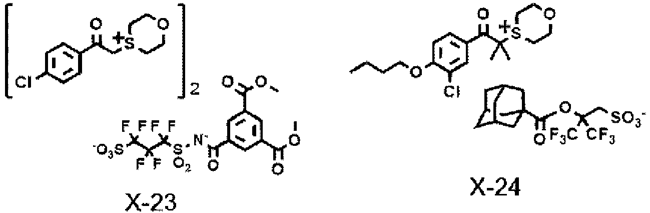

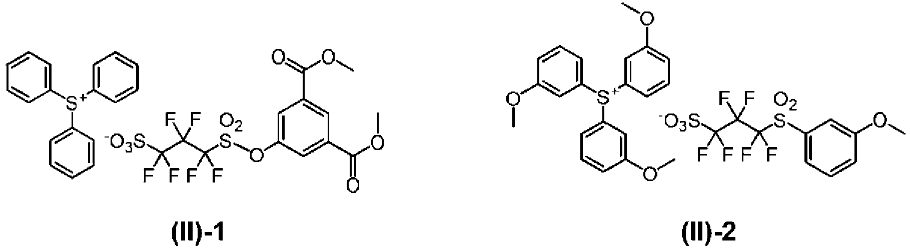

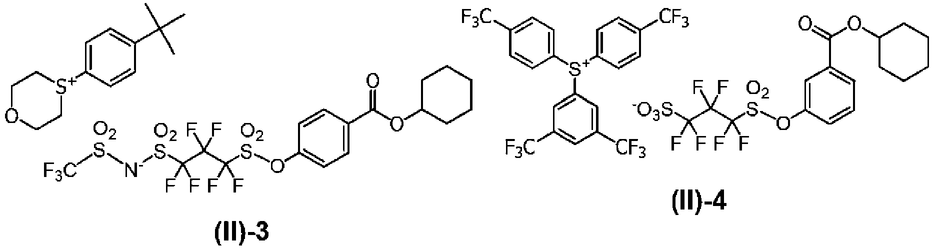

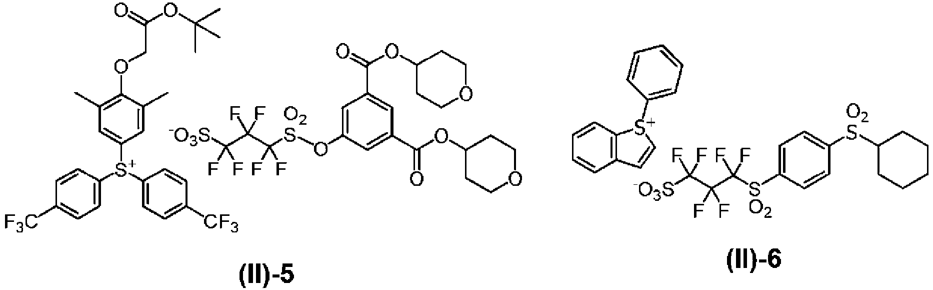

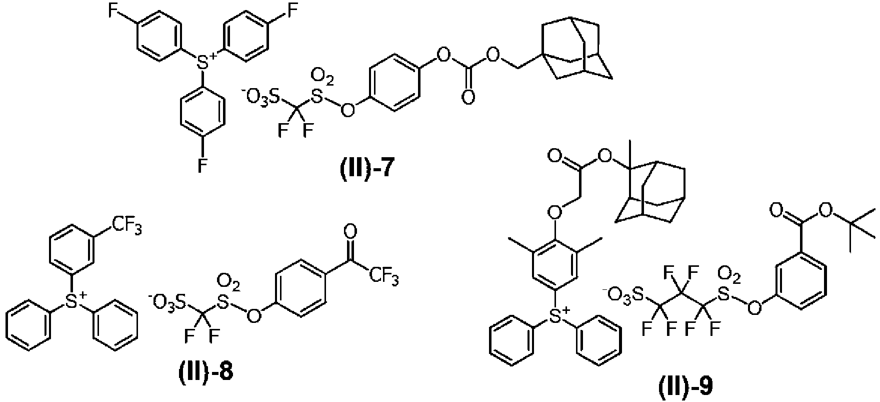

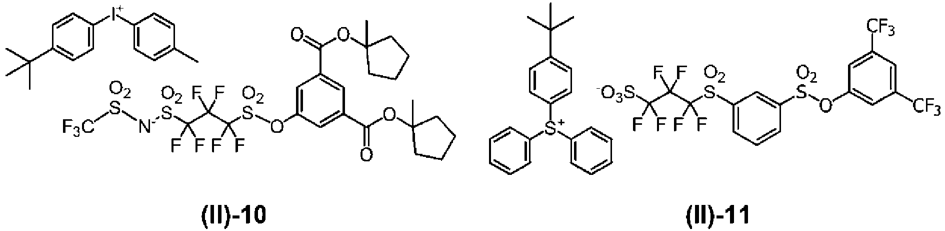

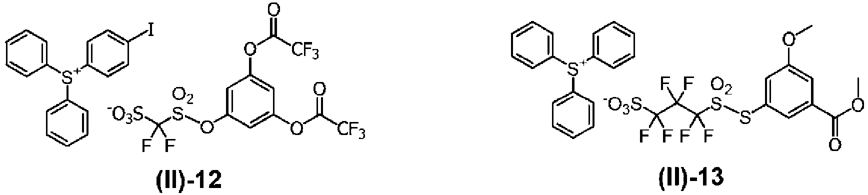

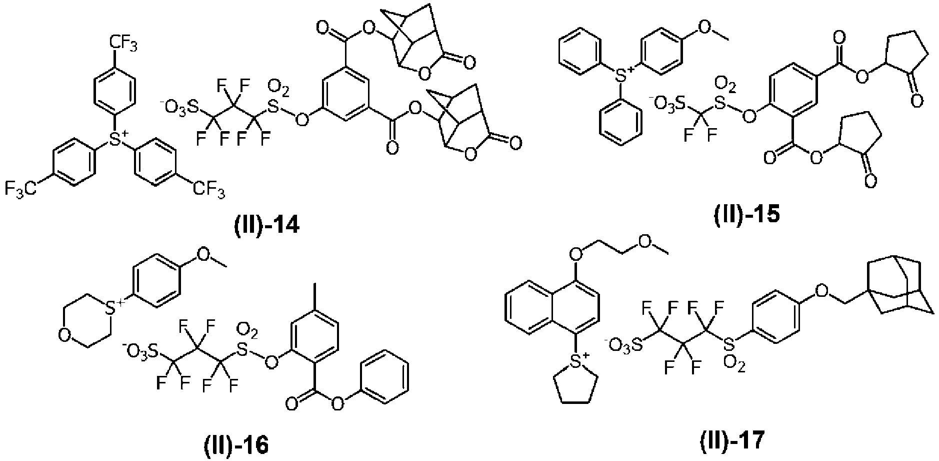

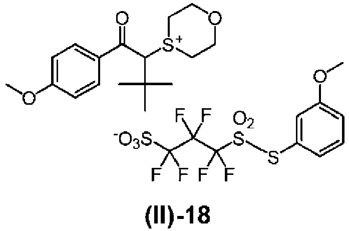

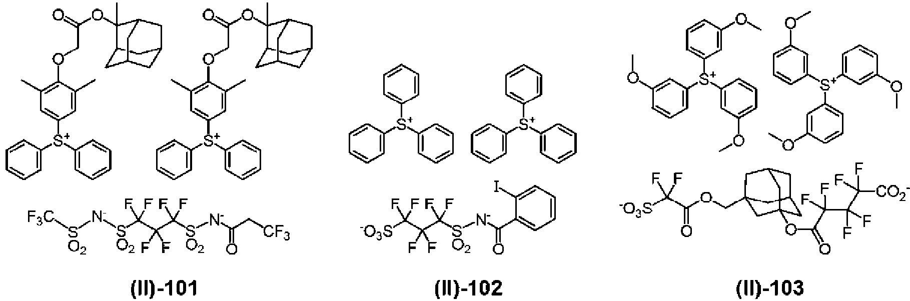

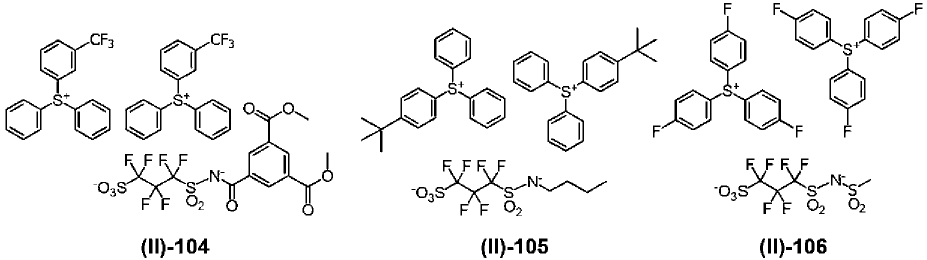

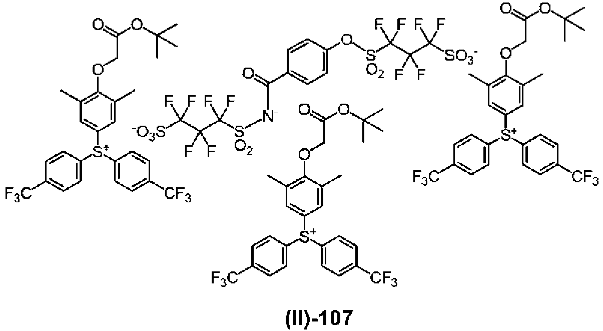

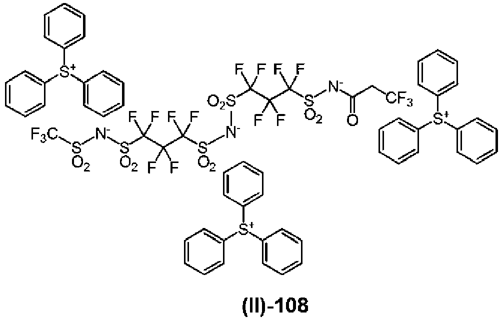

When the composition of the present invention includes the photoacid generator (P), the content thereof is not particularly limited, but is, from the viewpoint of forming a pattern having a more square profile, relative to the total solid content of the composition, preferably 0.5 mass% or more, and more preferably 1.0 mass% or more. The content is, relative to the total solid content of the composition, preferably 70.0 mass% or less, more preferably 60.0 mass% or less, and still more preferably 50.0 mass% or less.

Such photoacid generators (P) may be used alone or in combination of two or more thereof.

### Acid diffusion control agent (B)

The composition of the present invention may include an acid diffusion control agent different from the compound (Q).

The acid diffusion control agent serves as a quencher that traps the acid generated from the photoacid generator or the like upon exposure and that suppresses the reaction of the acid-decomposable resin, in the unexposed region, caused by an excess of generated acid.

The type of the acid diffusion control agent is not particularly limited, and examples thereof include a basic compound (CA), a low-molecular-weight compound (CB) having a nitrogen atom and having a group that leaves by the action of an acid, and a compound (CC) whose acid diffusion control ability is reduced or lost upon irradiation with an actinic ray or a radiation.

Examples of the compound (CC) include an onium salt compound (CD) that becomes a weak acid relative to the photoacid generator, and a basic compound (CE) whose basicity is reduced or lost upon irradiation with an actinic ray or a radiation.

Specific examples of the basic compound (CA) include, for example, those described in Paragraphs [0132] to [0136] of WO2020/066824A; specific examples of the basic compound (CE) whose basicity is reduced or lost upon irradiation with an actinic ray or a radiation include those described in Paragraphs [0137] to [0155] of WO2020/066824A, and those described in Paragraph [0164] of WO2020/066824A; and, specific examples of the low-molecular-weight compound (CB) having a nitrogen atom and having a group that leaves by the action of an acid include those described in Paragraphs [0156] to [0163] of WO2020/066824A.

Specific examples of the onium salt compound (CD) that becomes a weak acid relative to the photoacid generator include, for example, those described in Paragraphs [0305] to [0314] of WO2020/158337A.

In addition to those described above, for example, the publicly known compounds disclosed in Paragraphs [0627] to [0664] in US2016/0070167A1, Paragraphs [0095] to [0187] in US2015/0004544A1, Paragraphs [0403] to [0423] in US2016/0237190A1, and Paragraphs [0259] to [0328] in US2016/0274458A1 can be suitably used as acid diffusion control agents.

When the composition of the present invention includes an acid diffusion control agent, the content of the acid diffusion control agent (when there are a plurality of acid diffusion control agents, the total content thereof) is preferably, relative to the total solid content of the composition, 0.1 to 15.0 mass% and more preferably 1.0 to 15.0 mass%.

In the composition of the present invention, such acid diffusion control agents may be used alone or in combination of two or more thereof.

### Hydrophobic resin (c)

The composition of the present invention may further include a hydrophobic resin different from the resin (A).

The hydrophobic resin is preferably designed so as to be localized in the surface of a resist film; however, unlike surfactants, the hydrophobic resin does not necessarily need to have intramolecularly a hydrophilic group, and does not necessarily contribute to homogeneous mixing of a polar substance and a nonpolar substance.

Advantages due to addition of the hydrophobic resin may be control of static and dynamic contact angles (for water) at the surface of the resist film, and suppression of outgassing.

The hydrophobic resin, from the viewpoint of localization in the surface layer of the film, preferably has one or more species, more preferably two or more species, selected from the group consisting of a fluorine atom, a silicon atom, and a CH₃ moiety included in the side chain moiety of the resin. The hydrophobic resin preferably has a hydrocarbon group having 5 or more carbon atoms. The resin may have such a group in the main chain or, as a substituent, in a side chain.

Examples of the hydrophobic resin include the compounds described in Paragraphs [0275] to [0279] in WO2020/004306A.

When the composition of the present invention includes a hydrophobic resin, the content of the hydrophobic resin is, relative to the total solid content of the composition, preferably 0.01 to 20.0 mass%, and more preferably 0.1 to 15.0 mass%.

### Surfactant (E)

The composition of the present invention may include a surfactant. In the case of including a surfactant, a pattern having higher adhesiveness and a less number of development defects can be formed.

The surfactant is preferably a fluorine-based and/or silicone-based surfactant.

Examples of the fluorine-based and/or silicone-based surfactant include the surfactants disclosed in Paragraphs [0218] and [0219] of WO2018/193954A.

Such surfactants may be used alone or in combination of two or more thereof.

When the composition of the present invention includes a surfactant, the content of the surfactant is, relative to the total solid content of the composition, preferably 0.0001 to 2.0 mass%, more preferably 0.0005 to 1.0 mass%, and still more preferably 0.1 to 1.0 mass%. Solvent (F)

The composition of the present invention preferably includes a solvent.

The solvent preferably includes at least one of (M1) a propylene glycol monoalkyl ether carboxylate or (M2) at least one selected from the group consisting of a propylene glycol monoalkyl ether, a lactate, an acetate, an alkoxypropionate, a chain ketone, a cyclic ketone, a lactone, and an alkylene carbonate. Note that the solvent may further include a component other than the components (M1) and (M2).

A combination of the above-described solvent and the above-described resin is preferred from the viewpoint of improving the coatability of the resist composition and reducing the number of pattern development defects. The above-described solvent is well-balanced in terms of solubility of the above-described resin, boiling point, and viscosity, to thereby suppress, for example, unevenness of the film thickness of the resist film and generation of deposit during spin-coating.

Details of the component (M1) and the component (M2) are described in Paragraphs [0218] to [0226] in WO2020/004306A, and these contents are incorporated herein by reference.

When the solvent further includes a component other than the components (M1) and (M2), the content of the component other than the components (M1) and (M2) relative to the total amount of the solvent is preferably 5 to 30 mass%.

The content of the solvent in the composition of the present invention is determined such that the solid-content concentration is preferably 0.5 to 30 mass%, and more preferably 1 to 20 mass%. This further improves the coatability of the composition of the present invention.

Note that the solid content means all the components other than the solvent, and, as described above, means components that form the actinic ray-sensitive or radiation-sensitive film.

The solid-content concentration is a mass percentage of the mass of other components excluding the solvent relative to the total mass of the composition of the present invention.

The "total solid content" refers to the total mass of the components excluding the solvent from all the components of the composition of the present invention. As described above, the "solid content" is components excluding the solvent, and may be a solid or a liquid at 25°C, for example.

### Other additives

The composition of the present invention may further include a dissolution-inhibiting compound, a dye, a plasticizer, a photosensitizer, a light absorbent, and/or a compound that promotes solubility in a developer (for example, a phenol compound having a molecular weight of 1000 or less, or an alicyclic or aliphatic compound including a carboxyl group).

The "dissolution-inhibiting compound" is a compound that is decomposed by the action of an acid to undergo a decrease in the degree of solubility in organic-based developers, and has a molecular weight of 3000 or less.

The composition of the present invention is suitably used as a photosensitive composition for EUV exposure.

The EUV light has a wavelength of 13.5 nm, which is a shorter wavelength than in the ArF (wavelength: 193 nm) light and the like, and hence provides, upon exposure at the same sensitivity, a smaller number of incident photons. Thus, "photon shot noise", which is random variations in the number of photons, exerts a strong effect, which leads to degradation of LER and bridge defects. In order to reduce the photon shot noise, a method of increasing the exposure dose to increase the number of incident photons may be employed; however, there is a tradeoff between this method and the demand for an increase in the sensitivity.

### Actinic ray-sensitive or radiation-sensitive film and pattern forming method

The procedures of the pattern forming method using the composition are not particularly limited, but preferably have the following steps.
Step 1: a step of using the actinic ray-sensitive or radiation-sensitive resin composition to form an actinic ray-sensitive or radiation-sensitive film on a substrate
Step 2: a step of exposing the actinic ray-sensitive or radiation-sensitive film
Step 3: a step of developing the exposed actinic ray-sensitive or radiation-sensitive film using a developer

Hereinafter, procedures of the steps will be individually described in detail.

### Step 1: actinic ray-sensitive or radiation-sensitive film formation step

The step 1 is a step of using the actinic ray-sensitive or radiation-sensitive resin composition to form an actinic ray-sensitive or radiation-sensitive film on a substrate.

Examples of the method of using the actinic ray-sensitive or radiation-sensitive resin composition to form an actinic ray-sensitive or radiation-sensitive film (preferably, a resist film) on a substrate include a method of applying the composition of the present invention onto a substrate.

Note that the composition of the present invention is preferably filtered through a filter before application as needed. The filter preferably has a pore size of 0.1 µm or less, more preferably 0.05 µm or less, and still more preferably 0.03 µm or less. The filter is preferably formed of polytetrafluoroethylene, polyethylene, or nylon.

The composition of the present invention can be applied onto a substrate (such as a silicon, silicon dioxide-coated substrate) used in the production of an integrated circuit element, by an appropriate application process using a spinner, a coater, or the like. The application process is preferably spin-coating using a spinner. The spin-coating using a spinner is preferably performed at a rotation rate of 1000 to 3000 rpm.

After application of the composition of the present invention, the substrate may be dried to form an actinic ray-sensitive or radiation-sensitive film. Note that, as needed, as underlayers of the actinic ray-sensitive or radiation-sensitive film, various underlying films (an inorganic film, an organic film, or an antireflection film) may be formed.

The drying process may be, for example, a process of performing heating to achieve drying. The heating can be performed using means included in an ordinary exposure device and/or an ordinary development device, or may alternatively be performed using a hot plate, for example. The heating temperature is preferably 80 to 150°C, more preferably 80 to 140°C, and still more preferably 80 to 130°C. The heating time is preferably 30 to 1000 seconds, more preferably 60 to 800 seconds, and still more preferably 60 to 600 seconds.

The film thickness of the actinic ray-sensitive or radiation-sensitive film is not particularly limited, but is, from the viewpoint of enabling formation of more precise fine patterns, preferably 10 to 120 nm. In particular, in the case of employing EUV exposure, the film thickness of the actinic ray-sensitive or radiation-sensitive film is more preferably 10 to 65 nm, and still more preferably 15 to 50 nm. In the case of employing ArF liquid immersion exposure, the film thickness of the actinic ray-sensitive or radiation-sensitive film is more preferably 10 to 120 nm, and still more preferably 15 to 90 nm.

Note that, for an overlying layer of the actinic ray-sensitive or radiation-sensitive film, a topcoat composition may be used to form a topcoat.

The topcoat composition preferably does not mix with the actinic ray-sensitive or radiation-sensitive film, and can be uniformly applied for an overlying layer of the actinic ray-sensitive or radiation-sensitive film. The topcoat is not particularly limited; a publicly known topcoat can be formed by a publicly known process; for example, on the basis of descriptions of Paragraphs [0072] to [0082] in JP2014-059543A, a topcoat can be formed.

For example, a topcoat including a basic compound and described in JP2013-61648A is preferably formed on the actinic ray-sensitive or radiation-sensitive film. Specific examples of the basic compound that can be included in the topcoat include basic compounds that may be included in the resist composition.

The topcoat also preferably includes a compound including at least one group or bond selected from the group consisting of an ether bond, a thioether bond, a hydroxy group, a thiol group, a carbonyl bond, and an ester bond.

### Step 2: Exposure step

The step 2 is a step of exposing the actinic ray-sensitive or radiation-sensitive film.

The exposure process may be a process of irradiating the formed actinic ray-sensitive or radiation-sensitive film, through a predetermined mask, with an actinic ray or a radiation.

Examples of the actinic ray or the radiation include infrared light, visible light, ultraviolet light, far-ultraviolet light, extreme ultraviolet light, X-rays, and an electron beam; preferred is 250 nm or less, more preferred is 220 nm or less, and particularly preferred are far-ultraviolet light having a wavelength of 1 to 200 nm and specifically KrF excimer laser (248 nm), ArF excimer laser (193 nm), F₂ excimer laser (157 nm), EUV (13.5 nm), X-rays, and an electron beam.

After the exposure, before development, baking (heating) is preferably performed. The baking accelerates the reaction in the exposed regions, to provide higher sensitivity and a better pattern profile.

The heating temperature is preferably 80 to 150°C, more preferably 80 to 140°C, and still more preferably 80 to 130°C.

The heating time is preferably 10 to 1000 seconds, more preferably 10 to 180 seconds, and still more preferably 30 to 120 seconds.

The heating can be performed using means included in an ordinary exposure device and/or an ordinary development device, and may alternatively be performed using a hot plate, for example.

This step is also referred to as post-exposure baking.

### Step 3: Development step

The step 3 is a step of using a developer to develop the exposed actinic ray-sensitive or radiation-sensitive film, to form a pattern.

The developer may be an alkali developer or may be a developer containing an organic solvent (hereafter, also referred to as organic-based developer).

Examples of the development process include a process of immersing, for a predetermined time, the substrate in a tank filled with the developer (dipping process), a process of puddling, with the developer, the surface of the substrate using surface tension and leaving the developer at rest for a predetermined time to achieve development (puddling process), a process of spraying the developer to the surface of the substrate (spraying process), and a process of scanning, at a constant rate, over the substrate rotated at a constant rate, a developer ejection nozzle to continuously eject the developer (dynamic dispensing process).

After the step of performing development, a step of performing exchange with another solvent to stop the development may be performed.

The development time is not particularly limited as long as the resin in the unexposed regions is sufficiently dissolved in the time, is preferably 10 to 300 seconds, and more preferably 20 to 120 seconds.

The temperature of the developer is preferably 0 to 50°C, and more preferably 15 to 35°C.

The alkali developer employed is preferably an alkali aqueous solution including an alkali. The type of the alkali aqueous solution is not particularly limited, but may be, for example, an alkali aqueous solution including a quaternary ammonium salt represented by tetramethylammonium hydroxide, an inorganic alkali, a primary amine, a secondary amine, a tertiary amine, an alcoholamine, a cyclic amine, or the like. In particular, the alkali developer is preferably an aqueous solution of a quaternary ammonium salt represented by tetramethylammonium hydroxide (TMAH). To the alkali developer, an appropriate amount of an alcohol, a surfactant, or the like may be added. The alkali developer ordinarily preferably has an alkali concentration of 0.1 to 20 mass%. The alkali developer ordinarily preferably has a pH of 10.0 to 15.0.

The organic-based developer is preferably a developer containing at least one organic solvent selected from the group consisting of ketone-based solvents, ester-based solvents, alcohol-based solvents, amide-based solvents, ether-based solvents, and hydrocarbon-based solvents.

A plurality of such solvents may be mixed together, or such a solvent may be mixed with a solvent other than those described above or water. The developer as a whole has a moisture content of preferably less than 50 mass%, more preferably less than 20 mass%, still more preferably less than 10 mass%, and particularly preferably contains substantially no moisture.

In the organic-based developer, the content of the organic solvent relative to the total amount of the developer is preferably 50 mass% or more and 100 mass% or less, more preferably 80 mass% or more and 100 mass% or less, still more preferably 90 mass% or more and 100 mass% or less, and particularly preferably 95 mass% or more and 100 mass% or less.

### Other step

The pattern forming method preferably includes a step of, after the step 3, using a rinse liquid to perform rinsing.

After the development step using an alkali developer, in the rinsing step, the rinse liquid employed may be, for example, pure water. Note that, to the pure water, an appropriate amount of surfactant may be added.

To the rinse liquid, an appropriate amount of surfactant may be added.

After the development step using an organic-based developer, in the rinsing step, the rinse liquid employed is not particularly limited as long as it does not dissolve the pattern, and may be a solution including an ordinary organic solvent. The rinse liquid employed is preferably a rinse liquid containing at least one organic solvent selected from the group consisting of hydrocarbon-based solvents, ketone-based solvents, ester-based solvents, alcohol-based solvents, amide-based solvents, and ether-based solvents.

The process of performing the rinsing step is not particularly limited; examples include a process of continuously ejecting, onto the substrate rotated at a constant rate, the rinse liquid (spin-coating process), a process of immersing, in a tank filled with the rinse liquid, the substrate for a predetermined time (dipping process), and a process of spraying, to the surface of the substrate, the rinse liquid (spraying process).

The pattern forming method may include a heating step (Post Bake) performed after the rinsing step. In this step, baking removes the developer and the rinse liquid remaining between and within the patterns. In addition, this step also provides an effect of annealing the resist pattern to address the rough surface of the pattern. The heating step after the rinsing step is performed ordinarily at 40 to 250°C (preferably 90 to 200°C) for ordinarily 10 seconds to 3 minutes (preferably 30 seconds to 120 seconds).

The formed pattern may be used as a mask for subjecting the substrate to etching treatment. Specifically, the pattern formed in the step 3 may be used as a mask for processing the substrate (or the underlayer film and the substrate), to form a pattern in the substrate.

The process of processing the substrate (or the underlayer film and the substrate) is not particularly limited, but is preferably a process of using the pattern formed in the step 3 as a mask for subjecting the substrate (or the underlayer film and the substrate) to dry etching, to thereby form a pattern in the substrate. The dry etching is preferably oxygen plasma etching.

Various materials used in the composition of this Specification and the pattern forming method of this Specification (for example, a solvent, a developer, a rinse liquid, an antireflection film-forming composition, and a topcoat-forming composition) preferably do not include impurities such as metals. The content of impurities included in such materials is preferably 1 mass ppm or less, more preferably 10 mass ppb or less, still more preferably 100 mass ppt or less, particularly preferably 10 mass ppt or less, and most preferably 1 mass ppt or less. The lower limit is not particularly limited, but is preferably 0 mass ppt or more. Examples of the metallic impurities include Na, K, Ca, Fe, Cu, Mg, Al, Li, Cr, Ni, Sn, Ag, As, Au, Ba, Cd, Co, Pb, Ti, V, W, and Zn.

The process of removing, from the various materials, impurities such as metals may be, for example, filtration using a filter. The details of filtration using a filter are described in Paragraph [0321] in WO2020/004306A.

Examples of the process of reducing the amount of impurities such as metals included in the various materials include a process of selecting, as raw materials constituting the various materials, raw materials having lower metal content, a process of subjecting raw materials constituting the various materials to filtration using a filter, and a process of performing distillation under conditions under which contamination is minimized by, for example, lining the interior of the apparatuses with TEFLON (registered trademark).

Instead of the filtration using a filter, an adsorption material may be used to remove impurities; alternatively, the filtration using a filter may be used in combination with an adsorption material. Such adsorption materials can be publicly known adsorption materials, and examples include inorganic-based adsorption materials such as silica gel and zeolite, and organic-based adsorption materials such as active carbon. In order to reduce the amount of impurities such as metals included in the various materials, ingress of metallic impurities in the production steps needs to be prevented. Whether or not metallic impurities are sufficiently removed from the production apparatuses can be determined by measuring the content of metallic components included in the washing liquid having been used for washing the production apparatuses. The content of metallic components included in the washing liquid having been used is preferably 100 mass ppt (parts per trillion) or less, more preferably 10 mass ppt or less, and still more preferably 1 mass ppt or less. The lower limit is not particularly limited, but is preferably 0 mass ppt or more.

To organic-based treatment liquids such as the rinse liquid, in order to prevent electrostatic buildup and the subsequent electrostatic discharge causing failure of the chemical solution pipe and various parts (such as a filter, an O-ring, and a tube), a conductive compound may be added. The conductive compound is not particularly limited, but may be, for example, methanol. The amount of addition is not particularly limited, but is, from the viewpoint of maintaining preferred development performance or rinsing performance, preferably 10 mass% or less, and more preferably 5 mass% or less. The lower limit is not particularly limited, but is preferably 0.01 mass% or more.

Examples of the chemical solution pipe include various pipes formed of SUS (stainless steel), or coated with polyethylene, polypropylene, or a fluororesin (such as polytetrafluoroethylene or a perfluoroalkoxy resin) treated so as to be antistatic. Similarly for the filter and the O-ring, polyethylene, polypropylene, or a fluororesin (such as polytetrafluoroethylene or a perfluoroalkoxy resin) treated so as to be antistatic can be used.

### Method for producing electronic device

This Specification also relates to a method for producing an electronic device, the method including the above-described pattern forming method, and an electronic device produced by the production method.

The electronic device in this Specification is, in a preferred embodiment, mounted on electric or electronic devices (such as household appliances, OA (Office Automation), media-related devices, optical devices, and communication devices).

### EXAMPLES

Hereinafter, the present invention will be described further in detail with reference to Examples. In the following Examples, materials, usage amounts, ratios, details of treatments, orders of treatments, and the like can be appropriately changed without departing from the spirit and scope of the present invention. Thus, the scope of the present invention should not be construed as being limited to the following Examples.

### Components of resist compositions

The components included in resist compositions used in Examples and Comparative Examples will be described below.

### Compound (Q)

As the compound (Q) used for preparing the resist compositions, the above-described compounds (I)-1 to (I)-18 were used. The structures of comparative compounds (Z) (compounds (Z)-1 to (Z)-3) will be described below. Me represent a methyl group.

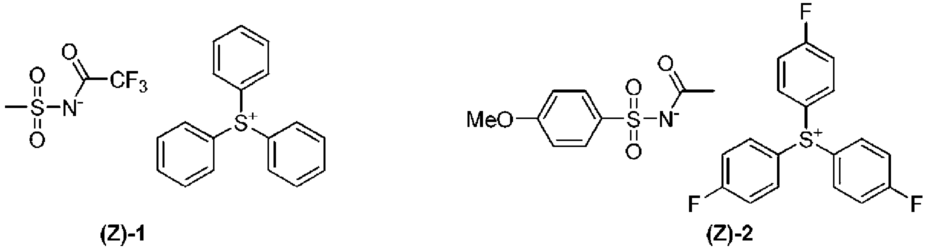

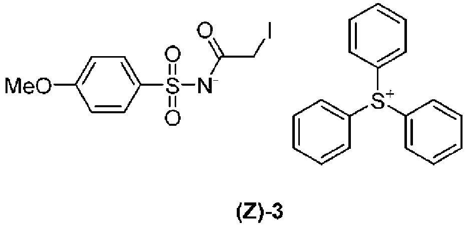

### Synthesis of compound (I)-1

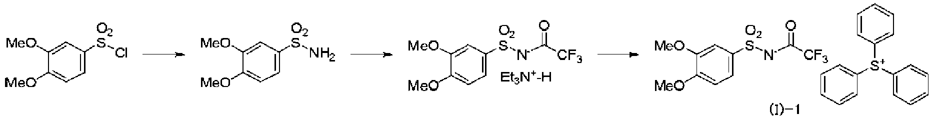

To dichloromethane (224 g), 3,4-dimethoxybenzenesulfonyl chloride (20 g) was added and cooled to 5°C or lower. Pyridine (13.4 g) and 28% aqueous ammonia (160 g) were added and subsequently stirred at room temperature (25°C) for 3 hours. Water (169 mL) and methanol (224 g) were added and subsequently the layers were separated. Dichloromethane (224 g) was used to perform extraction twice and concentration was performed to provide 8.5 g of 3,4-dimethoxybenzenesulfonamide.

¹H NMR (DMSO-d₆): d 7.36-7.42 (2H), 7.16-7.21 (2H), 7.07-7.12 (1H), 3.82 (3H), 3.81 (3H)

To dichloromethane (46.4 g), 3,4-dimethoxybenzenesulfonamide (3.50 g) and triethylamine (4.08 g) were added and cooled to 5°C or lower. Trifluoroacetic anhydride (4.06 g) was added and subsequently stirred at 5°C or lower for 30 minutes. Water (35.0 mL) was added and stirred at room temperature for 5 minutes; subsequently, triphenylsulfonium bromide (4.98 g) was added and stirred at room temperature for 15 minutes. The layers were separated and concentration was performed to thereby provide 6.6 g of a compound (I)-1.

The measurement results of the compound (I)-1 by ¹H-NMR are as follows. ¹H NMR (DMSO-d₆): d 7.75-7.90 (15H), 7.32-7.40 (2H), 6.97-7.02 (1H), 3.79 (3H), 3.76 (3H)

Compounds (I)-2 to (I)-18 and (Z)-1 to (Z)-3 were synthesized in accordance with the method for synthesizing the compound (I)-1.

### Compound (P)

As the compound (P), which is a photoacid generator different from the compound (Q) used for preparing the resist compositions, the above-described compounds (II)-1 to (II)-18 and the above-described compounds (II)-101 to (II)-108 were used.

### Resin (A)

Resins (A-1 to A-34 (corresponding to acid-decomposable resins)) used for preparing the resist compositions will be described below. The resins A-1 to A-34 used were synthesized in accordance with publicly known methods.

In Table 1, the columns "Molar ratio" indicate the content (mol%) of each repeating unit relative to all the repeating units.

In Table 1, the column "Mw" indicates the weight-average molecular weight.

In Table 1, the column "Mw/Mn" indicates the dispersity.

Note that, for the resins A-1 to A-34, the weight-average molecular weight (Mw) and the dispersity (Mw/Mn) were measured by GPC (carrier: tetrahydrofuran (THF)) (polystyrene-equivalent amounts). The compositional ratios (molar ratios) of the resins were measured using ¹³C-NMR (Nuclear Magnetic Resonance).

**Table 1**

| Table 1 | Repeating unit 1 | | Repeating unit 2 | | Repeating unit 3 | | Repeating unit 4 | | Mw | Mw/Mn |
|---|---|---|---|---|---|---|---|---|---|---|
| | Type | Molar ratio | Type | Molar ratio | Type | Molar ratio | Type | Molar ratio | | |
| Resin A-1 | MB-10 | 50 | MA-16 | 50 | - | - | - | - | 8500 | 1.60 |
| Resin A-2 | MB-15 | 40 | MA-7 | 60 | - | - | - | - | 9000 | 1.70 |
| Resin A-3 | MB-7 | 30 | MB-14 | 10 | MA-6 | 60 | - | - | 7000 | 1.55 |
| Resin A-4 | MB-5 | 50 | MB-12 | 10 | MA-15 | 40 | - | - | 7500 | 1.55 |
| Resin A-5 | MB-3 | 20 | MB-20 | 40 | MA-4 | 40 | - | - | 7000 | 1.60 |
| Resin A-6 | MB-4 | 20 | MB-14 | 30 | MA-2 | 50 | - | - | 6500 | 1.63 |
| Resin A-7 | MB-6 | 30 | MB-19 | 10 | MA-3 | 60 | - | - | 9500 | 1.45 |
| Resin A-8 | MB-9 | 30 | MB-20 | 10 | MA-13 | 60 | - | - | 12000 | 1.65 |
| Resin A-9 | MB-4 | 20 | MB-19 | 20 | MA-2 | 60 | - | - | 6000 | 1.55 |
| Resin A-10 | MB-16 | 30 | MA-17 | 70 | - | - | - | - | 8000 | 1.40 |
| Resin A-11 | MB-4 | 30 | MB-31 | 30 | MA-4 | 40 | - | - | 6500 | 1.65 |
| Resin A-12 | MB-30 | 20 | MA-8 | 80 | - | - | - | - | 5500 | 1.65 |
| Resin A-13 | MB-13 | 50 | MA-10 | 50 | - | - | - | - | 15000 | 1.75 |
| Resin A-14 | MB-8 | 30 | MA-20 | 70 | - | - | - | - | 9000 | 1.60 |
| Resin A-15 | MB-18 | 30 | MB-29 | 30 | MA-14 | 40 | - | - | 8000 | 1.55 |
| Resin A-16 | MB-3 | 30 | MB-20 | 20 | MA-2 | 50 | - | - | 7500 | 1.70 |
| Resin A-17 | MB-1 | 30 | MB-26 | 30 | MA-11 | 40 | - | - | 18000 | 1.80 |
| Resin A-18 | MB-27 | 60 | MA-5 | 40 | - | - | - | - | 7500 | 1.65 |
| Resin A-19 | MB-17 | 30 | MB-24 | 10 | MA-19 | 60 | - | - | 8000 | 1.70 |
| Resin A-20 | MB-11 | 30 | MB-28 | 40 | MA-9 | 30 | - | - | 9500 | 1.80 |
| Resin A-21 | MB-3 | 20 | MB-20 | 10 | MB-32 | 10 | MA-2 | 60 | 10000 | 1.70 |
| Resin A-22 | MB-2 | 60 | MA-1 | 40 | - | - | - | - | 11000 | 1.65 |
| Resin A-23 | MB-21 | 50 | MA-12 | 50 | - | - | - | - | 6500 | 1.60 |
| Resin A-24 | MB-23 | 40 | MA-10 | 60 | - | - | - | - | 8000 | 1.55 |
| Resin A-25 | MB-1 | 20 | MB-19 | 20 | MA-2 | 60 | - | - | 8000 | 1.55 |
| Resin A-26 | MB-25 | 30 | MA-2 | 70 | - | - | - | - | 7500 | 1.60 |
| Resin A-27 | MB-3 | 40 | MA-3 | 60 | - | - | - | - | 9500 | 1.60 |
| Resin A-28 | MB-22 | 40 | MA-4 | 60 | - | - | - | - | 10000 | 1.70 |
| Resin A-29 | MB-3 | 30 | MB-33 | 20 | MA-2 | 50 | - | - | 6600 | 1.63 |
| Resin A-30 | MB-7 | 30 | MB-14 | 10 | MA-18 | 60 | - | - | 8100 | 1.65 |
| Resin A-31 | MB-1 | 20 | MB-19 | 20 | MA-21 | 60 | - | - | 6600 | 1.65 |
| Resin A-32 | MB-17 | 60 | MA-24 | 40 | - | - | - | - | 8200 | 1.71 |
| Resin A-33 | MB-4 | 20 | MB-19 | 20 | MA-23 | 60 | - | - | 6500 | 1.60 |
| Resin A-34 | MB-14 | 30 | MA-22 | 70 | - | - | - | - | 7200 | 1.58 |

The structures of the monomers corresponding to the repeating units in the resins will be described below.

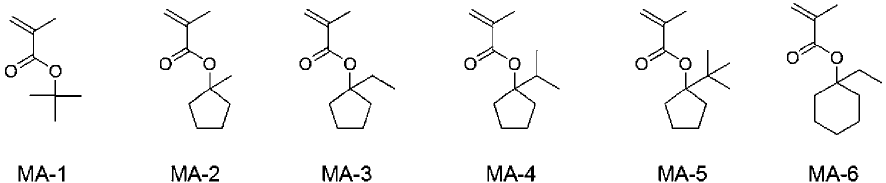

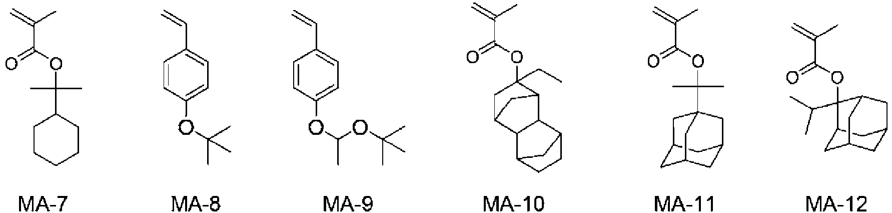

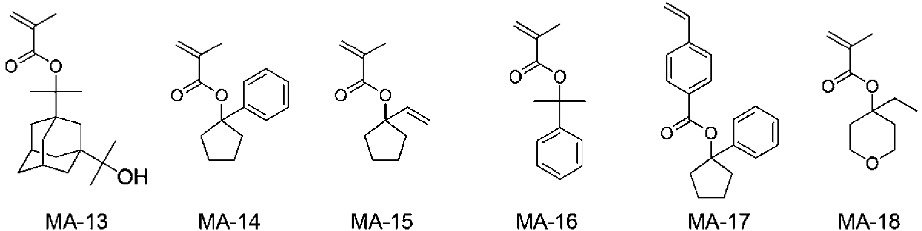

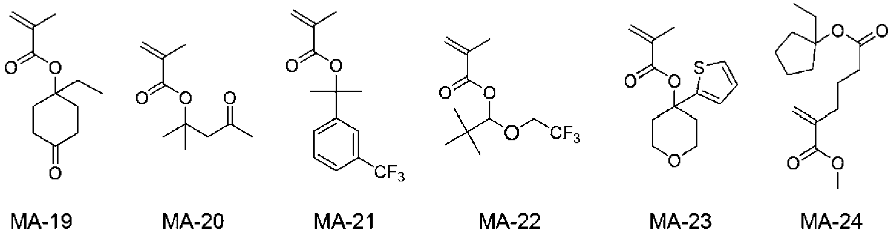

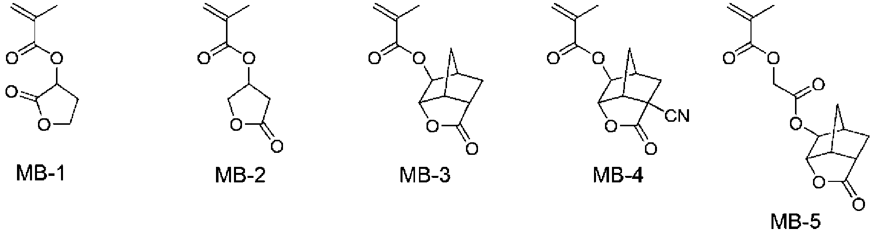

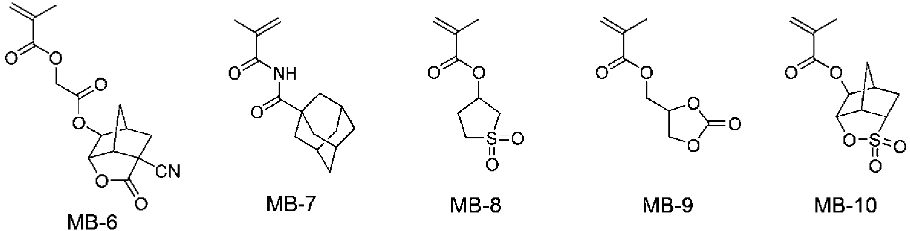

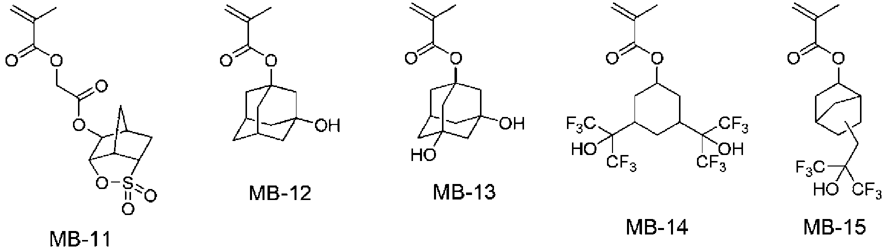

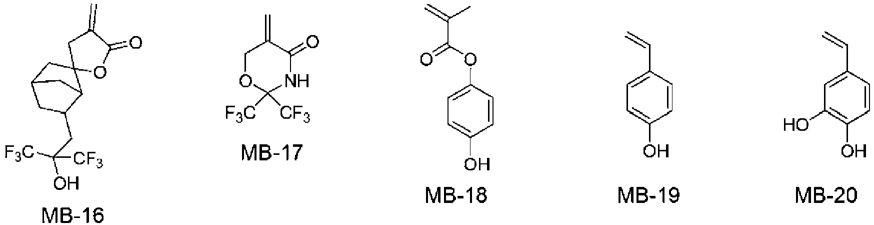

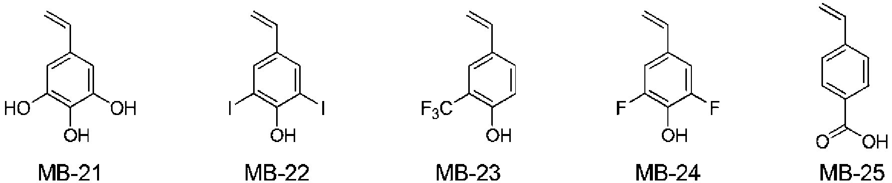

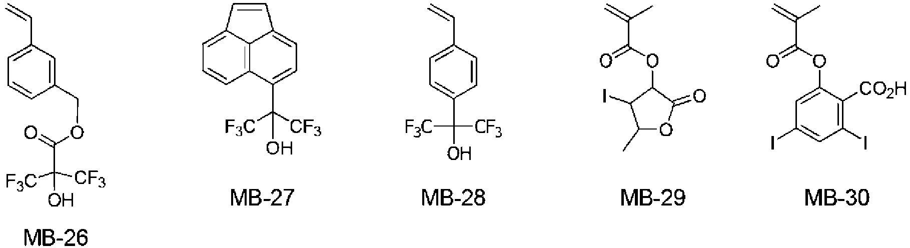

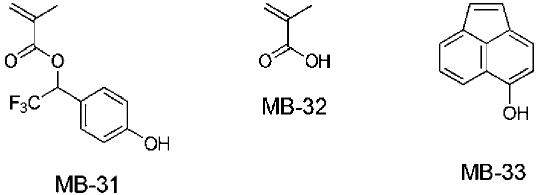

### Acid diffusion control agent

The structures of acid diffusion inhibitors (B-1 to B-5) different from the compound (Q) used for preparing the resist compositions will be described below.

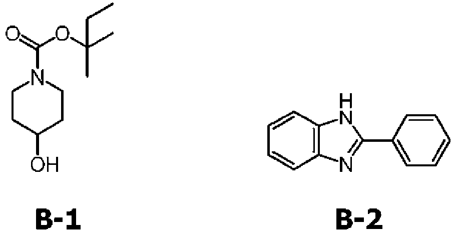

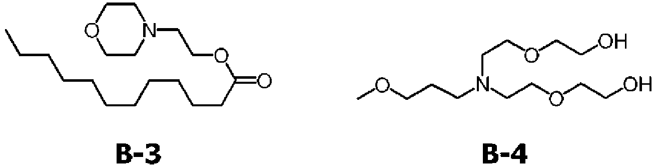

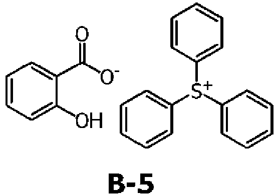

### Hydrophobic resin

Hydrophobic resins (C-1 to C-8) used for preparing the resist compositions will be described below.

In Table 2, the columns "Molar ratio" indicate the content (mol%) of each repeating unit relative to all the repeating units.

In Table 2, the column "Mw" indicates the weight-average molecular weight.

In Table 2, the column "Mw/Mn" indicates the dispersity.

Note that, for the resins C-1 to C-8, the weight-average molecular weight (Mw) and the dispersity (Mw/Mn) were measured by GPC (carrier: tetrahydrofuran (THF)) (polystyrene-equivalent amounts). The compositional ratios (molar ratios) of the resins were measured using ¹³C-NMR (Nuclear Magnetic Resonance).

**Table 2**

| Table 2 | Repeating unit 1 | | Repeating unit 2 | | Repeating unit 3 | | Repeating unit 4 | | Mw | Mw/Mn |
|---|---|---|---|---|---|---|---|---|---|---|
| | Type | Molar ratio | Type | Molar ratio | Type | Molar ratio | Type | Molar ratio | | |
| Resin C-1 | MC-9 | 50 | MC-1 | 50 | - | - | - | - | 12000 | 1.5 |
| Resin C-2 | MC-2 | 40 | MC-8 | 50 | MC-5 | 5 | MC-11 | 5 | 6000 | 1.3 |
| Resin C-3 | MC-6 | 50 | MC-2 | 50 | - | - | - | - | 15000 | 1.5 |
| Resin C-4 | MC-3 | 100 | - | - | - | - | - | - | 23000 | 1.7 |
| Resin C-5 | MC-8 | 10 | MC-10 | 85 | MC-5 | 5 | - | - | 11000 | 1.4 |
| Resin C-6 | MC-4 | 80 | MC-7 | 20 | - | - | - | - | 13000 | 1.4 |
| Resin C-7 | MC-3 | 50 | MC-12 | 50 | - | - | - | - | 12000 | 1.5 |
| Resin C-8 | MC-2 | 50 | MC-13 | 50 | - | - | - | - | 10000 | 1.6 |

The structures of the monomers corresponding to the repeating units in the hydrophobic resins will be described below.

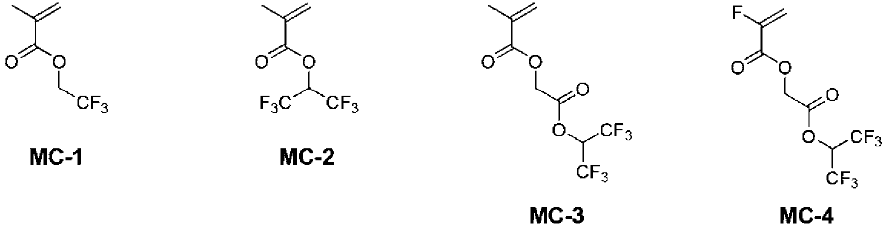

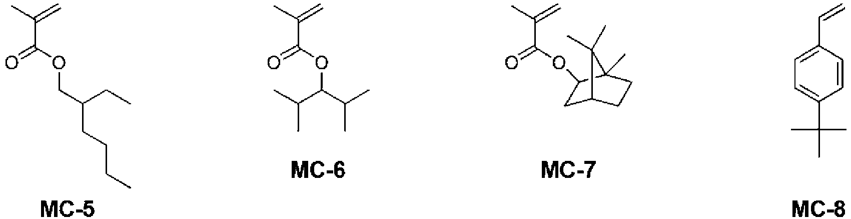

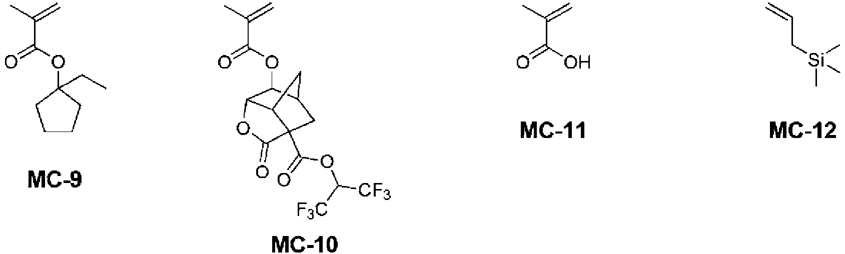

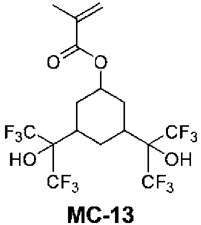

### Surfactant

Surfactants (E-1 to E-3) used for preparing the resist compositions will be described below.
E-1: MEGAFACE F176 (manufactured by DIC Corporation, fluorine-based surfactant)
E-2: MEGAFACE R08 (manufactured by DIC Corporation, fluorine-based and silicone-based surfactant)
E-3: PF656 (manufactured by OMNOVA SOLUTIONS INC., fluorine-based surfactant)

### Solvent

Solvents (F-1 to F-9) used for preparing the resist compositions will be described below.
F-1: propylene glycol monomethyl ether acetate (PGMEA)
F-2: propylene glycol monomethyl ether (PGME)
F-3: propylene glycol monoethyl ether (PGEE)
F-4: cyclohexanone
F-5: cyclopentanone
F-6: 2-heptanone
F-7: ethyl lactate
F-8: γ-butyrolactone
F-9: propylene carbonate

### Preparation and application of resist compositions

Components described in Table 3 below were mixed together so as to provide a solid content concentration of 2.0 mass%. The resulting mixed solution was filtered through a polyethylene filter having a pore size of 0.02 µm; in this way, the resist compositions were prepared.

"Solid content" means all the components other than the solvent.

In Table 3, the columns "mass%" indicate the content (mass%) of each component relative to the total solid content of the resist composition.

Such a resist composition was applied onto a 6-inch Si (silicon) wafer having been treated with hexamethyldisilazane (HMDS) in advance, using a spin coater "Mark8" manufactured by Tokyo Electron Ltd., and dried on a hot plate at 130°C for 300 seconds to obtain a resist film having a film thickness of 30 nm.

Here, 1 inch is 0.0254 m.

**Table 3**

| Table 3 | Compound (Q) | | Compound (P) | | Resin (A) | | Acid diffusion inhibitor | | Hydrophobic resin | | Surfactant | | Solvent | |
|---|---|---|---|---|---|---|---|---|---|---|---|---|---|---|
| Resist composition | Type | mass% | Type | mass% | Type | mass% | Type | mass% | Type | mass% | Type | mass% | Type | Mixing ratio (mass ratio) |
| Re-1 | (I)-1 | 11.5 | (II)-1 | 15.3 | A-25 | 73.2 | - | - | - | - | - | - | F-1/F-6 | 40/60 |
| Re-2 | (I)-2 | 15.0 | (II)-2 | 18.9 | A-7 | 66.1 | - | - | - | - | - | - | F-4 | 100 |
| Re-3 | (I)-3 | 16.9 | (II)-3 | 17.6 | A-34 | 65.5 | - | - | - | - | - | - | F-1 | 100 |
| Re-4 | (I)-4 | 11.6 | (II)-4 | 21.0 | A-3 | 66.6 | - | - | C-8 | 0.8 | - | - | F-1 | 100 |
| Re-5 | (I)-5 | 12.0 | (II)-5 | 20.4 | A-6 | 65.6 | - | - | C-5 | 2.0 | - | - | F-1/F-3 | 80/20 |
| Re-6 | (I)-6 | 15.8 | (II)-6 | 14.6 | A-20 | 67.3 | - | - | C-4 | 2.3 | - | - | F-1/F-9 | 90/10 |
| Re-7 | (I)-7 | 13.8 | (II)- 7 | 16.3 | A-12 | 69.9 | - | - | - | - | - | - | F-1/F-7 | 80/20 |
| Re-8 | (I)-8 | 12.7 | (II)-8 | 14.3 | A-9 | 72.8 | - | - | - | - | E-1/ E-2 | 0.1/ 0.1 | F-1/F-8 | 85/15 |
| Re-9 | (I)-9 | 13.5 | (II)-9 | 20.0 | A-15 | 66.5 | - | - | - | - | - | - | F-4 | 100 |
| Re-10 | (I)-10 | 13.0 | (II)-10 | 22.4 | A-30 | 63.1 | - | - | C-3 | 1.5 | - | - | F-1/F-5 | 50/50 |
| Re-11 | (I)-11 | 16.4 | (II)-11 | 19.7 | A-33 | 63.9 | - | - | - | - | - | - | F-1 | 100 |
| Re-12 | (I)-12 | 13.8 | (II)-12 | 18.0 | A-12 | 68.2 | - | - | - | - | - | - | F-1/F-2/F-8 | 70/25/5 |
| Re-13 | (I)-1/ (I)-13 | 5.8/ 7.8 | (II)-13 | 15.1 | A-1 | 71.3 | - | - | - | - | - | - | F-1/F-6 | 40/60 |
| Re-14 | (I)-14 | 10.8 | (II)-14 | 24.3 | A-2 | 62.9 | - | - | C-1 | 2.0 | - | - | F-1/F-5 | 50/50 |
| Re-15 | (I)-15 | 15.2 | (II)-15 | 16.7 | A-27 | 68.1 | - | - | - | - | - | - | F-1 | 100 |
| Re-16 | (I)-16 | 10.8 | (II)-16 | 14.7 | A-31 | 74.5 | - | - | - | - | - | - | F-1/F-2/F-8 | 70/25/5 |
| Re-17 | (I)-17 | 11.3 | (II)-17 | 16.5 | A-5 | 72.1 | - | - | - | - | E-1 | 0.1 | F-7 | 100 |
| Re-18 | (I)-18 | 16.2 | (II)-18 | 14.9 | A-23 | 68.9 | - | - | - | - | - | - | F-1/F-8 | 85/15 |
| Re-19 | (I)-1 | 1.2 | (II)-101 | 30.0 | A-29 | 68.8 | - | - | - | - | - | - | F-1/F-2 | 70/30 |
| Re-20 | (I)-2 | 1.2 | (II)-102 | 20.3 | A-17 | 78.4 | - | - | - | - | E-2 | 0.1 | F-1/F-5 | 50/50 |
| Re-21 | (I)-3 | 1.7 | (II)-103 | 24.1 | A-10 | 70.8 | - | - | C-6 | 3.4 | - | - | F-1/F-2 | 70/30 |
| Re-22 | (I)-4 | 1.2 | (II)-104 | 22.6 | A-4 | 76.2 | - | - | - | - | - | - | F-1/F-8 | 85/15 |
| Re-23 | (I)-5 | 1.2 | (II)-105 | 19.1 | A-32 | 76.8 | - | - | C-7 | 2.9 | - | - | F-1 | 100 |
| Re-24 | (I)-6 | 1.6 | (II)-106 | 19.4 | A-19 | 77.9 | - | - | C-2 | 1.1 | - | - | F-4 | 100 |
| Re-25 | (I)-7 | 4.1 | (II)-107 | 35.9 | A-18 | 60.0 | - | - | - | - | - | - | F-1/F-6 | 40/60 |

**Table 4**

| Table 3 (continued) | Compound (Q) | | Compound (P) | | Resin (A) | | Acid diffusion inhibitor | | Hydrophobic resin | | Surfactant | | Solvent | |
|---|---|---|---|---|---|---|---|---|---|---|---|---|---|---|
| Resist composition | Type | mass% | Type | mass% | Type | mass% | Type | mass% | Type | mass% | Type | mass% | Type | Mixing ratio (mass ratio) |
| Re-26 | (I)-8 | 3.8 | (II)-108 | 24.5 | A-8 | 71.6 | - | - | - | - | E-3 | 0.1 | F-4 | 100 |
| Re-27 | (I)-9 | 2.0 | (II)-107 | 35.9 | A-14 | 61.4 | B-1 | 0.7 | - | - | - | - | F-4 | 100 |
| Re-28 | (I)-10 | 6.5 | (II)-101/ (II)-108 | 21.3/ 34.3 | A-22/ A-24 | 30.0/ 7.9 | - | - | - | - | - | - | F-1 | 100 |
| Re-29 | (I)-1 | 0.6 | (II)-103 | 24.1 | A-11 | 75.0 | B-3 | 0.3 | - | - | - | - | F-1/F-8 | 85/15 |
| Re-30 | (I)-2 | 0.6 | (II)-104 | 22.6 | A-16 | 76.5 | B-4 | 0.3 | - | - | - | - | F-7 | 100 |
| Re-31 | (I)-3 | 8.5 | (II)-1 | 15.3 | A-26 | 74.1 | B-1 | 2.1 | - | - | - | - | F-1/F-8 | 85/15 |
| Re-32 | (I)-4 | 5.8 | (II)-2 | 15.1 | A-13 | 75.1 | B-5 | 4.0 | - | - | - | - | F-1/F-2 | 70/30 |
| Re-33 | (I)-4 | 5.8 | (II)-3 | 17.6 | A-21 | 73.5 | B-3 | 3.1 | - | - | - | - | F-1/F-3 | 90/10 |
| Re-34 | (I)-5 | 6.0 | (II)-4 | 21.0 | A-28 | 70.4 | B-4 | 2.6 | - | - | - | - | F-4 | 100 |
| Re-35 | (I)-17 | 5.6 | (II)-5 | 20.4 | A-1 | 71.8 | B-2 | 2.2 | - | - | - | - | F-1/F-8 | 85/15 |
| Re-C1 | (Z)-1 | 9.1 | (II)-1 | 15.1 | A-1 | 75.8 | - | - | - | - | - | - | F-1/F-6 | 40/60 |
| Re-C2 | (Z)-2 | 1.1 | (II)-101 | 30.0 | A-2 | 68.9 | - | - | - | - | - | - | F-1/F-6 | 40/60 |
| Re-C3 | (Z)-3 | 3.8 | (II)-107 | 24.5 | A-3 | 71.6 | - | - | - | - | E-3 | 0.1 | F-4 | 100 |

### Examples 1-1 to 1-35 and Comparative Examples 1-1 to 1-3

### Pattern forming method (1): EUV exposure and organic-solvent development EUV exposure

The wafer coated with the resist film obtained above was subjected to pattern exposure using an EUV exposure apparatus manufactured by Exitech Ltd. (Micro Exposure Tool, NA (numerical aperture): 0.3, Quadrupole, outer sigma: 0.68, inner sigma: 0.36). As the exposure mask, a mask having a line width of 20 nm and a 1:1 line and space pattern was used.

### Organic-solvent development

The exposed wafer was heated on a hot plate at 90°C for 60 seconds, and subsequently developed with n-butyl acetate for 30 seconds; this was spin-dried to obtain a negative resist pattern.

### Evaluation 1: roughness performance

The roughness performance was evaluated on the basis of LWR (Line Width Roughness).

A 20 nm (1:1) line and space pattern resolved at an optimum exposure dose for resolving a line pattern having an average line width of 20 nm was observed from above using a critical dimension-scanning electron microscope (CD SEM, Hitachi, Ltd., S-9380II): the line width was observed at randomly selected points, and the measurement variation was evaluated at 3σ (nm). This 3σ was defined as "LWR (nm)". The smaller the value, the higher the performance.

The resist composition immediately after production was used to form a pattern as described above, and LWR (nm) was calculated. The smaller the value, the higher the performance.

### Evaluation grades

A: 3.0 nm or less
B: more than 3.0 nm and 4.0 nm or less
C: more than 4.0 nm

### Evaluation 2: temporal roughness performance

Instead of the composition used above immediately after preparation, a composition having been left to stand in a 4°C environment for 3 months after preparation was used to obtain a pattern in accordance with the same procedures as described above, and LWR was measured in accordance with the same procedures as described above. Subsequently, the following formula (IA) was used to determine the LWR variation rate (%) in the case of using the composition after having been left to stand in a 4°C environment for 3 months, and evaluation was performed on the basis of evaluation grades below.

Formula (IA): LWR variation rate (%) = {|(LWR (nm) of pattern using composition after having been left to stand in 4°C environment for 3 months - LWR (nm) of pattern using composition immediately after production)|/LWR (nm) of pattern using composition immediately after production} × 100

### Evaluation grades

A: less than 1%
B: LWR variation rate of 1% or more and less than 3%
C: LWR variation rate of 3% or more

The evaluation results will be described in Table 4.

**Table 5**

| Table 4 | Resist composition | Evaluation 1 | Evaluation 2 |
|---|---|---|---|
| Example 1-1 | Re-1 | A | A |
| Example 1-2 | Re-2 | A | A |
| Example 1-3 | Re-3 | A | A |
| Example 1-4 | Re-4 | A | A |
| Example 1-5 | Re-5 | A | A |
| Example 1-6 | Re-6 | A | A |
| Example 1-7 | Re-7 | A | A |
| Example 1-8 | Re-8 | A | A |
| Example 1-9 | Re-9 | A | A |
| Example 1-10 | Re-10 | A | A |
| Example 1-11 | Re-11 | A | A |
| Example 1-12 | Re-12 | A | A |
| Example 1-13 | Re-13 | A | A |
| Example 1-14 | Re-14 | B | A |
| Example 1-15 | Re-15 | B | A |
| Example 1-16 | Re-16 | A | B |
| Example 1-17 | Re-17 | A | B |
| Example 1-18 | Re-18 | A | B |
| Example 1-19 | Re-19 | A | A |
| Example 1-20 | Re-20 | A | A |
| Example 1-21 | Re-21 | A | A |
| Example 1-22 | Re-22 | A | A |
| Example 1-23 | Re-23 | A | A |
| Example 1-24 | Re-24 | A | A |
| Example 1-25 | Re-25 | A | A |
| Example 1-26 | Re-26 | A | A |
| Example 1-27 | Re-27 | A | A |
| Example 1-28 | Re-28 | A | A |
| Example 1-29 | Re-29 | A | A |
| Example 1-30 | Re-30 | A | A |
| Example 1-31 | Re-31 | A | A |
| Example 1-32 | Re-32 | A | A |
| Example 1-33 | Re-33 | A | A |
| Example 1-34 | Re-34 | A | A |
| Example 1-35 | Re-35 | A | B |
| Comparative Example 1-1 | Re-C1 | B | C |
| Comparative Example 1-2 | Re-C2 | C | B |
| Comparative Example 1-3 | Re-C3 | B | C |

### Examples 2-1 to 2-35 and Comparative Examples 2-1 to 2-3

### Pattern forming method (2): EUV exposure and alkali development

### EUV exposure

The wafer coated with the resist film obtained above was subjected to pattern exposure using an EUV exposure apparatus manufactured by Exitech Ltd. (Micro Exposure Tool, NA (numerical aperture): 0.3, Quadrupole, outer sigma: 0.68, inner sigma: 0.36). As the exposure mask, a mask having a line width of 20 nm and a 1:1 line and space pattern was used.

### Alkali development

The exposed wafer was heated on a hot plate at 100°C for 90 seconds, subsequently immersed in a 2.38 mass% aqueous tetramethylammonium hydroxide (TMAH) solution for 60 seconds, and subsequently rinsed with water for 30 seconds. Subsequently, the wafer was rotated at a rotation rate of 4000 rpm for 30 seconds and subsequently baked at 95°C for 60 seconds for drying, to obtain a positive resist pattern.

### Evaluation

The roughness performance and the temporal roughness performance were evaluated in the same manner as in the negative resist patterns obtained by organic solvent development. The evaluation results will be described in Table 5.

**Table 6**

| Table 5 | Resist composition | Evaluation 1 | Evaluation 2 |
|---|---|---|---|
| Example 2-1 | Re-1 | A | A |
| Example 2-2 | Re-2 | A | A |
| Example 2-3 | Re-3 | A | A |
| Example 2-4 | Re-4 | A | A |
| Example 2-5 | Re-5 | A | A |
| Example 2-6 | Re-6 | A | A |
| Example 2-7 | Re-7 | A | A |
| Example 2-8 | Re-8 | A | A |
| Example 2-9 | Re-9 | A | A |
| Example 2-10 | Re-10 | A | A |
| Example 2-11 | Re-11 | A | A |
| Example 2-12 | Re-12 | A | A |
| Example 2-13 | Re-13 | A | A |
| Example 2-14 | Re-14 | B | A |
| Example 2-15 | Re-15 | B | A |
| Example 2-16 | Re-16 | A | B |
| Example 2-17 | Re-17 | A | B |
| Example 2-18 | Re-18 | A | B |
| Example 2-19 | Re-19 | A | A |
| Example 2-20 | Re-20 | A | A |
| Example 2-21 | Re-21 | A | A |
| Example 2-22 | Re-22 | A | A |
| Example 2-23 | Re-23 | A | A |
| Example 2-24 | Re-24 | A | A |
| Example 2-25 | Re-25 | A | A |
| Example 2-26 | Re-26 | A | A |
| Example 2-27 | Re-27 | A | A |
| Example 2-28 | Re-28 | A | A |
| Example 2-29 | Re-29 | A | A |
| Example 2-30 | Re-30 | A | A |
| Example 2-31 | Re-31 | A | A |
| Example 2-32 | Re-32 | A | A |
| Example 2-33 | Re-33 | A | A |
| Example 2-34 | Re-34 | A | A |
| Example 2-35 | Re-35 | A | B |
| Comparative Example 2-1 | Re-C1 | B | C |
| Comparative Example 2-2 | Re-C2 | C | B |
| Comparative Example 2-3 | Re-C3 | B | C |

The results of Table 4 and Table 5 have demonstrated that the resist patterns formed using the compositions of the present invention have both of high roughness performance and high temporal roughness performance.

## Claims

1. An actinic ray-sensitive or radiation-sensitive resin composition comprising a compound (Q) represented by a formula (I-1) below and a resin having a polarity that increases through decomposition by an action of an acid, 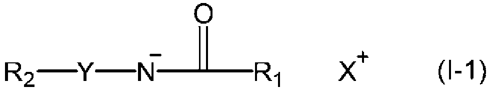 wherein, in the formula (I-1), R₁ represents a hydrocarbon group having at least one or more fluorine atoms, R₂ represents an aromatic group, Y represents -SO₂- or -CO-, and X⁺ represents a counter cation.

2. The actinic ray-sensitive or radiation-sensitive resin composition according to claim 1, wherein R₁ is a group represented by a formula (II) or (III) below: 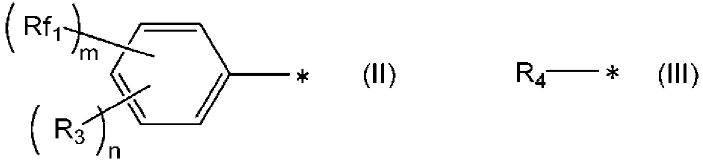
wherein, in the formula (II),
Rf₁ represents a fluorine atom or a perfluoroalkyl group,
R₃ represents a substituent,
m represents an integer of 1 to 5,
n represents an integer of 0 to 4,
wherein 1 ≤ (m + n) ≤ 5 is satisfied,
when m is an integer of 2 or more, a plurality of Rf₁'s may be the same or different,
when n is an integer of 2 or more, a plurality of R₃'s may be the same or different and may be linked together to form a ring, and
* represents a bonding site, and
wherein, in the formula (III), R₄ represents an alkyl group or cycloalkyl group having at least one or more fluorine atoms, and * represents a bonding site.

3. The actinic ray-sensitive or radiation-sensitive resin composition according to claim 1 or 2, wherein R₁ is a perfluoroalkyl group, a perfluorocycloalkyl group, or a perfluoroaryl group.

4. The actinic ray-sensitive or radiation-sensitive resin composition according to any one of claims 1 to 3, wherein R₂ is an aromatic group having one or more substituents.

5. The actinic ray-sensitive or radiation-sensitive resin composition according to any one of claims 1 to 4, wherein R₂ is an aromatic group having at least one substituent selected from the group consisting of a hydroxy group, an alkoxy group, an acyloxy group, an alkoxycarbonyl group, an aliphatic sulfonyl group, an aromatic sulfonyl group, a fluorine atom, a chlorine atom, a bromine atom, an iodine atom, an amino group, a nitro group, a cyano group, -NRₐ-C(=O)-R_{b}, and -NRₐ-C(=O)-O-R_{b} where Rₐ and R_{b} each independently represent a hydrogen atom or a hydrocarbon group.

6. The actinic ray-sensitive or radiation-sensitive resin composition according to any one of claims 1 to 5, wherein R₂ is an aromatic group having at least one substituent selected from the group consisting of a hydroxy group, an alkoxy group, an acyloxy group, an alkoxycarbonyl group, an aliphatic sulfonyl group, an aromatic sulfonyl group, an amino group, a nitro group, a cyano group, -NRₐ-C(=O)-R_{b}, and -NRₐ-C(=O)-O-R_{b} where Rₐ and R_{b} each independently represent a hydrogen atom or a hydrocarbon group.

7. The actinic ray-sensitive or radiation-sensitive resin composition according to any one of claims 1 to 6, wherein Y represents -SO₂-.

8. The actinic ray-sensitive or radiation-sensitive resin composition according to any one of claims 1 to 7, wherein X⁺ is a sulfonium cation or an iodonium cation.

9. The actinic ray-sensitive or radiation-sensitive resin composition according to any one of claims 1 to 8, further comprising a compound (P) that is different from the compound (Q) and generates an acid by irradiation with an actinic ray or a radiation.

10. An actinic ray-sensitive or radiation-sensitive film formed from the actinic ray-sensitive or radiation-sensitive resin composition according to any one of claims 1 to 9.

11. A pattern forming method comprising:
a step of using the actinic ray-sensitive or radiation-sensitive resin composition according to any one of claims 1 to 9 to form an actinic ray-sensitive or radiation-sensitive film on a substrate;
a step of exposing the actinic ray-sensitive or radiation-sensitive film; and
a step of using a developer to develop the exposed actinic ray-sensitive or radiation-sensitive film to form a pattern.

12. A method for producing an electronic device, the method comprising the pattern forming method according to claim 11.

13. A compound represented by a formula (I-1) below: 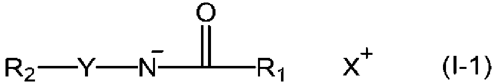 wherein, in the formula (I-1), R₁ represents a hydrocarbon group having at least one or more fluorine atoms, R₂ represents an aromatic group, Y represents -SO₂- or -CO-, and X⁺ represents a counter cation.

14. The compound according to claim 13, wherein R₁ is a group represented by a formula (II) or (III) below: 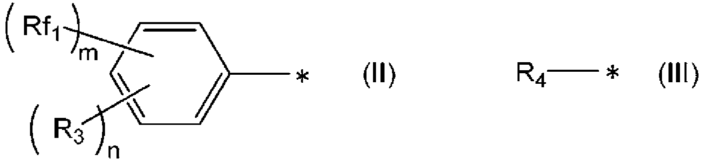
wherein, in the formula (II),
Rf₁ represents a fluorine atom or a perfluoroalkyl group,
R₃ represents a substituent,
m represents an integer of 1 to 5,
n represents an integer of 0 to 4,
wherein 1 ≤ (m + n) ≤ 5 is satisfied,
when m is an integer of 2 or more, a plurality of Rf₁'s may be the same or different,
when n is an integer of 2 or more, a plurality of R₃'s may be the same or different and may be linked together to form a ring, and
* represents a bonding site, and
wherein, in the formula (III), R₄ represents an alkyl group or cycloalkyl group having at least one or more fluorine atoms, and * represents a bonding site.

15. The compound according to claim 13 or 14, wherein R₁ is a perfluoroalkyl group, a perfluorocycloalkyl group, or a perfluoroaryl group.

16. The compound according to any one of claims 13 to 15, wherein R₂ is an aromatic group having at least one substituent selected from the group consisting of a hydroxy group, an alkoxy group, an acyloxy group, an alkoxycarbonyl group, an aliphatic sulfonyl group, an aromatic sulfonyl group, a fluorine atom, a chlorine atom, a bromine atom, an iodine atom, an amino group, a nitro group, a cyano group, -NRₐ-C(=O)-R_{b}, and -NRₐ-C(=O)-O-R_{b} where Rₐ and R_{b} each independently represent a hydrogen atom or a hydrocarbon group.

17. The compound according to any one of claims 13 to 16, wherein R₂ is an aromatic group having at least one substituent selected from the group consisting of a hydroxy group, an alkoxy group, an acyloxy group, an alkoxycarbonyl group, an aliphatic sulfonyl group, an aromatic sulfonyl group, an amino group, a nitro group, a cyano group, -NRₐ-C(=O)-R_{b}, and -NRₐ-C(=O)-O-R_{b} where Rₐ and R_{b} each independently represent a hydrogen atom or a hydrocarbon group.

18. The compound according to any one of claims 13 to 17, wherein Y represents -SO₂-.

19. The compound according to any one of claims 13 to 18, wherein X⁺ is a sulfonium cation or an iodonium cation.
